# EUROPEAN PATENT APPLICATION

(11) **EP 4 650 345 A1**
(43) Date of publication of application: **19.11.2025**
(21) Application number: 24741240.6
(22) Date of filing: 09.01.2024
(51) Int. Cl.: C07D 209/08

(54) **HETEROCYCLIC COMPOUND AND USE THEREOF IN MEDICINE**

(30) Priority: 09.01.2023 CN 202310024567; 14.08.2023 CN 202311021829
(71) Applicant: Kangbaida (Sichuan) Biotechnology Co., Ltd., Chengdu, Sichuan 610000 (CN)
(72) Inventor: CHU, Hongzhu, Chengdu, Sichuan 611130 (CN); WEI, Yonggang, Chengdu, Sichuan 611130 (CN); YE, Fei, Chengdu, Sichuan 611130 (CN); CHEN, Jiabao, Chengdu, Sichuan 611130 (CN); SUN, Yi, Chengdu, Sichuan 611130 (CN)
(74) Representative: Kador & Partner Part mbB
(86) International application number: PCT/CN2024/071332
(87) International publication number: WO 2024/149242

(57) **Abstract**

The present invention relates to a heterocyclic compound represented by general formula (I), or a stereoisomer, deuterated product, pharmaceutically acceptable salt, solvate, prodrug, metabolite or co-crystal thereof, and also relates to a pharmaceutical composition thereof and a use thereof in a drug for the treatment of MRGPRX4-dependent disorders.

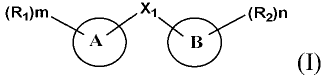

## Description

### Technical Field

The present invention relates to a heterocyclic compound or a pharmaceutically acceptable salt, isomer, hydrate, solvate, isotopically labeled compound, etc. thereof, a pharmaceutical composition thereof, and the use thereof in medicine.

### Background Art

Mas-related gene G protein-coupled receptors (MRGPRs) are a group of orphan receptors with limited expression in very specific tissues. Members of the MRGPR family are divided into 9 subclasses, namely MRGPRA-H and MRGPRX, respectively. The MRGPRX (MRGX) subfamily is expressed in small-diameter sensory neurons of the dorsal root ganglia, keratinocytes, and a few other tissues. The MRGPRX family consists of 4 subtypes (MRGPRX1-X4), which are expressed in primates and not found in rodents. To date, non-primate orthologues of MRGPRX4 have not been identified.

MRGPRX4 is activated by many other components of bile, including bile acids and metabolites thereof, and heme metabolites, including bilirubin and urobilin. Bile acid and bilirubin are highly elevated in cholestatic pruritus, and urobilin is an effective itch-inducing mediator in a mouse model and thus may be important for itching sensation in the case of elevated urobilin, such as uremic pruritus. In addition, MRGPRX4 is the receptor of urobilin, which is an effective itch-inducing mediator in a mouse model, and thus may be important for itching sensation in conditions with elevated urobilin, such as uremic pruritus. Therefore, regulating MRGPRX4 can treat pruritus-related conditions, pain-related conditions, autoimmune conditions, etc.

MRGPRX4 receptor represents a basically new drug target, and the development of effective MRGPRX4 receptor antagonists and agonists can be used to treat MRGPRX4-related conditions.

### Summary of the Invention

An object of the present invention is to provide a new heterocyclic compound, or a pharmaceutically acceptable salt, isomer, hydrate, solvate, isotopically labeled compound, etc. thereof, a pharmaceutical composition thereof, and the use thereof in medicine.

One or more embodiments of the present invention provide a compound represented by formula (I), or a stereoisomer, deuterated compound, pharmaceutically acceptable salt, solvate, prodrug, metabolite, or co-crystal thereof: 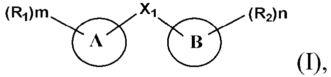
wherein ring A is selected from 6- to 10-membered aryl or 5- to 10-membered heteroaryl, wherein the 5- to 10-membered heteroaryl contains 1 to 3 heteroatoms selected from N, O, or S;
ring B is selected from 6- to 12-membered carbocyclyl or 6- to 12-membered heterocyclyl, wherein the 6- to 12-membered heterocyclyl contains 1 to 3 heteroatoms selected from N, O, or S;
X₁ is selected from C₁₋₆ alkylene, C₁₋₆ alkenylene, C₁₋₆ alkynylene, -C(O)-, -NR₃-, -O-, -S-, or -C(O)NR₃-, wherein the C₁₋₆ alkylene is optionally further substituted with a C₁₋₆ alkyl substituent;
R₁, R₂, and R₃ are each independently selected from H, cyano, halogen, hydroxyl, carboxyl, C₁₋₆ alkyl, C₂₋₆ alkenyl, C₂₋₆ alkynyl, C₁₋₆ haloalkyl, -O-C₁₋₆ alkyl, -O-C₁₋₆ haloalkyl, -C(O)NH₂, 3- to 6-membered cycloalkyl, 3- to 6-membered heterocycloalkyl, 5- to 6-membered heterocyclyl, or -C₁₋₆ alkylene-O-C₁₋₆ alkyl, wherein the C₁₋₆ alkyl substituent is optionally further substituted with a -O-C₁₋₆ alkyl, or cyano substituent; and
m and n are each independently selected from 0, 1, 2, 3, or 4.

As for the compound, or the stereoisomer, deuterated compound, pharmaceutically acceptable salt, solvate, prodrug, metabolite, or co-crystal thereof, as provided in one or more embodiments of the present invention, the compound is selected from compounds represented by general formula (II): 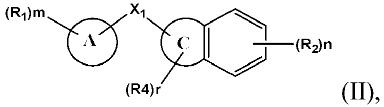
wherein ring A is selected from 6- to 8-membered aryl or 5- to 6-membered heteroaryl, wherein the 5- to 6-membered heteroaryl contains 1 to 3 heteroatoms selected from N, O, or S;
ring C is selected from 5- to 6-membered heterocyclyl, wherein the 5- to 6-membered heterocyclyl contains 1 to 3 heteroatoms selected from N, O, or S;
X₁ is selected from C₁₋₆ alkylene or -C(O)-, wherein the C₁₋₆ alkylene is optionally further substituted with a C₁₋₆ alkyl substituent;
each R₁ is independently selected from halogen, cyano, carboxyl, -C(O)NH₂, C₁₋₆ haloalkyl, or 5-membered heterocyclyl, wherein the 5-membered heterocyclyl contains 3 to 4 heteroatoms selected from N or O, and the 5-membered heterocyclyl is optionally further substituted with a =O substituent;
each R₂ is independently selected from halogen, cyano, C₁₋₆ alkyl, C₂₋₆ alkynyl, C₁₋₆ haloalkyl, -O-C₁₋₆ haloalkyl, -C₁₋₆ alkylene-O-C₁₋₆ alkyl, 3- to 6-membered cycloalkyl, 5-membered heteroaryl, or 6-membered heterocycloalkyl, wherein the C₁₋₆ alkyl is optionally further substituted with a C₁₋₆ alkoxy, cyano, or halogen substituent;
each R₄ is independently selected from C₁₋₆ alkyl or cyano;
m is selected from 1 or 2;
r is selected from 0, 1, or 2; and
n is selected from 0, 1, 2, 3, or 4.

As for the compound or the stereoisomer, deuterated compound, pharmaceutically acceptable salt, solvate, prodrug, metabolite, or co-crystal thereof, as provided in one or more embodiments of the present invention,
ring C is selected from 5- to 6-membered azaheterocyclyl;
ring A is selected from 6-membered aryl or 5- to 6-membered heteroaryl, wherein the 5- to 6-membered heteroaryl contains 1 to 3 heteroatoms selected from N, O, or S;
X₁ is selected from C₁₋₄ alkylene or -C(O)-, wherein the C₁₋₄ alkylene is optionally further substituted with a C₁₋₄ alkyl substituent;
each R₁ is independently selected from halogen, cyano, carboxyl, -C(O)NH₂, C₁₋₄ haloalkyl, or 5-membered heterocyclyl, wherein the 5-membered heterocyclyl contains 3 to 4 heteroatoms selected from N or O, and the 5-membered heterocyclyl is optionally further substituted with a =O substituent;
each R₂ is independently selected from halogen, cyano, C₁₋₄ alkyl, C₂₋₄ alkynyl, C₁₋₄ haloalkyl, -O-C₁₋₄ haloalkyl, -C₁₋₄ alkylene-O-C₁₋₄ alkyl, 3- to 6-membered cycloalkyl, 5-membered heteroaryl, or 6-membered heterocycloalkyl, wherein the C₁₋₄ alkyl is optionally further substituted with a C₁₋₄ alkoxy, cyano, or halogen substituent; and
each R₄ is independently selected from C₁₋₄ alkyl or cyano.

As for the compound, or the stereoisomer, deuterated compound, pharmaceutically acceptable salt, solvate, prodrug, metabolite, or co-crystal thereof, as provided in one or more embodiments of the present invention,
ring C is selected from 5-membered azaheterocyclyl.

As for the compound, or the stereoisomer, deuterated compound, pharmaceutically acceptable salt, solvate, prodrug, metabolite, or co-crystal thereof, as provided in one or more embodiments of the present invention,
ring A is selected from 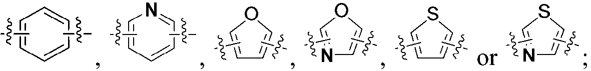 a condensed ring system formed from ring C and a benzene ring is selected from 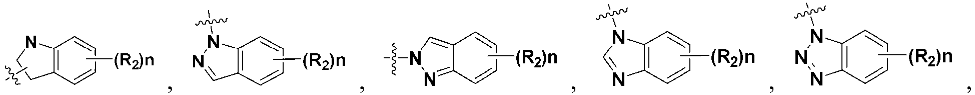 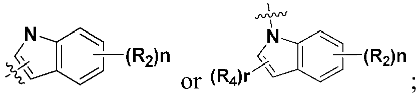 each R₂ is independently selected from halogen, cyano, C₁₋₄ alkyl, C₂₋₄ alkynyl, C₁₋₄ haloalkyl, -O-C₁₋₄ haloalkyl, 3- to 6-membered cycloalkyl, 5-membered heteroaryl, or 6-membered heterocycloalkyl, wherein the C₁₋₄ alkyl is optionally further substituted with a C₁₋₄ alkoxy, cyano or halogen substituent.

As for the compound, or the stereoisomer, deuterated compound, pharmaceutically acceptable salt, solvate, prodrug, metabolite, or co-crystal thereof, as provided in one or more embodiments of the present invention, the compound is selected from the following structures: 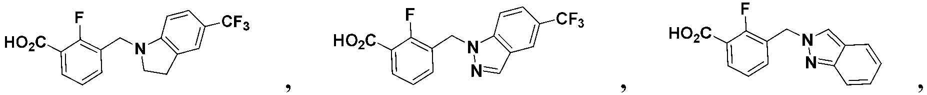 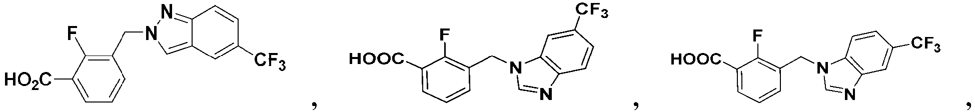 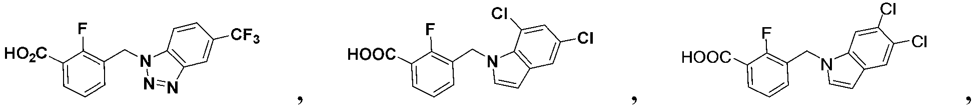 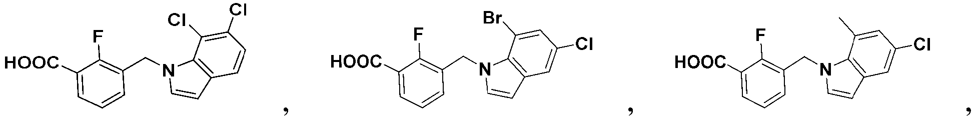 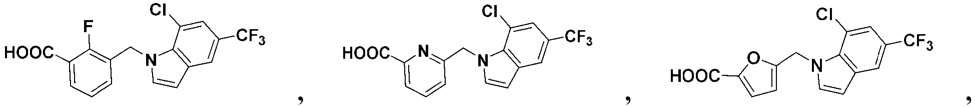 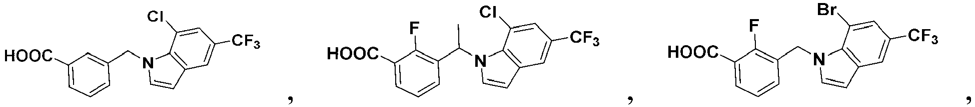 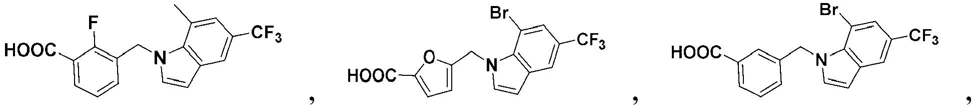 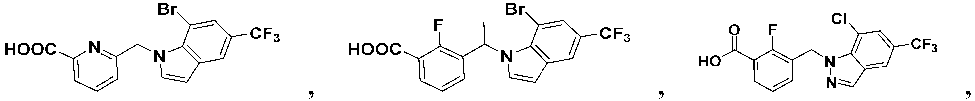 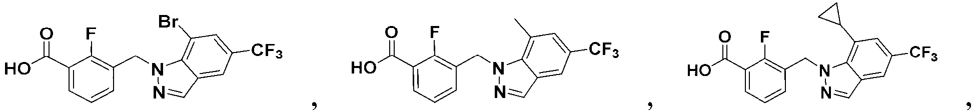 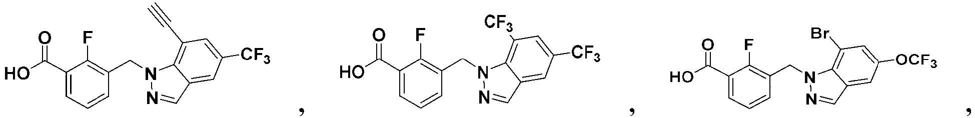 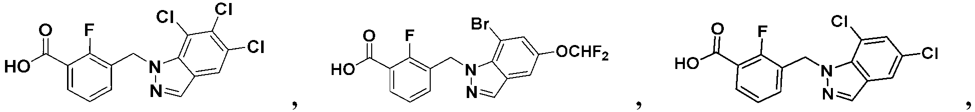 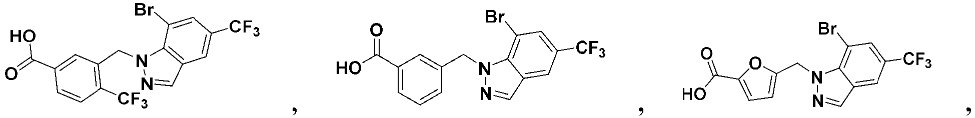 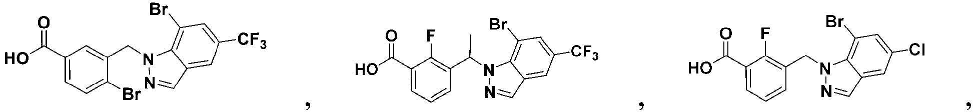 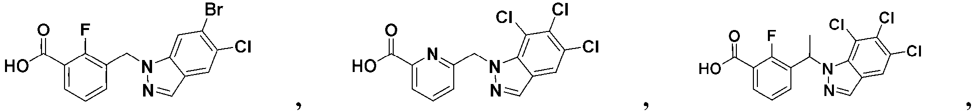 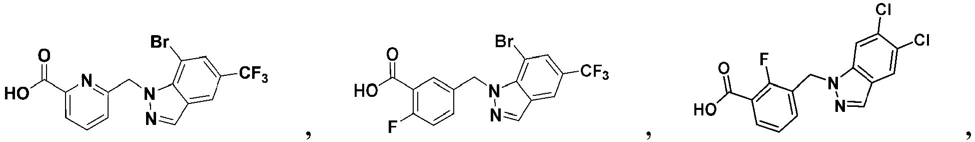 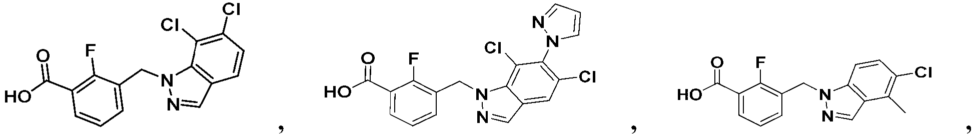 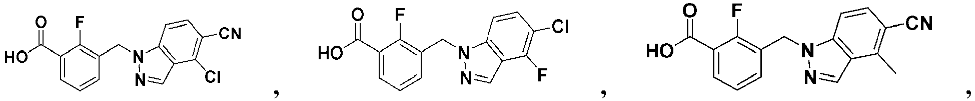 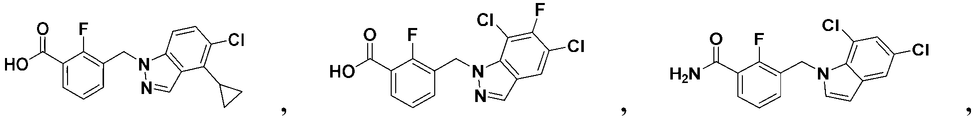 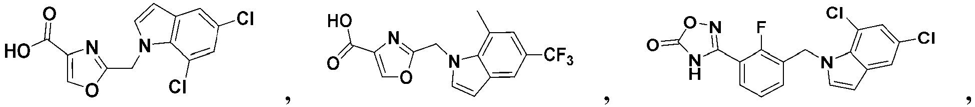 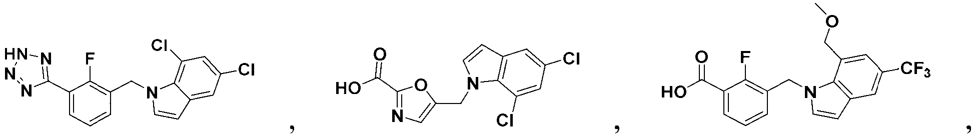 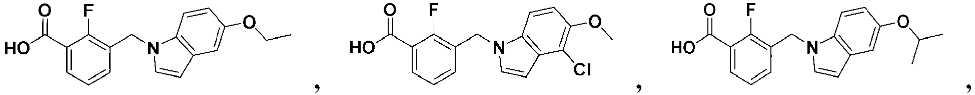 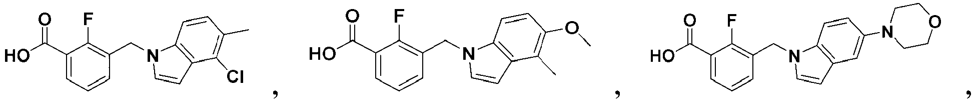 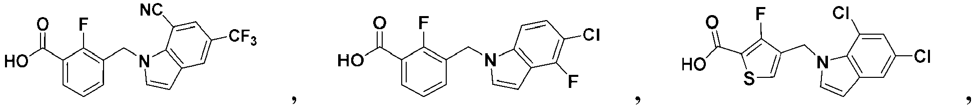 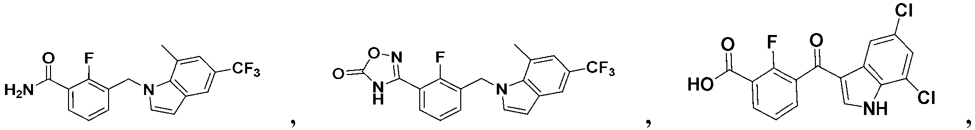 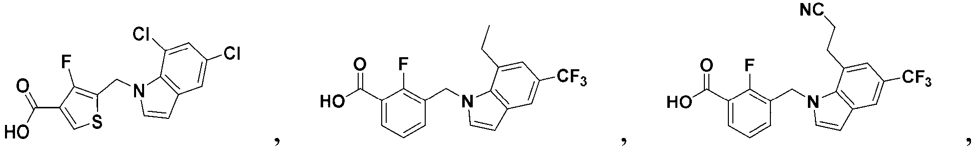 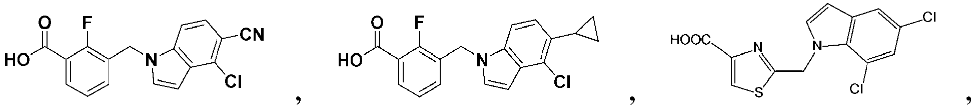 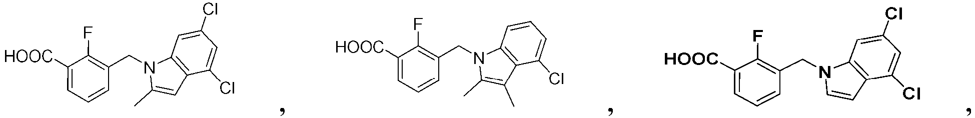 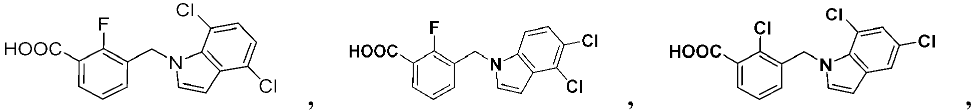 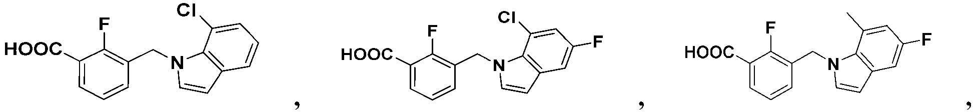 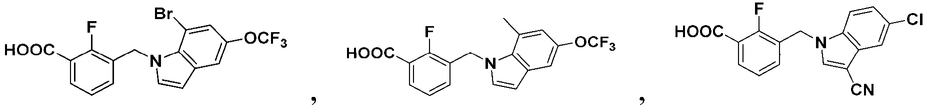 or

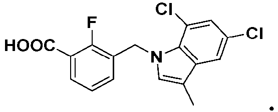

One or more embodiments of the present invention further provide a pharmaceutical composition comprising: the compound, or the stereoisomer, deuterated compound, pharmaceutically acceptable salt, solvate, prodrug, metabolite, or co-crystal thereof, as described in one or more embodiments of the present invention; and one or more pharmaceutically acceptable carriers and/or excipients.

One or more embodiments of the present invention further provide the use of the pharmaceutical composition as described in one or more embodiments of the present invention, or the compound, or the stereoisomer, deuterated compound, pharmaceutically acceptable salt, solvate, prodrug, metabolite, or co-crystal thereof, as described in one or more embodiments of the present invention, as a medicament (i.e., for a therapeutic use).

One or more embodiments of the present invention further provide the use of the pharmaceutical composition according to one or more embodiments of the present invention, or the compound, or the stereoisomer, deuterated compound, pharmaceutically acceptable salt, solvate, prodrug, metabolite, or co-crystal thereof, as described in one or more embodiments of the present invention, in the preparation of a medicament for treating an MRGPRX4-dependent condition.

One or more embodiments of the present invention further provide a method for treating an MRGPRX4-dependent condition, comprising administering to an individual in need thereof a therapeutically effective dose of the pharmaceutical composition as described in one or more embodiments of the present invention, or the compound, or the stereoisomer, deuterated compound, pharmaceutically acceptable salt, solvate, prodrug, metabolite, or co-crystal thereof, as described in one or more embodiments of the present invention.

In one or more embodiments of the present invention, the MRGPRX4-dependent condition is a pruritus-related condition, a pain-related condition, or an autoimmune condition.

In one or more embodiments of the present invention, the pruritus-related condition is pruritus caused by diseases such as chronic pruritus, cholestatic pruritus, contact dermatitis, allergic inflammation, atopic dermatitis, cholestasis, end-stage kidney disease, hemodialysis, eczematous dermatitis, or uremia.

In one or more embodiments of the present invention, the pruritus-related condition is cholestatic pruritus, uremic pruritus, chronic inducible urticaria, chronic spontaneous urticaria, or atopic dermatitis.

In one or more embodiments of the present invention, the pain-related condition is pain caused by arthritis, backache, cancer pain, central pain syndrome, acute pain, chronic pain, dermatomyositis, diabetic peripheral neuropathy (DPN), endometriosis, fibromyalgia, leg pain, lumbago-hematuria syndrome, lupus, migraine, musculoskeletal pain, myofascial pain, myositis, neck pain, neuropathic pain, osteoarthritis, postherpetic neuralgia (herpes zoster), psoriatic arthritis, rheumatoid arthritis (RA), sciatica, herpes zoster, trigeminal neuralgia, neuropathic pain, etc.

In one or more embodiments of the present invention, the autoimmune disease is chronic inflammation, multiple sclerosis, dermatitis, rhinitis, tendinitis, asthma, autoinflammatory diseases, or allergic reaction.

Unless stated to the contrary, the terms used in the specification and claims have the following meanings.

The carbon, hydrogen, oxygen, sulfur, nitrogen, F, Cl, Br, and I involved in the groups and compounds described in the present invention all include isotopes thereof, and the carbon, hydrogen, oxygen, sulfur, or nitrogen involved in the groups and compounds described in the present invention is optionally further replaced by one or more corresponding isotopes thereof, wherein isotopes of carbon include ¹²C, ¹³C, and ¹⁴C, isotopes of hydrogen include protium (H), deuterium (D, also called heavy hydrogen), and tritium (T, also called superheavy hydrogen), isotopes of oxygen include ¹⁶O, ¹⁷O, and ¹⁸O, isotopes of sulfur include ³²S, ³³S, ³⁴S, and ³⁶S, isotopes of nitrogen include ¹⁴N and ¹⁵N, isotopes of fluorine include ¹⁷F and ¹⁹F, isotopes of chlorine include ³⁵Cl and ³⁷Cl, and isotopes of bromine include ⁷⁹Br and ⁸¹Br.

"Alkylene" refers to a divalent group derived from an alkane, which can be linear or branched.

"Alkyl" refers to a linear or branched saturated aliphatic hydrocarbon group having 1 to 20 carbon atoms, preferably an alkyl group having 1 to 8 (e.g., 1, 2, 3, 4, 5, 6, 7, or 8) carbon atoms, more preferably an alkyl group having 1 to 6 carbon atoms, further preferably an alkyl group having 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl, and various branched isomers thereof. When the alkyl is substituted, it may be optionally further substituted with 1 or more substituents.

"Alkoxy" refers to a group formed by the substitution of at least 1 carbon atom of an alkyl group with an oxygen atom. Non-limiting examples include methoxy, ethoxy, n-propoxy, isopropoxy, n-butoxy, sec-butoxy, tert-butoxy, n-pentoxy, n-hexoxy, cyclopropoxy, and cyclobutoxy. The alkyl is defined in the same way as for the "alkyl" described above.

"Alkenyl" refers to a linear or branched unsaturated aliphatic hydrocarbon group containing 1 to 10 (e.g., 1, 2, 3, 4, 5, 6, 7, 8, 9, or 10) carbon-carbon double bonds and consisting of 2 to 20 carbon atoms, preferably an alkenyl group having 2 to 12 (e.g., 2, 3, 4, 5, 6, 7, 8, 9, 10, 11, or 12) carbon atoms, more preferably an alkenyl group having 2 to 8 carbon atoms, further preferably an alkenyl group having 2 to 6 carbon atoms. Non-limiting examples include vinyl, propen-2-yl, buten-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl, and undecen-3-yl. The alkenyl may be optionally further substituted with 1 or more substituents.

"Alkynyl" refers to a linear or branched unsaturated aliphatic hydrocarbon group containing 1 to 10 (e.g., 1, 2, 3, 4, 5, 6, 7, 8, 9, or 10) carbon-carbon triple bonds and consisting of 2 to 20 carbon atoms, preferably an alkynyl group having 2 to 12 (e.g., 2, 3, 4, 5, 6, 7, 8, 9, 10, 11, or 12) carbon atoms, more preferably an alkynyl group having 2 to 8 carbon atoms, further preferably an alkynyl group having 2 to 6 carbon atoms. Non-limiting examples include ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyn-3-yl, nonyn-3-yl, decyn-4-yl, undec-3-yl, and dodecyn-4-yl. The alkynyl may be optionally further substituted with 1 or more substituents.

"Aryl" refers to a substituted or unsubstituted aromatic ring, which can be a 5- to 8-membered (e.g., 5, 6, 7, or 8-membered) monocyclic, 5- to 12-membered (e.g., 5, 6, 7, 8, 9, 10, 11, or 12 membered) bicyclic, or 10- to 15-membered (e.g., 10, 11, 12, 13, 14, or 15-membered) tricyclic ring system which may be a bridged ring or a spiro ring. Non-limiting examples include phenyl and naphthyl. The aryl may be optionally further substituted with 1 or more substituents.

"Heteroaryl" refers to a substituted or unsubstituted aromatic ring, which can be a 3- to 8-membered (e.g., 3, 4, 5, 6, 7, or 8-membered) monocyclic, 5- to 12-membered (e.g., 5, 6, 7, 8, 9, 10, 11, or 12-membered) bicyclic, or 10- to 15-membered (e.g., 10, 11, 12, 13, 14, or 15-membered) tricyclic ring system containing 1 to 6 (e.g., 1, 2, 3, 4, 5, or 6) heteroatoms selected from N, O and S, preferably a 5- to 8-membered heteroaryl, wherein 1 to 4 (e.g., 1, 2, 3, or 4) N and S selectively substituted in the ring of the heteroaryl can be oxidized into various oxidation states. The heteroaryl can be attached to a heteroatom or a carbon atom, and the heteroaryl can be a bridged ring or a spiro ring. Non-limiting examples include cyclic pyridinyl, furanyl, thienyl, pyranyl, pyrrolyl, pyrimidinyl, pyrazinyl, pyridazinyl, imidazolyl, piperidinyl, benzimidazolyl, benzopyridinyl, and pyrrolopyridinyl. Heteroaryl is optionally further substituted with 1 or more substituents.

"Heterocyclyl" or "heterocycle" refers to a saturated or unsaturated aromatic heterocyclic or non-aromatic heterocyclic ring. When it is an aromatic heterocyclic ring, the definition thereof is the same as that of "heteroaryl" above; and when it is a non-aromatic heterocyclic ring, it may be a 3- to 10-membered (e.g., 3, 4, 5, 6, 7, 8, 9, or 10-membered) monocyclic, 4- to 12-membered (e.g., 4, 5, 6, 7, 8, 9, 10, 11, or 12-membered) bicyclic, or 10- to 15-membered (e.g., 10, 11, 12, 13, 14, or 15 membered) tricyclic ring system containing 1 to 4 (e.g., 1, 2, 3, or 4) heteroatoms selected from N, O, or S, preferably 3- to 8-membered heterocyclyl. 1 to 4 (e.g., 1, 2, 3, or 4) N and S optionally substituted in the ring of the "heterocyclyl" or "heterocycle" can be oxidized into various oxidation states; "heterocyclyl" or "heterocycle" may be attached to a heteroatom or a carbon atom; and "heterocyclyl" or "heterocycle" may be a bridged ring or a spiro ring. Non-limiting examples of "heterocyclyl" or "heterocycle" include epoxyethyl, epoxypropyl, aziridinyl, oxetanyl, azetidinyl, thietanyl, 1,3-dioxolanyl, 1,4-dioxolanyl, 1,3-dioxanyl, azepanyl, oxepanyl, thiepanyl, oxoazepinyl, diazepinyl, thiazepinyl, pyridinyl, homopiperidinyl, furanyl, thienyl, pyranyl, N-alkylpyrrolyl, pyrimidinyl, pyrazinyl, pyridazinyl, piperazinyl, homopiperazinyl, imidazolyl, piperidinyl, morpholinyl, thiomorpholinyl, oxathianyl, 1,3-dithianyl, dihydrofuranyl, dithiacyclopentyl, tetrahydrofuranyl, tetrahydrothienyl, tetrahydropyridyl, tetrahydrothiopyranyl, tetrahydropyrrolyl, tetrahydroimidazolyl, tetrahydrothiazolyl, tetrahydropyranyl, benzimidazolyl, benzopyridinyl, pyrrolopyridinyl, pyrazolopyrimidinyl, imidazopyrazinyl, benzodihydrofuranyl, 2-pyrrolinyl, 3-pyrrolinyl, indolinyl, 2H-pyranyl, 4H-pyranyl, dioxacyclohexyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3H-indolylquinolizinyl, N-pyridylurea, 1,1-dioxothiomorpholinyl, azabicyclo[3.2.1]octyl, azabicyclo[5.2.0]nonyl, oxatricyclo[5.3.1.1]dodecyl, aza-adamantyl, and oxaspiro[3.3]heptyl. The "heterocyclyl" or "heterocycle" may be optionally further substituted with 1 or more substituents.

"Cycloalkyl" refers to a saturated cyclic hydrocarbon group, which may be a 3- to 10-membered (e.g., 3, 4, 5, 6, 7, 8, 9, or 10-membered) monocyclic, 4- to 12-membered (e.g., 4, 5, 6, 7, 8, 9, 10, 11, or 12-membered) bicyclic, or 10- to 20-membered (e.g., 10, 11, 12, 13, 14, 15, 16, 17, 18, 19, or 20-membered) polycyclic ring system, wherein the ring carbon atoms are preferably 3 to 10 carbon atoms, further preferably 3 to 8 carbon atoms. Non-limiting examples of "cycloalkyl" include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl, cycloheptatrienyl, etc. When the cycloalkyl is substituted, it may be optionally further substituted with 1 or more substituents.

"Heterocycloalkyl" refers to a substituted or unsubstituted saturated non-aromatic cyclic group, which may be a 3- to 8-membered (e.g., 3, 4, 5, 6, 7, 8-membered) monocyclic, 4- to 12-membered (e.g., 4, 5, 6, 7, 8, 9, 10, 11, 12-membered) bicyclic, or 10- to 15-membered (e.g., 10, 11, 12, 13, 14, 15-membered) bicyclic ring system containing 1, 2, or 3 heteroatoms selected from N, O, or S, preferably 3- to 8-membered heterocyclyl. 1, 2 or 3 N and S optionally substituted in the ring of "heterocycloalkyl" can be oxidized into various oxidation states; "heterocycloalkyl" may be attached to a heteroatom or a carbon atom; and "heterocycloalkyl" may be a bridged ring or a spiro ring. Non-limiting examples of "heterocycloalkyl" include epoxyethyl, aziridinyl, oxetanyl, azetidinyl, 1,3-dioxolanyl, 1,4-dioxolanyl, 1,3-dioxanyl, azepanyl, piperidinyl, morpholinyl, thiomorpholinyl, 1,3-dithianyl, tetrahydrofuranyl, tetrahydropyrrolyl, tetrahydroimidazolyl, tetrahydrothiazolyl, tetrahydropyranyl, azabicyclo[3.2.1]octyl, azabicyclo[5.2.0]nonyl, oxatricyclo[5.3.1.1]dodecyl, aza-adamantyl, and oxaspiro[3.3]heptyl.

When the "alkyl", "alkoxy", "alkenyl", "alkynyl", "aryl", " heteroaryl", "carbocyclyl", "carbocycle", "heterocyclyl", "heterocycle", "cycloalkyl", and "heterocycloalkyl" described above are substituted, they may be further substituted with 0, 1, 2, 3, 4, 5, 6, 7, 8, 9, or 10 substituents selected from F, Cl, Br, I, hydroxyl, mercapto, nitro, cyano, amino, C₁₋₆ alkylamino, =O, C₁₋₆ alkyl, C₁₋₆ alkoxy, C₂₋₆ alkenyl, C₂₋₆ alkynyl, -NR^{q4}R^{q5}, =NR^{q6}, -C(=O)OC₁₋₆ alkyl, - OC(=O)C₁₋₆ alkyl, -C(=O)NR^{q4}R^{q5}, C₃₋₈ cycloalkyl, C₃₋₈ heterocycloalkyl, C₆₋₁₀ aryl, C₅₋₁₀ heteroaryl, -C(=O)OC₆₋₁₀ aryl, -OC(=O)C₆₋₁₀ aryl, -OC(=O)C₅₋₁₀ heteroaryl, -C(=O)OC₅₋₁₀ heteroaryl, -OC(=O)C₃₋₈ heterocycloalkyl, -C(=O)OC₃₋₈ heterocycloalkyl, -OC(=O)C₃₋₈ cycloalkyl, -C(=O)OC₃₋₈ cycloalkyl, -NHC(=O)C₃₋₈ heterocycloalkyl, -NHC(=0)C₆₋₁₀ aryl, - NHC(=O)C₅₋₁₀ heteroaryl, -NHC(=O)C₃₋₈ cycloalkyl, -NHC(=O)C₃₋₈ heterocycloalkyl, - NHC(=O)C₂₋₆ alkenyl, or -NHC(=O)C₂₋₆ alkynyl, and the C₁₋₆ alkyl, C₁₋₆ alkoxy, C₂₋₆ alkenyl, C₂₋₆ alkynyl, C₃₋₈ cycloalkyl, C₃₋₈ heterocycloalkyl, C₆₋₁₀ aryl, C₅₋₁₀ heteroaryl, -NHC(=O)C₆₋₁₀ aryl, -NHC(=O)C₅₋₁₀ heteroaryl, -NHC(=O)C₃₋₈ heterocycloalkyl, or -NHC(=O)C₃₋₈ cycloalkyl substituent is optionally further substituted with 1 to 3 substituents selected from OH, F, Cl, Br, I, C₁₋₆ alkyl, C₁₋₆ alkoxy, -NR^{q4}R^{q5}, or =O; R^{q1} is selected from C₁₋₆ alkyl, C₁₋₆ alkoxy, or C₆₋₁₀ aryl; R^{q2} and R^{q3} are each independently selected from H or C₁₋₆ alkyl; wherein R^{q4} and R^{q5} are each independently selected from H, C₁₋₆ alkyl, -NH(C=NR^{q1})NR^{q2}R^{q3}, -S(=O)₂NR^{q2}R^{q3}, -C(=O)R^{q1}, or -C(=O)NR^{q2}R^{q3}, wherein the C₁₋₆ alkyl is optionally further substituted with 1 or more substituents selected from OH, F, Cl, Br, I, C₁₋₆ alkyl, C₁₋₆ alkoxy, C₆₋₁₀ aryl, C₅₋₁₀ heteroaryl, C₃₋₈ cycloalkyl, or C₃₋₈ heterocycloalkyl; or R^{q4} and R^{q5}, together with an N atom, form a 3- to 8-membered heterocycle, which may contain 1 or more heteroatoms selected from N, O, or S.

"Pharmaceutical composition" refers to a mixture of one or more of the compounds described in the present invention, or stereoisomers, deuterated compounds, pharmaceutically acceptable salts, solvates, prodrugs, metabolites, co-crystals, etc. thereof and other chemical components, wherein the "other chemical components" refer to pharmaceutically acceptable carriers, excipients and/or one or more other therapeutic agents.

"Carrier" refers to a material that does not cause significant irritation to an organism and does not eliminate the biological activity and characteristics of the administered compound.

"Excipient" refers to an inert substance added to a pharmaceutical composition to facilitate administration of a compound. Non-limiting examples include calcium carbonate, calcium phosphate, sugars, starches, cellulose derivatives (including microcrystalline cellulose), gelatin, vegetable oils, polyethylene glycols, diluents, granulating agents, lubricants, binders, and disintegrants.

"Prodrug" refers to a compound that can be converted into a bioactive compound of the present invention by metabolism in vivo. The prodrug of the present invention is prepared by modifying the amino or carboxyl in the compound of the present invention, wherein the modification can be removed by conventional operation or in vivo to obtain the parent compound. When the prodrug of the present invention is administered to a mammalian individual, the prodrug is cleaved to form free amino or carboxyl.

"Co-crystal" refers to a crystal formed by the combination of an active pharmaceutical ingredient (API) and a co-crystal former (CCF) under the action of hydrogen bonds or other noncovalent bonds, wherein the pure states of the API and CCF are both solid at room temperature, and there is a fixed stoichiometric ratio between the components. Co-crystal is a multi-component crystal, which includes not only a binary co-crystal formed between two neutral solids but also a multi-component co-crystal formed between a neutral solid and a salt or solvate.

"Stereoisomer" refers to isomers resulting from different spatial arrangements of atoms in a molecule, including cis/trans isomers, enantiomers, and conformers.

"Optional", "optionally", "selective", or "selectively" means that the subsequently described event or circumstance may, but not necessarily, occur, and the description includes cases where the event or circumstance occurs and cases where it does not. For example, "heterocyclyl optionally substituted with alkyl" means that the alkyl may be, but not necessarily, present, and the description includes the case where the heterocyclyl is substituted with alkyl and the case where the heterocyclyl is not substituted with alkyl.

### Detailed Description of Embodiments

The technical solution of the present invention will be explained in detail by the following examples; however, the scope of protection of the present invention includes but is not limited thereto.

### Example 1

2-Fluoro-3-((5-(trifluoromethyl)-1H-indazol-1-yl)methyl)benzoic acid **(Compound 1-1)**
2-Fluoro-3-((5-(trifluoromethyl)-1H-indazol-1-yl)methyl)benzoic acid
2-Fluoro-3-((5-(trifluoromethyl)-2H-indazol-2-yl)methyl)benzoic acid **(Compound 1-2)**
2-Fluoro-3-((5-(trifluoromethyl)-2H-indazol-2-yl)methyl)benzoic acid

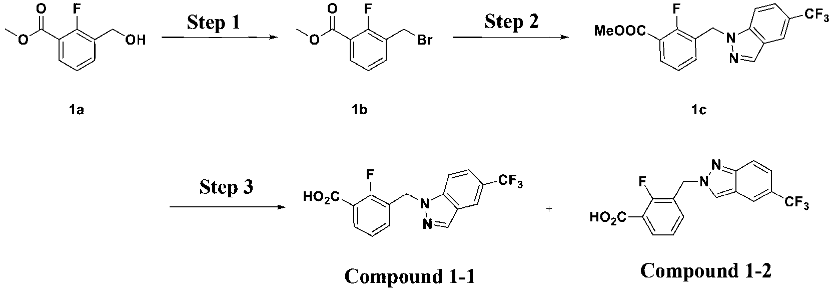

### Step 1:

### Methyl 3-(bromomethyl)-2-fluorobenzoate (1b)

### Methyl 3-(bromomethyl)-2-fluorobenzoate

In a 100 mL round-bottomed flask, **1a** (500 mg, 2.72 mmol) and N-bromosuccinimide (968 mg, 5.44 mmol) were dissolved in anhydrous dichloromethane (15 mL), triphenylphosphine (1.43 g, 5.44 mmol) was added under stirring, and the mixture was reacted for 3 h at room temperature. The reaction was monitored by TLC. The reaction was quenched with water, the reaction liquid was extracted with dichloromethane (20 mL × 3), dried over anhydrous sodium sulfate, and filtered, and the organic solvent was removed under reduced pressure. The residue was purified by column chromatography (PE : EA = 30 : 1) to obtain **1b** as a colorless oil (448 mg, yield: 66.6%).

### Step 2:

### Methyl 2-fluoro-3-((5-(trifluoromethyl)-1H-indazol-1-yl)methyl)benzoate (1c)

### Methyl 2-fluoro-3-((5-(trifluoromethyl)-1H-indazol-1-yl)methyl)benzoate

In a 100 mL round-bottomed flask, **1b** (200 mg, 0.813 mmol), 5-(trifluoromethyl)-1H-imidazole (152 mg, 0.813 mmol), and potassium carbonate (225 mg, 1.63 mmol) were dissolved in acetonitrile (15 mL), and the mixture was reacted under reflux for 3 h. The reaction was complete as monitored by TLC. The reaction liquid was filtered, the organic solvent was removed under reduced pressure, and the residue **(1c)** was directly used for the next step without purification.

### Step 3:

### 2-Fluoro-3-((5-(trifluoromethyl)-1H-indazol-1-yl)methyl)benzoic acid (Compound 1-1) and 2-fluoro-3-((5-(trifluoromethyl)-2H-indazol-2-yl)methyl)benzoic acid (Compound 1-2)

### 2-Fluoro-3-((5-(trifluoromethyl)-1H-indazol-1-yl)methyl)benzoic acid and 2-fluoro-3-((5-(trifluoromethyl)-2H-indazol-2-yl)methyl)benzoic acid

In a 100 mL round-bottomed flask, **1c** (286 mg, 0.813 mmol) was dissolved in methanol/tetrahydrofuran/water (10 : 5 : 2.5 mL), sodium hydroxide (91 mg, 1.63 mmol) was added under stirring, and the mixture was reacted under reflux for 2 h. The reaction was complete as monitored by TLC. The reaction was quenched with water, and the pH was adjusted to acidity with 1N HCl. After filtration, a crude product was obtained, which was subjected to medium-pressure preparative purification to obtain **Compound 1-1** (as a white solid, 62 mg, yield: 22.9%) and **Compound 1-2** (as a white solid, 102 mg, yield: 37.2%).

**Compound 1-1:** ¹H NMR (400 MHz, DMSO-*d*₆) δ 13.36 (s, 1H), 8.76 (s, 1H), 8.26 (s, 1H), 7.84 (m, 1H), 7.79 (d, 1H), 7.57 - 7.49 (m, 1H), 7.45 (dd, 1H), 7.29 (t, 1H), 5.84 (s, 2H); LC-MS m/z (ESI) = 339.1 [M+1].

**Compound 1-2:** ¹H NMR (400 MHz, DMSO-*d*₆) δ 8.30 (s, 1H), 8.25 (s, 1H), 7.98 (d, 1H), 7.79 (m, 1H), 7.71 (dd, 1H), 7.42 - 7.35 (m, 1H), 7.23 (t, 1H), 5.81 (s, 2H).

### Example 2

### 2-Fluoro-3-((5-(trifluoromethyl)indolin-1-yl)methyl)benzoic acid (Compound 2)

### 2-Fluoro-3-((5-(trifluoromethyl)indolin-1-yl)methyl)benzoic acid

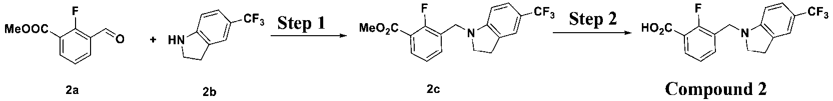

### Step 1:

### Methyl 2-fluoro-3-((5-(trifluoromethyl)indolin-1-yl)methyl)benzoate (2c)

### Methyl 2-fluoro-3-((5-(trifluoromethyl)indolin-1-yl)methyl)benzoate

In a 100 mL round-bottomed flask, **2b** (500 mg, 2.67 mmol), **2a** (536 mg, 2.94 mmol), and sodium triacetylborohydride (1.13 g, 5.34 mmol) were dissolved in anhydrous dichloromethane (20 mL), and the mixture was reacted overnight at room temperature. The reaction was complete as monitored by TLC. The reaction was quenched with water, the reaction liquid was extracted with ethyl acetate (20 mL × 3), dried over anhydrous sodium sulfate, and filtered, and the organic solvent was removed under reduced pressure. The residue was purified by column chromatography (PE : EA = 10 : 1) to obtain **2c** as a yellow solid (869 mg, yield: 92.2%).

### Step 2:

### 2-Fluoro-3-((5-(trifluoromethyl)indol-1-yl)methyl)benzoic acid (Compound 2)

### 2-Fluoro-3-((5-(trifluoromethyl)indolin-1-yl)methyl)benzoic acid

In a 100 mL round-bottomed flask, **2c** (869 mg, 2.46 mmol) was dissolved in methanol/tetrahydrofuran/water (10 : 5 : 2.5 mL), sodium hydroxide (275 mg, 4.92 mmol) was added under stirring, and the mixture was reacted under reflux for 2 h. The reaction was complete as monitored by TLC. The reaction was quenched with water, and the pH was adjusted to acidity with 1N HCl. The resulting material was filtered to obtain **Compound 2** as a white solid (306 mg, yield: 36.6%).

**Compound 2:** ¹H NMR (400 MHz, DMSO-*d*₆) δ 13.34 (s, 1H), 7.78 (t, 1H), 7.59 (t, 1H), 7.38-7.19 (m, 3H), 6.67 (d, 1H), 4.47 (s, 2H), 3.46 (t, 2H), 3.00 (t, 2H); LC-MS m/z (ESI) = 340.1 [M+1].

### Example 3

### 2-Fluoro-3-((6-(trifluoromethyl)-1H-benzo[d]imidazol-1-yl)methyl)benzoic acid (Compound 3-1) and 2-fluoro-3-((5-(trifluoromethyl)-1H-benzo[d]imidazol-1-yl)methyl)benzoic acid (Compound 3-2)

### 2-Fluoro-3-((6-(trifluoromethyl)-1H-benzo[d]imidazol-1-yl)methyl)benzoic acid and 2-fluoro-3-((5-(trifluoromethyl)-1H-benzo[d]imidazol-1-yl)methyl)benzoic acid

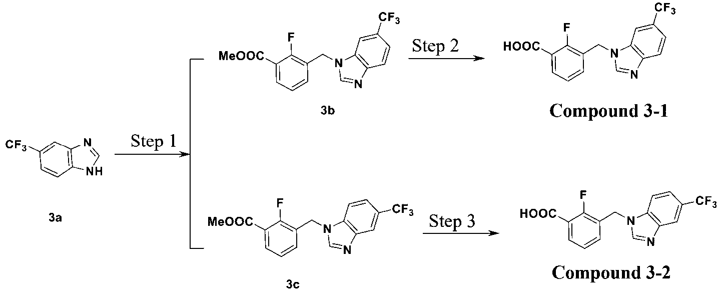

### Step 1:

### Methyl 2-fluoro-3-(6-(trifluoromethyl)-1H-benzo[d]imidazol-1-yl)methyl)benzoate (3b) and methyl 2-fluoro-3-(5-(trifluoromethyl)-1H-benzo[d]imidazol-1-yl)methyl)benzoate (3c)

### Methyl 2-fluoro-3-((6-(trifluoromethyl)-1H-benzo[d]imidazol-1-yl)methyl)benzoate and methyl 2-fluoro-3-((5-(trifluoromethyl)-1H-benzo[d]imidazol-1-yl)methyl)benzoate

In a 50 mL single-necked flask, methyl 3-(bromomethyl)-2-fluorobenzoate (200 mg, 0.81 mmol) and **3a** (166 mg, 0.89 mmol) were dissolved in 4 mL of acetonitrile, and potassium carbonate (224 mg, 1.62 mmol) was added. The reaction was carried out at 60°C for 5 h. The reaction was complete as monitored by TLC. The reaction liquid was filtered, and the organic solvent was removed under reduced pressure. The crude product was purified by preparative thin-layer chromatography (PE : EA = 3 : 1) to obtain **3b** as a white solid (120 mg, yield: 42.8%) and **3c** as a white solid (90 mg, yield: 31.4%).

LCMS m/z (ESI) = 353.1 [M+1].

### Step 2:

### 2-Fluoro-3-((6-(trifluoromethyl)-1H-benzo[d]imidazol-1-yl)methyl)benzoic acid (Compound 3-1)

### 2-Fluoro-3-((6-(trifluoromethyl)-1H-benzo[d]imidazol-1-yl)methyl)benzoic acid

In a 50 mL three-necked flask, **3b** (120 mg, 0.4 mmol) was dissolved in a mixed solvent of methanol (1 mL), tetrahydrofuran (1 mL), and water (1 mL), and sodium hydroxide (32 mg, 0.8 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by preparative thin-layer chromatography (DCM : MeOH = 10 : 1) to obtain **Compound 3-1** as a white solid (10 mg, yield: 9.81%).

LCMS m/z (ESI) = 339.1 [M+1].

¹H NMR (400 MHz, DMSO-*d*₆) δ 8.54 (s, 1H), 8.03 (d, 1H), 7.76 (d, 1H), 7.57 (dd, 1H), 7.39 (td, 1H), 7.11 (td, 1H), 6.98 (t, 1H), 5.58 (s, 2H).

¹⁹F NMR (376 MHz, DMSO-*d*₆) δ -58.83, -120.06.

### Step 3:

### 2-Fluoro-3-((5-(trifluoromethyl)-1H-benzo[d]imidazol-1-yl)methyl)benzoic acid (Compound 3-2)

### 2-Fluoro-3-((5-(trifluoromethyl)-1H-benzo[d]imidazol-1-yl)methyl)benzoic acid

In a 50 mL three-necked flask, **3c** (90 mg, 0.28 mmol) was dissolved in a mixed solvent of methanol (1 mL), tetrahydrofuran (1 mL), and water (1 mL), and sodium hydroxide (23 mg, 0.57 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by preparative thin-layer chromatography (DCM : MeOH = 10 : 1) to obtain **Compound 3-2** as a white solid (30 mg, yield: 34.15%).

LCMS m/z (ESI) = 339.1 [M+1].

¹H NMR (400 MHz, DMSO-*d*₆) δ 8.54 (s, 1H), 8.03 (s, 1H), 7.86 (d, 1H), 7.52 (dd, 1H), 7.41 (td, 1H), 7.15 (td, 1H), 7.00 (t, 1H), 5.62 (s, 2H).

¹⁹F NMR (376 MHz, DMSO-*d*₆) δ -58.85, -119.76.

### Example 4

### 2-Fluoro-3-(5-(trifluoromethyl)-1H-benzo[d][1,2,3]triazol-1-yl)methyl)benzoic acid (Compound 4)

### 2-Fluoro-3-((5-(trifluoromethyl)-1H-benzo[d][1,2,3]triazol-1-yl)methyl)benzoic acid

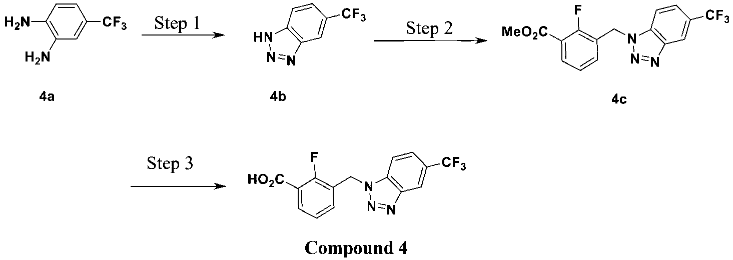

### Step 1:

### 5-(Trifluoromethyl)-1H-benzo[d][1,2,3]triazole (4b)

### 5-(Trifluoromethyl)-1H-benzo[d][1,2,3]triazole

In a 50 mL single-necked flask, **4a** (500 mg, 2.84 mmol) was dissolved in 2 mL of acetic acid and 4 mL of water. The mixture was cooled to 0°C. Sodium nitrite (235 mg, 3.40 mmol) was dissolved in 2 mL of water and then dropwise added to the reaction liquid, and the mixture was naturally heated to room temperature and reacted for 2 h. The reaction was complete as monitored by TLC. The pH was adjusted to 8 with a saturated aqueous sodium bicarbonate solution, and the resulting material was extracted with EA (20 mL × 3). The organic phases were combined, dried over anhydrous sodium sulfate, and filtered. The organic solvent was removed under reduced pressure, and the crude product was purified by column chromatography (PE : EA = 1 : 0-1 : 1) to obtain **4b** as a yellow solid(570 mg, yield: 87.43%).

¹H NMR (400 MHz, DMSO-*d*₆) δ 16.20 (s, 1H), 8.45 (s, 1H), 8.12 (d, 1H), 7.77 (dd, 1H). Step 2:
Methyl 2-fluoro-3-(5-(trifluoromethyl)-1*H*-benzo[d][1,2,3]triazol-1-yl)methyl)benzoate **(4c)**
Methyl-2-fluoro-3-((5-(trifluoromethyl)-1*H*-benzo[d][1,2,3]triazol-1-yl)methyl)benzoate

In a 50 mL single-necked flask, **4b** (169 mg, 0.89 mmol) and methyl 3-(bromomethyl)-2-fluorobenzoate (200 mg, 0.81 mmol) were dissolved in 5 mL of acetonitrile, and potassium carbonate (224 mg, 1.62 mmol) was added. The mixture was reacted at 60°C for 5 h. The reaction was complete as monitored by TLC. The reaction liquid was filtered, the organic solvent was removed under reduced pressure, and the crude product was purified by preparative thin-layer chromatography (PE : EA = 1 : 1) to obtain **4c** as a white solid (38 mg, yield: 7.82%).

LCMS m/z (ESI) = 354.1 [M+1].

¹H NMR (400 MHz, DMSO-*d*₆) δ 8.50 (d, 1H), 8.19 (d, 1H), 7.92 (ddd, 1H), 7.80 (td, 1H), 7.70 (dd, 1H), 7.38 (t, 1H), 6.20 (s, 2H), 3.84 (s, 3H).

¹⁹F NMR (376 MHz, DMSO-*d*₆) δ -60.59, -114.82.

### Step 3:

### 2-Fluoro-3-(5-(trifluoromethyl)-1H-benzo[d][1,2,3]triazol-1-yl)methyl)benzoic acid (Compound 4)

### 2-Fluoro-3-((5-(trifluoromethyl)-1H-benzo[d][1,2,3]triazol-1-yl)methyl)benzoic acid

In a 50 mL three-necked flask, **4c** (38 mg, 0.11 mmol) was dissolved in a mixed solvent of methanol (1 mL), tetrahydrofuran (1 mL), and water (1 mL), and sodium hydroxide (9 mg, 0.22 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by preparative thin-layer chromatography (DCM:MeOH = 10 : 1) to obtain **Compound 4** as a white solid (30 mg, yield: 80.39%).

LCMS m/z (ESI) = 340.1 [M+1].

¹H NMR (400 MHz, DMSO-*d*₆) δ 8.56 (d, 1H), 8.08 (d, 1H), 7.89 (dd, 1H), 7.43 (td, 1H), 7.19 (td, 1H), 7.01 (t, 1H), 6.04 (s, 2H).

¹⁹F NMR (376 MHz, DMSO-*d₆*) δ -59.63, -119.60.

### Example 5

### 3-(5,7-Dichloro-1H-indol-1-yl)methyl)-2-fluorobenzoic acid (Compound 5)

### 3-((5,7-Dichloro-1H-indol-1-yl)methyl)-2-fluorobenzoic acid

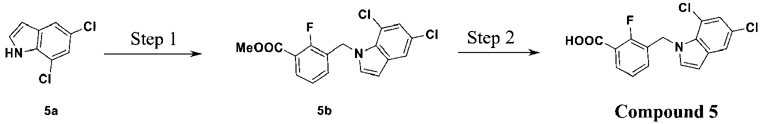

### Step 1:

### Methyl 3-(5,7-dichloro-1H-indol-1-yl)methyl)-2-fluorobenzoate (5b)

### Methyl 3-((5,7-dichloro-1H-indol-1-yl)methyl)-2-fluorobenzoate

In a 50 mL single-necked flask, 5,7-dichloro-1*H*-indole **5a** (38 mg, 0.20 mmol) and methyl 3-(bromomethyl)-2-fluorobenzoate (50 mg, 0.20 mmol) were dissolved in 5 mL of acetonitrile, and cesium carbonate (132 mg, 0.40 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **5b** as a white solid (45 mg, yield: 63.89%).

LCMS m/z (ESI) = 352.0 [M+1].

### Step 2:

### 3-(5,7-Dichloro-1H-indol-1-yl)methyl)-2-fluorobenzoic acid (Compound 5)

### 3-((5,7-Dichloro-1H-indol-1-yl)methyl)-2-fluorobenzoic acid

In a 50 mL single-necked flask, **5b** (45 mg, 0.13 mmol) was dissolved in a mixed solvent of methanol (1 mL), tetrahydrofuran (1 mL), and water (1 mL), and sodium hydroxide (5 mg, 0.26 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 5** as a white solid (20 mg, yield: 45.50%).

¹H NMR (400 MHz, DMSO-*d*₆) δ 13.35 (s, 1H), 7.74 (td, 1H), 7.69 (d, 1H), 7.64 (d, 1H), 7.22 (d, 1H), 7.15 (t, 1H), 6.66 (d, 1H), 6.56 (t, 1H), 5.86 (s, 2H).

¹⁹F NMR (376 MHz, DMSO-*d*₆) δ -116.62.

### Example 6

### 3-(5,6-Dichloro-1H-indol-1-yl)methyl)-2-fluorobenzoic acid (Compound 6)

### 3-((5,6-Dichloro-1H-indol-1-yl)methyl)-2-fluorobenzoic acid

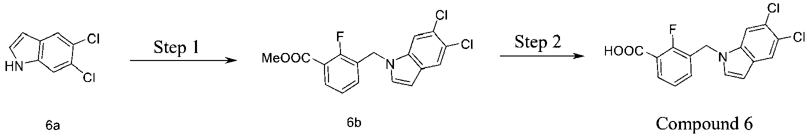

### Step 1:

### Methyl 3-(5,6-dichloro-1H-indol-1-yl)methyl)-2-fluorobenzoate (6b)

### Methyl 3-((5,6-dichloro-1H-indol-1-yl)methyl)-2-fluorobenzoate

In a 50 mL single-necked flask, 5,6-dichloroindole **6a** (40 mg, 0.21 mmol) and methyl 3-(bromomethyl)-2-fluorobenzoate (50 mg, 0.20 mmol) were dissolved in 5 mL of acetonitrile, and cesium carbonate (130 mg, 0.40 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **6b** as a white solid (63 mg, yield: 89.44%).

LCMS m/z (ESI) = 352.0 [M+1].

### Step 2:

### 3-(5,6-Dichloro-1H-indol-1-yl)methyl)-2-fluorobenzoic acid (Compound 6)

### 3-((5,6-Dichloro-1H-indol-1-yl)methyl)-2-fluorobenzoic acid

In a 50 mL single-necked flask, **6b** (63 mg, 0.13 mmol) was dissolved in a mixed solvent of methanol (1 mL), tetrahydrofuran (1 mL), and water (1 mL), and sodium hydroxide (14 mg, 0.36 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 6** as a white solid (20 mg, yield: 32.86%).

¹H NMR (400 MHz, DMSO-*d*₆) δ 13.42 (s, 1H), 7.89 (d, 1H), 7.84 (s, 1H), 7.72 (td, 1H), 7.55 (d, 1H), 7.23 - 7.08 (m, 2H), 6.53 (dd, 1H), 5.54 (s, 2H).

¹⁹F NMR (376 MHz, DMSO-*d*₆) δ -115.66.

### Example 7

### 3-(6,7-Dichloro-1H- indol-1-yl)methyl)-2-fluorobenzoic acid (Compound 7)

### 3-((6,7-Dichloro-1H-indol-1-yl)methyl)-2-fluorobenzoic acid

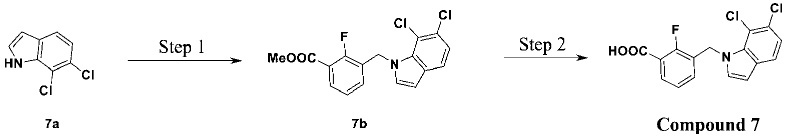

### Step 1:

### Methyl 3-(6,7-dichloro-1H-indol-1-yl)methyl)-2-fluorobenzoate (7b)

### Methyl 3-((6,7-dichloro-1H-indol-1-yl)methyl)-2-fluorobenzoate

In a 50 mL single-necked flask, 6,7-dichloro-1H-indole **7a** (40 mg, 0.21 mmol) and methyl 3-(bromomethyl)-2-fluorobenzoate (50 mg, 0.20 mmol) were dissolved in 5 mL of acetonitrile, and cesium carbonate (130 mg, 0.40 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **7b** as a white solid (54 mg, yield: 76.66%).

LCMS m/z (ESI) = 352.0 [M+1].

### Step 2:

### 3-(6,7-Dichloro-1H- indol-1-yl)methyl)-2-fluorobenzoic acid (Compound 7)

### 3-((6,7-Dichloro-1H-indol-1-yl)methyl)-2-fluorobenzoic acid

In a 50 mL single-necked flask, **7b** (54 mg, 0.15 mmol) was dissolved in a mixed solvent of methanol (1 mL), tetrahydrofuran (1 mL), and water (1 mL), and sodium hydroxide (12 mg, 0.31 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 7** as a white solid (10 mg, yield: 19.71%).

¹H NMR (400 MHz, DMSO-*d*₆) δ 7.70 (s, 1H), 7.65 - 7.55 (m, 2H), 7.26 (d, 1H), 7.11 (t, 1H), 6.68 (d, 1H), 6.51 (t, 1H), 5.88 (s, 2H).

¹⁹F NMR (376 MHz, DMSO-*d*₆) δ -117.04.

### Example 8

### 3-(7-Bromo-5-chloro-1H-indol-1-yl)methyl)-2-fluorobenzoic acid (Compound 8)

### 3-((7-Bromo-5-chloro-1H-indol-1-yl)methyl)-2-fluorobenzoic acid

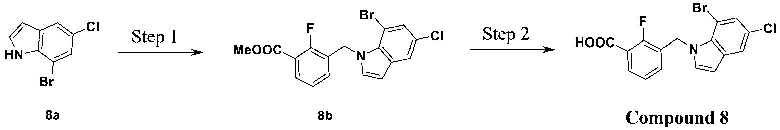

### Step 1:

### Methyl 3-(7-bromo-5-chloro-1H-indol-1-yl)methyl)-2-fluorobenzoate (8b)

### Methyl 3-((7-bromo-5-chloro-1H-indol-1-yl)methyl)-2-fluorobenzoate

In a 50 mL single-necked flask, 7-bromo-5-chloro-indole **8a** (187 mg, 0.81 mmol) and methyl 3-(bromomethyl)-2-fluorobenzoate (200 mg, 0.81 mmol) were dissolved in 5 mL of acetonitrile, and cesium carbonate (528 mg, 1.62 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **8b** as a white solid (204 mg, yield: 63.50%).

¹H NMR (400 MHz, DMSO-*d*₆) δ 7.85 - 7.71 (m, 2H), 7.64 (d, 1H), 7.36 (d, 1H), 7.17 (t, 1H), 6.66 (d, 1H), 6.57 - 6.46 (m, 1H), 5.91 (s, 2H), 3.87 (s, 3H).

¹⁹F NMR (376 MHz, DMSO-*d*₆) δ -116.24.

### Step 2:

### 3-(7-Bromo-5-chloro-1H-indol-1-yl)methyl)-2-fluorobenzoic acid (Compound 8)

### 3-((7-Bromo-5-chloro-1H-indol-1-yl)methyl)-2-fluorobenzoic acid

In a 50 mL single-necked flask, **8b** (60 mg, 0.15 mmol) was dissolved in a mixed solvent of methanol (1 mL), tetrahydrofuran (1 mL), and water (1 mL), and sodium hydroxide (12 mg, 0.31 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 8** as a white solid (20 mg, yield: 34.85%).

¹H NMR (400 MHz, DMSO-*d*₆) δ 7.76 - 7.68 (m, 2H), 7.64 (d, 1H), 7.37 (d, 1H), 7.11 (t, 1H), 6.65 (d, 1H), 6.48 - 6.36 (m, 1H), 5.89 (s, 2H).

¹⁹F NMR (376 MHz, DMSO-*d*₆) δ -116.75.

### Example 9

### 3-(7-Methyl-5-chloro-1H-indol-1-yl)methyl)-2-fluorobenzoic acid (Compound 9)

### 3-((7-Methyl-5-chloro-1H-indol-1-yl)methyl)-2-fluorobenzoic acid

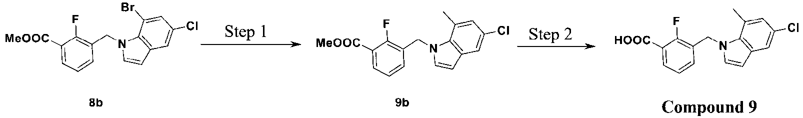

### Step 1:

### Methyl 3-(7-methyl-5-chloro-1H-indol-1-yl)methyl)-2-fluorobenzoate (9b)

### Methyl 3-((7-methyl-5-chloro-1H-indol-1-yl)methyl)-2-fluorobenzoate

In a 50 mL single-necked flask, **8b** (150 mg, 0.38 mmol) and methylboronic acid (46 mg, 0.76 mmol) were dissolved in 5 mL of 1,4-dioxane and 0.5 mL of water, and 1,1-bis(diphenylphosphino)palladium(II) dichloride (30 mg, 0.04 mmol) and cesium carbonate (372 mg, 1.14 mmol) were added. After nitrogen displacement, the mixture was reacted at 90°C for 8 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **9b** as a white solid (113 mg, yield: 89.63%).

LCMS m/z (ESI) =332.1 [M+1].

### Step 2:

### 3-(7-Methyl-5-chloro-1H-indol-1-yl)methyl)-2-fluorobenzoic acid (Compound 9)

### 3-((7-Methyl-5-chloro-1H-indol-1-yl)methyl)-2-fluorobenzoic acid

In a 50 mL single-necked flask, **9b** (110 mg, 0.33 mmol) was dissolved in a mixed solvent of methanol (1 mL), tetrahydrofuran (1 mL), and water (1 mL), and sodium hydroxide (27 mg, 0.66 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 9** as a white solid (80 mg, yield: 76.30%).

¹H NMR (400 MHz, DMSO-*d*₆) δ 7.57 (t, 1H), 7.50 - 7.44 (m, 2H), 6.98 (t, 1H), 6.88 - 6.82 (m, 1H), 6.51 (d, 1H), 6.27 - 6.16 (m, 1H), 5.68 (s, 2H), 2.41 (s, 3H).

¹⁹F NMR (376 MHz, DMSO-*d*₆) δ -115.65.

### Example 10

### 3-(7-Chloro-5-(trifluoromethyl)-1H-indol-1-yl)methyl)-2-fluorobenzoic acid (Compound 10)

### 3-((7-Chloro-5-(trifluoromethyl)-1H-indol-1-yl)methyl)-2-fluorobenzoic acid

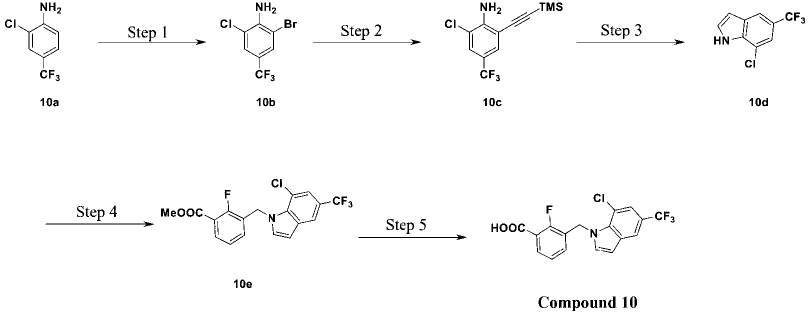

### Step 1:

### 2-Bromo-6-chloro-4-trifluoromethyl-aniline (10b)

### 2-Bromo-6-chloro-4-(trifluoromethyl)aniline

In a 250 mL single-necked flask, **10a** (8.78 g, 45.02 mmol) was dissolved in 100 mL of acetonitrile, and N-bromosuccinimide (16.03 g, 90.04 mmol) was added. The mixture was reacted at room temperature for 5 h. The reaction was complete as monitored by TLC. The reaction was quenched with an excessive amount of a saturated aqueous sodium sulfite solution and extracted with EA (50 mL × 3). The organic phases were combined, dried over anhydrous sodium sulfate, and filtered, and the organic solvent was removed under reduced pressure. The crude product was purified by silica gel column chromatography (PE : EA = 1 : 0-10 : 1) to obtain **10b** as a yellow liquid (11 g, yield: 89.02%).

¹H NMR (400 MHz, DMSO-*d*₆) δ 7.73 (d, 1H), 7.65 (d, 1H), 6.19 (s, 2H).

### Step 2:

### 2-Chloro-4-(trifluoromethyl)-6-(trimethylsilyl)ethynyl)aniline (10c)

### 2-Chloro-4-(trifluoromethyl)-6-((trimethylsilyl)ethynyl)aniline

In a 100 mL three-necked flask, **10b** (5 g, 18.22 mmol) and trimethylsilylethyne (2.24 g, 22.77 mmol) were dissolved in triethylamine (50 mL), and bis(triphenylphosphine)palladium dichloride (256 mg, 0.36 mmol), cuprous iodide (73 mg, 0.72 mmol), and triphenylphosphine (189 mg, 0.72 mmol) were added. After nitrogen displacement, the mixture was reacted at 80°C for 8 h. The reaction was complete as monitored by TLC. The reaction liquid was filtered, and the organic solvent was removed under reduced pressure. The crude product was purified by silica gel column chromatography (PE : EA = 1 : 0-20 : 1) to obtain **10c** as a yellow liquid (3.56 g, yield: 66.97%).

LCMS m/z (ESI) = 292.1 [M+1].

### Step 3:

### 7-Chloro-5-trifluoromethyl-1H-indole (10d)

### 7-Chloro-5-(trifluoromethyl)-1H-indole

In a 50 mL single-necked flask, **10c** (1 g, 3.43 mmol) was dissolved in N-methylpyrrolidone (5 mL), and potassium tert-butoxide (769 mg, 6.85 mmol) was added. The mixture was reacted at room temperature for 8 h. The reaction was complete as monitored by LCMS. The reaction liquid was diluted with EA (100 mL). The organic phase was washed with water (20 mL × 3). The organic phases were dried over anhydrous sodium sulfate and filtered, and the solvent was removed under reduced pressure. The crude product was purified by silica gel column chromatography (PE : EA = 1 : 0-20 : 1) to obtain **10d** as a light yellow liquid (506 mg, yield: 67.18%).

¹H NMR (400 MHz, DMSO-*d*₆) δ 11.99 (s, 1H), 7.97 (s, 1H), 7.61 (t, 1H), 7.49 (d, 1H), 6.73 (dd, 1H).

### Step 4:

### Methyl 3-(7-chloro-5-(trifluoromethyl)-1H-indol-1-yl)methyl) -2-fluorobenzoate (10e)

### Methyl 3-((7-chloro-5-(trifluoromethyl)-1H-indol-1-yl)methyl)-2-fluorobenzoate

In a 50 mL single-necked flask, **10d** (99 mg, 0.45 mmol) and methyl 3-(bromomethyl)-2-fluorobenzoate (100 mg, 0.40 mmol) were dissolved in 5 mL of acetonitrile, and potassium carbonate (111 mg, 0.80 mmol) was added. The mixture was reacted at 60°C for 5 h. The reaction was complete as monitored by TLC. The reaction liquid was filtered, the organic solvent was removed under reduced pressure, and the crude product was purified by preparative thin-layer chromatography (PE : EA = 10 : 1) to obtain **10e** as a white solid (46 mg, yield: 29.81%).

¹H NMR (400 MHz, DMSO-*d*₆) δ 8.05 (dd, 1H), 7.84 - 7.72 (m, 2H), 7.45 (d, 1H), 7.19 (t, 1H), 6.85 (d, 1H), 6.66 (td, 1H), 5.94 (s, 2H), 3.87 (s, 3H).

¹⁹F NMR (376MHz, DMSO-*d*₆) δ -59.06, -116.35.

### Step 5:

### 3-(7-Chloro-5-(trifluoromethyl)-1H-indol-1-yl)methyl)-2-fluorobenzoic acid (Compound 10)

### 3-((7-Chloro-5-(trifluoromethyl)-1H-indol-1-yl)methyl)-2-fluorobenzoic acid

In a 50 mL three-necked flask, **10e** (46 mg, 0.12 mmol) was dissolved in a mixed solvent of methanol (1 mL), tetrahydrofuran (1 mL), and water (1 mL), and sodium hydroxide (10 mg, 0.24 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 10** as a white solid (20 mg, yield: 44.84%).

¹H NMR (400 MHz, DMSO-*d*₆) δ 13.19 (s, 1H), 8.04 (d, 1H), 7.74 (t, 2H), 7.45 (d, 1H), 7.15 (t, 1H), 6.85 (d, 1H), 6.64 - 6.56 (m, 1H), 5.93 (s, 2H).

¹⁹F NMR (376 MHz, DMSO-*d*₆) δ -59.06, -116.60.

### Example 11

### 6-(7-Chloro-5-(trifluoromethyl)-1H-indol-1-yl)methyl)picolinic acid (Compound 11)

### 6-((7-Chloro-5-(trifluoromethyl)-1H-indol-1-yl)methyl)picolinic acid

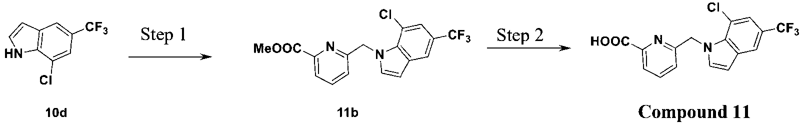

### Step 1:

### Methyl 6-(7-chloro-5-(trifluoromethyl)-1H-indol-1-yl)methyl)picolinate (11b)

### Methyl 6-((7-chloro-5-(trifluoromethyl)-1H-indol-1-yl)methyl)picolinate

In a 50 mL single-necked flask, **10d** (105 mg, 0.48 mmol) and methyl 6-(bromomethyl)picolinate (100 mg, 0.43 mmol) were dissolved in 5 mL of acetonitrile, and potassium carbonate (119 mg, 0.86 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-3 : 1) to obtain **11b** as a white solid (118 mg, yield: 74.42%).

¹H NMR (400 MHz, DMSO-*d*₆) δ 8.05 (dd, 1H), 7.97 - 7.86 (m, 2H), 7.79 (d, 1H), 7.43 (d, 1H), 6.86 (d, 1H), 6.74 (dd, 1H), 5.99 (s, 2H), 3.87 (s, 3H).

¹⁹F NMR (376MHz, DMSO-*d*₆) δ -59.03.

### Step 2:

### 6-(7-Chloro-5-(trifluoromethyl)-1H-indol-1-yl)methyl)picolinic acid (Compound 11)

### 6-((7-Chloro-5-(trifluoromethyl)-1H-indol-1-yl)methyl)picolinic acid

In a 50 mL three-necked flask, **11b** (115 mg, 0.32 mmol) was dissolved in a mixed solvent of methanol (1 mL), tetrahydrofuran (1 mL), and water (1 mL), and sodium hydroxide (25 mg, 0.64 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 11** as a white solid (60 mg, yield: 52.86%).

¹H NMR (400 MHz, DMSO-*d*₆) δ 13.24 (s, 1H), 8.05 (d, 1H), 7.95 - 7.84 (m, 2H), 7.80 (d, 1H), 7.43 (d, 1H), 6.86 (d, 1H), 6.73 (dd, 1H), 5.98 (s, 2H).

¹⁹F NMR (376MHz, DMSO-*d*₆) δ -59.03.

### Example 12

### 5-((7-Chloro-5-(trifluoromethyl)-1H-indol-1-yl)methyl)furan-2-carboxylic acid (Compound 12)

### 5-((7-Chloro-5-(trifluoromethyl)-1H-indol-1-yl)methyl)furan-2-carboxylic acid

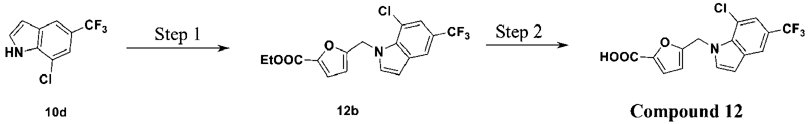

### Step 1:

### Ethyl 5-((7-chloro-5-(trifluoromethyl)-1H-indol-1-yl)methyl)furan-2-carboxylate (12b)

### Ethyl 5-((7-chloro-5-(trifluoromethyl)-1H-indol-1-yl)methyl)furan-2-carboxylate

In a 50 mL single-necked flask, **10d** (70 mg, 0.32 mmol) and ethyl 5-chloromethyl-2-furancarboxylate (50 mg, 0.29 mmol) were dissolved in 5 mL of acetonitrile, and cesium carbonate (189 mg, 0.58 mmol) was added. The mixture was reacted at 60°C for 5 h. The reaction was complete as monitored by TLC. The reaction liquid was filtered, the organic solvent was removed under reduced pressure, and the crude product was purified by preparative thin-layer chromatography (PE : EA = 10 : 1) to obtain **12b** as a white solid (60 mg, yield: 55.66%).

LCMS m/z (ESI) = 372.1 [M+1].

### Step 2:

### 5-((7-Chloro-5-(trifluoromethyl)-1H-indol-1-yl)methyl)furan-2-carboxylic acid (Compound 12)

### 5-((7-Chloro-5-(trifluoromethyl)-1H-indol-1-yl)methyl)furan-2-carboxylic acid

In a 50 mL three-necked flask, **12b** (60 mg, 0.16 mmol) was dissolved in a mixed solvent of methanol (1 mL), tetrahydrofuran (1 mL), and water (1 mL), and sodium hydroxide (13 mg, 0.32 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 12** as a white solid (30 mg, yield: 54.55%).

¹H NMR (400 MHz, DMSO-*d*₆) δ 13.09 (s, 1H), 8.01 (dd, 1H), 7.73 (d, 1H), 7.48 (d, 1H), 7.12 (d, 1H), 6.80 (d, 1H), 6.32 (d, 1H), 5.89 (s, 2H).

¹⁹F NMR (376 MHz, DMSO-*d*₆) δ -59.06.

### Example 13

### 3-((7-Chloro-5-(trifluoromethyl)-1H-indol-1-yl)methyl)benzoic acid (Compound 13)

### 3-((7-Chloro-5-(trifluoromethyl)-1H-indol-1-yl)methyl)benzoic acid

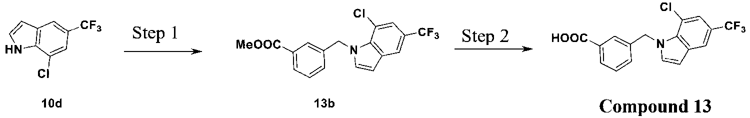

### Step 1:

### Methyl 3-((7-chloro-5-(trifluoromethyl)-1H-indol-1-yl)methyl)benzoate (13b)

### Methyl 3-((7-chloro-5-(trifluoromethyl)-1H-indol-1-yl)methyl)benzoate

In a 50 mL single-necked flask, **10d** (53 mg, 0.24 mmol) and methyl 3-bromomethylbenzoate (50 mg, 0.22 mmol) were dissolved in 5 mL of acetonitrile, and cesium carbonate (142 mg, 0.44 mmol) was added. The mixture was reacted at 60°C for 5 h. The reaction was complete as monitored by TLC. The reaction liquid was filtered, the organic solvent was removed under reduced pressure, and the crude product was purified by preparative thin-layer chromatography (PE : EA = 10 : 1) to obtain **13b** as a white solid (70 mg, yield: 86.52%).

LCMS m/z (ESI) = 368.1 [M+1].

### Step 2:

### 3-((7-Chloro-5-(trifluoromethyl)-1H-indol-1-yl)methyl)benzoic acid (Compound 13)

### 3-((7-Chloro-5-(trifluoromethyl)-1H-indol-1-yl)methyl)benzoic acid

In a 50 mL three-necked flask, **13b** (70 mg, 0.19 mmol) was dissolved in a mixed solvent of methanol (1 mL), tetrahydrofuran (1 mL), and water (1 mL), and sodium hydroxide (15 mg, 0.38 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 13** as a white solid (20 mg, yield: 54.55%).

¹H NMR (400 MHz, DMSO-*d*₆) δ 12.99 (s, 1H), 8.03 (d, 1H), 7.82 (q, 2H), 7.57 (d, 1H), 7.48 - 7.41 (m, 2H), 7.25 (dt, 1H), 6.85 (d, 1H), 5.90 (s, 2H).

¹⁹F NMR (376 MHz, DMSO-*d*₆) δ -59.03.

### Example 14

### 3-(1-(7-Bromo-5-(trifluoromethyl)-1H-indazol-1-yl)ethyl)-2-fluorobenzoic acid (Compound 14)

### 3-(1-(7-Bromo-5-(trifluoromethyl)-1H-indazol-1-yl)ethyl)-2-fluorobenzoic acid

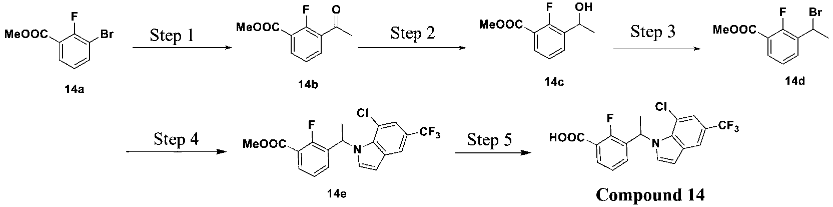

### Step 1:

### Methyl 3-acetyl-2-fluorobenzoate (14b)

### Methyl 3-acetyl-2-fluorobenzoate

In a 100 mL three-necked flask, **14a** (5 g, 21.46 mmol) and tributyl(ethoxyvinyl)tin (15.5 g, 42.92 mmol) were dissolved in toluene (50 mL), and tris(dibenzylideneacetone)dipalladium (1.9 g, 0.21 mmol), 2-dicyclohexylphosphino-2',4',6'-triisopropylbiphenyl (2.05 g, 4.3 mmol) were added. After nitrogen displacement, the mixture was reacted at 100°C for 8 h. The reaction was complete as monitored by LC-MS. The organic solvent was removed under reduced pressure to obtain a crude product. The crude product was dissolved with 50 mL of 1,4-dioxane, and 2M dilute hydrochloric acid (10 mL) was added and stirred for 1 h. The reaction was complete as monitored by TLC. The reaction was quenched by adding a saturated aqueous cesium fluoride solution (10 mL) to the reaction liquid and stirring the mixture for 1 h, and the reaction liquid was extracted with EA (100 mL × 3). The organic phases were combined, dried over anhydrous sodium sulfate, and filtered, and the organic solvent was removed under reduced pressure. The crude product was purified by silica gel column chromatography (PE : EA = 1 : 0-20 : 1) to obtain **14b** as a yellow solid (4 g, yield: 95.01%).

¹H NMR (400 MHz, DMSO-*d*₆) δ 8.06 (dddd, 2H), 7.44 (t, 1H), 3.88 (s, 3H), 2.61 (d, 3H). Step 2:

### Methyl 2-fluoro-3-(1-hydroxyethyl)benzoate (14c)

### Methyl 2-fluoro-3-(1-hydroxyethyl)benzoate

In a 100 mL three-necked flask, **14b** (4 g, 20.4 mmol) was dissolved in methanol (50 mL), and the solution was cooled to 0°C. Sodium borohydride (2.32 g, 61.2 mmol) was slowly added in portions, and the mixture was reacted at 0°C for 0.5 h. The reaction liquid was heated to room temperature and reacted for 1 h. The reaction was complete as monitored by TLC. The reaction was quenched by adding water (20 mL) to the reaction liquid. The reaction liquid was extracted with EA (50 mL × 3). The organic phases were combined, dried over anhydrous sodium sulfate, and filtered, and the organic solvent was removed under reduced pressure. The crude product was purified by silica gel column chromatography (PE : EA = 1 : 0-5 : 1) to obtain **14c** as a colorless liquid (1.74 g, yield: 43.04%).

¹H NMR (400 MHz, DMSO-*d*₆) δ 7.88 - 7.63 (m, 2H), 7.31 (t, 1H), 5.42 (d, 1H), 5.10 - 4.96 (m, 1H), 3.84 (s, 3H), 1.33 (d, 3H).

### Step 3:

### Methyl 3-(1-bromoethyl)-2-fluorobenzoate (14d)

### Methyl 3-(1-bromoethyl)-2-fluorobenzoate

In a 100 mL three-necked flask, **14c** (937 mg, 4.73 mmol) was dissolved in dichloromethane (20 mL). After nitrogen displacement, the mixture was cooled to 0°C, and phosphorus tribromide (1.54 g, 5.67 mmol) was slowly added. The mixture was heated to room temperature and reacted for 2 h. The reaction was complete as monitored by TLC. The pH was adjusted to 6 by adding a saturated aqueous sodium bicarbonate solution to the reaction liquid. The reaction liquid was extracted with EA (20 mL × 3). The organic phases were combined, dried over anhydrous sodium sulfate, and filtered. The organic solvent was removed under reduced pressure to obtain a crude product. The crude product was directly brought to the next step without purification, and the crude product **14d** was a light yellow liquid (1.25 g).

### Step 4:

### Methyl 3-(1-(7-chloro-5-(trifluoromethyl)-1H-indol-1-yl)ethyl) -2-fluorobenzoate (14e)

### Methyl 3-(1-(7-chloro-5-(trifluoromethyl)-1H-indol-1-yl)ethyl)-2-fluorobenzoate

In a 50 mL single-necked flask, **14d** (50 mg, 0.19 mmol) and 7-chloro-5-trifluoromethyl-1*H-*indole (46 mg, 0.21 mmol) were dissolved in 5 mL of acetonitrile, and cesium carbonate (124 mg, 0.38 mmol) was added. The mixture was reacted at 60°C for 5 h. The reaction was complete as monitored by TLC. The reaction liquid was filtered, the organic solvent was removed under reduced pressure, and the crude product was purified by preparative thin-layer chromatography (PE : EA = 10 : 1) to obtain **14e** as a white solid (59 mg, yield: 69.90%).

LCMS m/z (ESI) = 400.1 [M+1].

### Step 5:

### 3-(1-(7-Chloro-5-(trifluoromethyl) -1H- indol-1-yl)ethyl) -2-fluorobenzoic acid (Compound 14)

### 3-(1-(7-Chloro-5-(trifluoromethyl)-1H-indol-1-yl)ethyl)-2-fluorobenzoic acid

In a 50 mL three-necked flask, **14e** (70 mg, 0.19 mmol) was dissolved in a mixed solvent of methanol (1 mL), tetrahydrofuran (1 mL), and water (1 mL), and sodium hydroxide (15 mg, 0.38 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 14** as a white solid (30 mg, yield: 51.85%).

¹H NMR (400 MHz, DMSO-*d*₆) δ 8.02 (d, 1H), 7.85 (d, 1H), 7.74 - 7.68 (m, 1H), 7.43 (d, 1H), 7.16 (t, 1H), 6.92 (tt, 2H), 6.86 (d, 1H), 1.91 (d, 3H).

¹⁹F NMR (376 MHz, DMSO-*d*₆) δ -59.13, -117.81.

### Example 15

### 3-((7-Bromo-5-(trifluoromethyl)-1H-indol-1-yl)methyl)-2-fluorobenzoic acid (Compound 15)

### 3-((7-Bromo-5-(trifluoromethyl)-1H-indol-1-yl)methyl)-2-fluorobenzoic acid

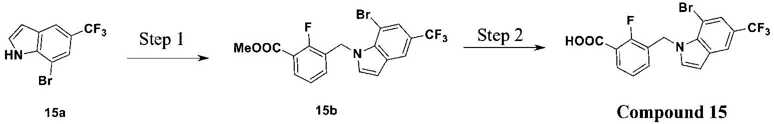

### Step 1:

### Methyl 3-(7-bromo-5-(trifluoromethyl)-1H-indol-1-yl)methyl) -2-fluorobenzoate (15b)

### Methyl 3-((7-bromo-5-(trifluoromethyl)-1H-indol-1-yl)methyl)-2-fluorobenzoate

In a 50 mL single-necked flask, 7-bromo-5-trifluoromethyl-indole **15a** (300 mg, 1.14 mmol) and methyl 3-(bromomethyl)-2-fluorobenzoate (255 mg, 1.03 mmol) were dissolved in 5 mL of acetonitrile, and potassium carbonate (285 mg, 2.06 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **15b** as a white solid (200 mg, yield: 45.14%).

LCMS m/z (ESI) = 430.0 [M+1].

### Step 2:

### 3-((7-Bromo-5-(trifluoromethyl)-1H-indol-1-yl)methyl)-2-fluorobenzoic acid (Compound 15)

### 3-((7-Bromo-5-(trifluoromethyl)-1H-indol-1-yl)methyl)-2-fluorobenzoic acid

In a 50 mL three-necked flask, **15b** (60 mg, 0.14 mmol) was dissolved in a mixed solvent of methanol (1 mL), tetrahydrofuran (1 mL), and water (1 mL), and sodium hydroxide (11 mg, 0.28 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 15** as a white solid (30 mg, yield: 51.49%).

¹H NMR (400 MHz, DMSO-*d*₆) δ 13.35 (s, 1H), 8.10 (d, 1H), 7.81 - 7.71 (m, 2H), 7.60 (d, 1H), 7.15 (t, 1H), 6.86 (d, 1H), 6.48 (t, 1H), 5.98 (s, 2H).

¹⁹F NMR (376 MHz, DMSO-*d*₆) δ -59.03, -116.38.

### Example 16

### 3-((7-Methyl-5-(trifluoromethyl)-1H-indol-1-yl)methyl)-2-fluorobenzoic acid (Compound 16)

### 3-((7-Methyl-5-(trifluoromethyl)-1H-indol-1-yl)methyl)-2-fluorobenzoic acid

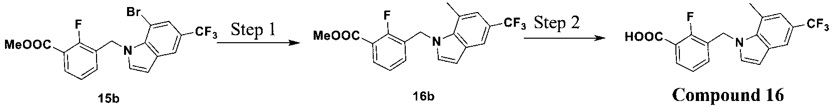

### Step 1:

### Methyl 3-((7-methyl-5-(trifluoromethyl)-1H-indol-1-yl)methyl)-2-fluorobenzoate (16b)

### Methyl 3-((7-methyl-5-(trifluoromethyl)-1H-indol-1-yl)methyl)-2-fluorobenzoate

In a 50 mL single-necked flask, **15b** (60 mg, 0.14 mmol) and methylboronic acid (17 mg, 0.28 mmol) were dissolved in 5 mL of 1,4-dioxane and 0.5 mL of water, and 1,1-bis(diphenylphosphine)palladium(II) dichloride (15 mg, 0.02 mmol) and cesium carbonate (137 mg, 0.42 mmol) were added. After nitrogen displacement, the mixture was reacted at 90°C for 8 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **16b** as a white solid (30 mg, yield: 58.66%).

LCMS m/z (ESI) = 366.1 [M+1].

### Step 2:

### 3-((7-Methyl-5-(trifluoromethyl)-1H-indol-1-yl)methyl)-2-fluorobenzoic acid (Compound 16)

### 3-((7-Methyl-5-(trifluoromethyl)-1H-indol-1-yl)methyl)-2-fluorobenzoic acid

In a 50 mL three-necked flask, **16b** (30 mg, 0.08 mmol) was dissolved in a mixed solvent of methanol (1 mL), tetrahydrofuran (1 mL), and water (1 mL), and sodium hydroxide (7 mg, 0.16 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 16** as a white solid (10 mg, yield: 35.58%).

¹H NMR (400 MHz, Chloroform-*d*) δ 7.95 (td, 1H), 7.82 (s, 1H), 7.15 (dd, 2H), 7.08 (t, 1H), 6.69 (d, 1H), 6.61 - 6.52 (m, 1H), 5.70 (s, 2H), 2.53 (s, 3H).

¹⁹F NMR (376 MHz, DMSO-*d*₆) δ -58.81, -116.50.

### Example 17

### 5-((7-Bromo-5-(trifluoromethyl)-1H-indol-1-yl)methyl)furan-2-carboxylic acid (Compound 17)

### 5-((7-Bromo-5-(trifluoromethyl)-1H-indol-1-yl)methyl)furan-2-carboxylic acid

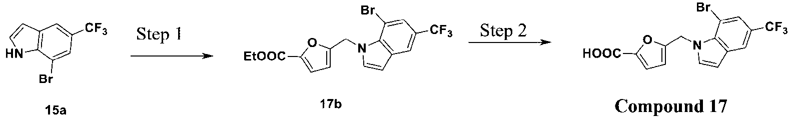

### Step 1:

### Ethyl 5-((7-bromo-5-(trifluoromethyl)-1H-indol-1-yl)methyl)furan-2-carboxylate (17b)

### Ethyl 5-((7-bromo-5-(trifluoromethyl)-1H-indol-1-yl)methyl)furan-2-carboxylate

In a 50 mL single-necked flask, **15a** (79 mg, 0.32 mmol) and ethyl 5-chloromethyl-2-furancarboxylate (50 mg, 0.29 mmol) were dissolved in 5 mL of acetonitrile, and cesium carbonate (189 mg, 0.58 mmol) was added. The mixture was reacted at 60°C for 5 h. The reaction was complete as monitored by TLC. The reaction liquid was filtered, the organic solvent was removed under reduced pressure, and the crude product was purified by preparative thin-layer chromatography (PE : EA = 10 : 1) to obtain **17b** as a white solid (60 mg, yield: 49.71%).

LCMS m/z (ESI) = 416.1 [M+1].

### Step 2:

### 5-((7-Bromo-5-(trifluoromethyl)-1H-indol-1-yl)methyl)furan-2-carboxylic acid (Compound 17)

### 5-((7-Bromo-5-(trifluoromethyl)-1H-indol-1-yl)methyl)furan-2-carboxylic acid

In a 50 mL three-necked flask, **17b** (60 mg, 0.16 mmol) was dissolved in a mixed solvent of methanol (1 mL), tetrahydrofuran (1 mL), and water (1 mL), and sodium hydroxide (13 mg, 0.32 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 17** as a white solid (30 mg, yield: 48.31%).

¹H NMR (400 MHz, DMSO-*d*₆) δ 13.09 (s, 1H), 8.05 (d, 1H), 7.74 (d, 1H), 7.63 (d, 1H), 7.12 (d, 1H), 6.80 (d, 1H), 6.25 (d, 1H), 5.94 (s, 2H).

¹⁹F NMR (376 MHz, DMSO-*d*₆) δ -59.04.

### Example 18

### 3-((7-Bromo-5-(trifluoromethyl)-1H-indol-1-yl)methyl)benzoic acid (Compound 18)

### 3-((7-Bromo-5-(trifluoromethyl)-1H-indol-1-yl)methyl)benzoic acid

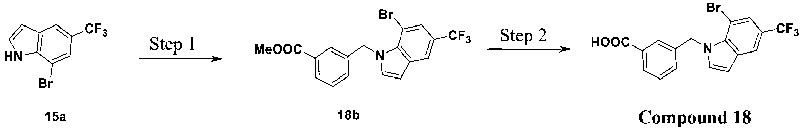

### Step 1:

### Methyl 3-((7-bromo-5-(trifluoromethyl)-1H-indol-1-yl)methyl)benzoate (18b)

### Methyl 3-((7-bromo-5-(trifluoromethyl)-1H-indol-1-yl)methyl)benzoate

In a 50 mL single-necked flask, **15a** (63 mg, 0.24 mmol) and methyl 3-bromomethylbenzoate (50 mg, 0.22 mmol) were dissolved in 5 mL of acetonitrile, and cesium carbonate (142 mg, 0.44 mmol) was added. The mixture was reacted at 60°C for 5 h. The reaction was complete as monitored by TLC. The reaction liquid was filtered, the organic solvent was removed under reduced pressure, and the crude product was purified by preparative thin-layer chromatography (PE : EA = 10 : 1) to obtain **18b** as a white solid (75 mg, yield: 82.70%).

LCMS m/z (ESI) = 412.0 [M+1].

### Step 2:

### 3-((7-Bromo-5-(trifluoromethyl)-1H-indol-1-yl)methyl)benzoic acid (Compound 18)

### 3-((7-Bromo-5-(trifluoromethyl)-1H-indol-1-yl)methyl)benzoic acid

In a 50 mL three-necked flask, **18b** (75 mg, 0.18 mmol) was dissolved in a mixed solvent of methanol (1 mL), tetrahydrofuran (1 mL), and water (1 mL), and sodium hydroxide (14 mg, 0.36 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 18** as a white solid (30 mg, yield: 41.86%).

¹H NMR (400 MHz, DMSO-*d*₆) δ 12.98 (s, 1H), 8.08 (dd, 1H), 7.84 - 7.78 (m, 2H), 7.59 (d, 1H), 7.54 (d, 1H), 7.45 (t, 1H), 7.21 (ddd, 1H), 6.85 (d, 1H), 5.95 (s, 2H).

¹⁹F NMR (376 MHz, DMSO-*d₆*) δ -59.00.

### Example 19

### 6-((7-Bromo-5-(trifluoromethyl)-1H-indol-1-yl)methyl)picolinic acid (Compound 19)

### 6-((7-Bromo-5-(trifluoromethyl)-1H-indol-1-yl)methyl)picolinic acid

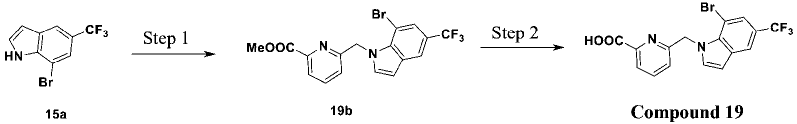

### Step 1:

### Methyl 6-((7-bromo-5-(trifluoromethyl)-1H-indol-1-yl)methyl)picolinate (19b)

### Methyl 6-((7-bromo-5-(trifluoromethyl)-1H-indol-1-yl)methyl)picolinate

In a 50 mL single-necked flask, **15a** (119 mg, 0.48 mmol) and methyl 6-(bromomethyl)picolinate (100 mg, 0.43 mmol) were dissolved in 5 mL of acetonitrile, and cesium carbonate (283 mg, 0.87 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-3 : 1) to obtain **19b** as a white solid (161 mg, yield: 87.03%).

¹H NMR (400 MHz, DMSO-*d*₆) δ 8.10 (d, 1H), 7.98 - 7.84 (m, 2H), 7.79 (d, 1H), 7.58 (d, 1H), 6.86 (d, 1H), 6.66 (dd, 1H), 6.03 (s, 2H), 3.88 (s, 3H).

¹⁹F NMR (376 MHz, DMSO-*d*₆) δ -59.02.

### Step 2:

### 6-((7-Bromo-5-(trifluoromethyl)-1H-indol-1-yl)methyl)picolinic acid (Compound 19)

### 6-((7-Bromo-5-(trifluoromethyl)-1H-indol-1-yl)methyl)picolinic acid

In a 50 mL three-necked flask, **19b** (155 mg, 0.38 mmol) was dissolved in a mixed solvent of methanol (1 mL), tetrahydrofuran (1 mL), and water (1 mL), and sodium hydroxide (30 mg, 0.75 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 19** as a white solid (110 mg, yield: 72.52%).

¹H NMR (400 MHz, DMSO-*d*₆) δ 13.23 (s, 1H), 8.09 (d, 1H), 7.95 - 7.83 (m, 2H), 7.80 (d, 1H), 7.58 (d, 1H), 6.86 (d, 1H), 6.65 (d, 1H), 6.03 (s, 2H).

¹⁹F NMR (376 MHz, DMSO-*d*₆) δ -59.01.

### Example 20

### 3-(1-(7-Bromo-5-(trifluoromethyl)-1H-indol-1-yl)ethyl)-2-fluorobenzoic acid (Compound 20)

### 3-(1-(7-Bromo-5-(trifluoromethyl)-1H-indol-1-yl)ethyl)-2-fluorobenzoic acid

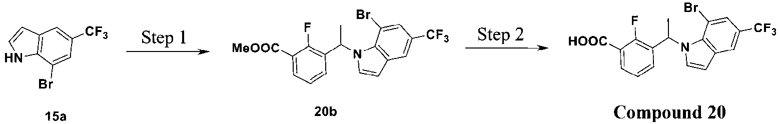

### Step 1:

### Methyl 3-(1-(7-bromo-5-(trifluoromethyl)-1H-indol-1-yl)ethyl)-2-fluorobenzoate (20b)

### Methyl 3-(1-(7-bromo-5-(trifluoromethyl)-1H-indol-1-yl)ethyl)-2-fluorobenzoate

In a 50 mL single-necked flask, **15a** (56 mg, 0.21 mmol) and **14d** (50 mg, 0.21 mmol) were dissolved in 5 mL of acetonitrile, and cesium carbonate (124 mg, 0.38 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **20b** as a white solid (61 mg, yield: 72.27%).

LCMS m/z (ESI) = 444.0 [M+1].

### Step 2:

### 3-(1-(7-Bromo-5-(trifluoromethyl)-1H-indol-1-yl)ethyl)-2-fluorobenzoic acid (Compound 20)

### 3-(1-(7-Bromo-5-(trifluoromethyl)-1H-indol-1-yl)ethyl)-2-fluorobenzoic acid

In a 50 mL three-necked flask, **20b** (61 mg, 0.14 mmol) was dissolved in a mixed solvent of methanol (1 mL), tetrahydrofuran (1 mL), and water (1 mL), and sodium hydroxide (11 mg, 0.28 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 20** as a white solid (30 mg, yield: 49.81%).

¹H NMR (400 MHz, DMSO-*d₆*) δ 8.06 (d, 1H), 7.80 (d, 1H), 7.66 (t, 1H), 7.59 (d, 1H), 7.10 (dt, 2H), 6.89 - 6.80 (m, 2H), 1.91 (d, 3H).

¹⁹F NMR (376 MHz, DMSO-*d₆*) δ -59.08, -117.12.

### Example 21

### 3-((7-Chloro-5-(trifluoromethyl)-1H-indazol-1-yl)methyl)-2-fluorobenzoic acid (Compound 21)

### 3-((7-Chloro-5-(trifluoromethyl)-1H-indazol-1-yl)methyl)-2-fluorobenzoic acid

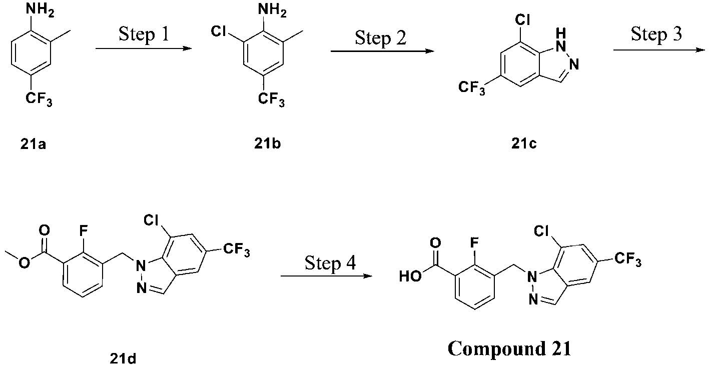

### Step 1:

### 2-Chloro-6-methyl-4-(trifluoromethyl)aniline (21b)

### 2-Chloro-6-methyl-4-(trifluoromethyl)aniline

In a 250 mL round-bottomed flask, **21a** (500 mg, 2.86 mmol) and N-chlorosuccinimide (424.87 mg, 3.18 mmol) were dissolved in acetonitrile (20 mL) and reacted at room temperature for 6 h. The reaction was complete as monitored by TLC. Acetonitrile was removed under reduced pressure. The residue was subjected to column chromatography (PE/EA = 20 : 1) to obtain **21b** as a brown liquid (437 mg, yield: 72.95%).

LC-MS m/z (ESI) = 210.08 [M+1].

### Step 2:

### 7-Chloro-5-(trifluoromethyl)-1H-indazole (21c)

### 7-Chloro-5-(trifluoromethyl)-1H-indazole

In a 250 mL round-bottomed flask, **21b** (437 mg, 2.09 mmol) was dissolved in glacial acetic acid (30 mL) and precooled at 0°C for 5 min, and a saturated aqueous solution of sodium nitrite (215.79 mg, 3.13 mmol) was then added and reacted at room temperature for 6 h. The reaction was complete as monitored by TLC. The reaction liquid was poured into ice water. Next, the pH was adjusted to neutrality with saturated sodium bicarbonate. A solid was precipitated, and after suction filtration, a filter cake was collected to obtain **21c** as a yellow solid (363 mg, yield: 93.32%).

### Step 3:

### Methyl 3-((7-chloro-5-(trifluoromethyl)-1H-indazol-1-yl)methyl)-2-fluorobenzoate (21d)

### Methyl 3-((7-chloro-5-(trifluoromethyl)-1H-indazol-1-yl)methyl)-2-fluorobenzoate

In a 100 mL round-bottomed flask, **21c** (186.17 mg, 0.85 mmol), potassium carbonate (265.65 mg, 1.93 mmol) and methyl 3-(bromomethyl)-2-fluorobenzoate (200 mg, 0.77 mmol) were dissolved in acetonitrile (20 mL), and the mixture was heated and reacted under reflux for 3 h. The reaction was complete as monitored by TLC. Acetonitrile was removed under reduced pressure. The residue was subjected to column chromatography (PE/EA = 20 : 1) to **21d** as a yellowish white solid (160 mg, yield: 59.25%).

LC-MS m/z (ESI) = 387.12 [M+1].

### Step 4:

### 3-((7-Chloro-5-(trifluoromethyl)-1H-indazol-1-yl)methyl)-2-fluorobenzoic acid (Compound 21)

### 3-((7-Chloro-5-(trifluoromethyl)-1H-indazol-1-yl)methyl)-2-fluorobenzoic acid

In a 100 mL round-bottomed flask, **21d** (160 mg, 0.42 mmol) and sodium hydroxide (50 mg, 1.24 mmol) were dissolved in ethanol and water (8:2 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 21** as a white solid (30 mg, yield: 19.23%).

LC-MS m/z (ESI) = 373.13 [M+1].

¹H NMR (400 MHz, DMSO-*d₆*) δ 8.47 (s, 1H), 8.32 (t, 1H), 7.84 (d, 1H), 7.75 (td, 1H), 7.16 (t, 1H), 6.94 (td, 1H), 6.09 (s, 2H).

### Example 22

### 3-((7-Bromo-5-(trifluoromethyl)-1H-indazol-1-yl)methyl)-2-fluorobenzoic acid (Compound 22)

### 3-((7-Bromo-5-(trifluoromethyl)-1H-indazol-1-yl)methyl)-2-fluorobenzoic acid

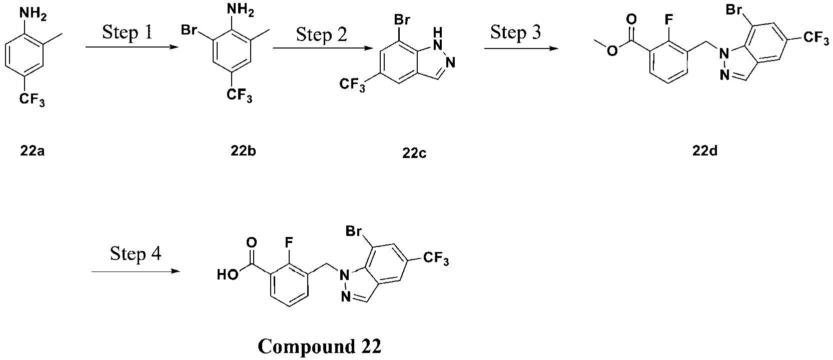

### Step 1:

### 2-Bromo-6-methyl-4-(trifluoromethyl)aniline (22b)

### 2-Bromo-6-methyl-4-(trifluoromethyl)aniline

In a 250 mL round-bottomed flask, **22a** (2.0 g, 11.42 mmol) and N-bromosuccinimide (2.24 g, 12.56 mmol) were dissolved in acetonitrile (50 mL) and reacted at room temperature for 6 h. The reaction was complete as monitored by TLC. The reaction liquid was filtered. Acetonitrile was removed under reduced pressure. The residue was subjected to column chromatography (PE/EA = 20 : 1) to obtain **22b** as a brown liquid (2.34 g, yield: 75.86%).

LC-MS m/z (ESI) = 272.15 [M+1].

### Step 2:

### 7-Bromo-5-(trifluoromethyl)-1H-indazole (22c)

### 7-Bromo-5-(trifluoromethyl)-1H-indazole

In a 250 mL round-bottomed flask, **22b** (1.9 g, 7.52 mmol) was dissolved in glacial acetic acid (150 mL) and precooled at 0°C for 5 min, and a saturated aqueous solution of sodium nitrite (881.23 mg, 12.78 mmol) was then added and reacted at room temperature for 6 h. The reaction was complete as monitored by TLC. The reaction liquid was poured into ice water. Next, the pH was adjusted to neutrality with saturated sodium bicarbonate. A solid was precipitated, and after suction filtration, a filter cake was collected to obtain **22c** as a yellow solid (1.1 g, yield: 44.71%).

### Step 3:

### Methyl 3-((7-bromo-5-(trifluoromethyl)-1H-indazol-1-yl)methyl)-2-fluorobenzoate (22d)

### Methyl 3-((7-bromo-5-(trifluoromethyl)-1H-indazol-1-yl)methyl)-2-fluorobenzoate

In a 100 mL round-bottomed flask, **22c** (1.1 g, 4.15 mmol), cesium carbonate (3.3 g, 10.38 mmol) and methyl 3-(bromomethyl)-2-fluorobenzoate (1.08 g, 4.15 mmol) were dissolved in acetonitrile (30 mL), and the mixture was heated and reacted under reflux for 3 h. The reaction was complete as monitored by TLC. Acetonitrile was removed under reduced pressure. The residue was subjected to column chromatography (PE/EA = 20 : 1) to **22d** as a yellowish white solid (800 mg, yield: 59.25%).

LC-MS m/z (ESI) = 432.12 [M+1].

### Step 4:

### 3-((7-Bromo-5-(trifluoromethyl)-1H-indazol-1-yl)methyl)-2-fluorobenzoic acid (Compound 22)

### 3-((7-Bromo-5-(trifluoromethyl)-1H-indazol-1-yl)methyl)-2-fluorobenzoic acid

In a 100 mL round-bottomed flask, **22d** (50 mg, 0.12 mmol) and sodium hydroxide (14 mg, 0.35 mmol) were dissolved in ethanol and water (6 : 1 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 22** as a white solid (30 mg, yield: 59.80%).

LC-MS m/z (ESI) = 373.13 [M+1].

¹H NMR (400 MHz, DMSO-*d₆*) δ 8.47 (s, 1H), 8.37 (t, 1H), 7.97 (d, 1H), 7.74 (td, 1H), 7.15 (t, 1H), 6.84 - 6.78 (m, 1H), 6.14 (s, 2H).

### Example 23

### 2-Fluoro-3-((7-methyl-5-(trifluoromethyl)-1H-indazol-1-yl)methyl)benzoic acid (Compound 23)

### 2-Fluoro-3-((7-methyl-5-(trifluoromethyl)-1H-indazol-1-yl)methyl)benzoic acid

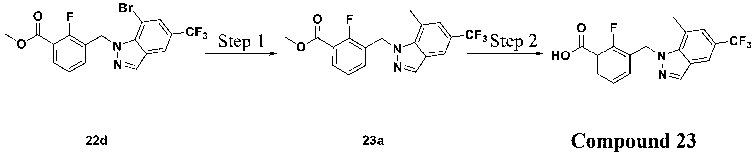

### Step 1:

### Methyl 2-fluoro-3-((7-methyl-5-(trifluoromethyl)-1H-indazol-1-yl)methyl)benzoate (23a)

### Methyl 2-fluoro-3-((7-methyl-5-(trifluoromethyl)-1H-indazol-1-yl)methyl)benzoate

In a 100 mL round-bottomed flask, **22d** (120 mg, 0.28 mmol), cesium carbonate (228.07 mg, 0.7 mmol), methylboronic acid (33.32 mg, 0.56 mmol), and DPPF palladium dichloride (22.85 mg, 0.03mmol) were dissolved in 1,4-dioxane (10 mL), and under nitrogen protection, the mixture was heated and reacted under reflux for 8 h. The reaction was complete as monitored by LCMS. 1,4-Dioxane was removed under reduced pressure. The residue was subjected to column chromatography (PE/EA = 20 : 1) to obtain **23a** as a yellow solid (100 mg, yield: 98.03%).

LC-MS m/z (ESI) = 367.52 [M+1].

### Step 2:

### 2-Fluoro-3-((7-methyl-5-(trifluoromethyl)-1H-indazol-1-yl)methyl)benzoic acid (Compound 23)

### 2-Fluoro-3-((7-methyl-5-(trifluoromethyl)-1H-indazol-1-yl)methyl)benzoic acid

In a 100 mL round-bottomed flask, **23a** (100 mg, 0.27 mmol) and sodium hydroxide (32.76 mg, 0.82 mmol) were dissolved in ethanol and water (8 : 1 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 23** as a white solid (70 mg, yield: 73.44%).

LC-MS m/z (ESI) = 364.13 [M+1].

¹H NMR (400 MHz, DMSO-*d*₆) δ 8.33 (s, 1H), 8.11 (s, 1H), 7.76 - 7.62 (m, 1H), 7.45 (s, 1H), 7.12 (t, 1H), 6.73 (t, 1H), 5.96 (s, 2H), 2.66 (s, 3H).

### Example 24

### 3-((7-Cyclopropyl-5-(trifluoromethyl)-1H-indazol-1-yl)methyl)-2-fluorobenzoic acid (Compound 24)

### 3-((7-Cyclopropyl-5-(trifluoromethyl)-1H-indazol-1-yl)methyl)-2-fluorobenzoic acid

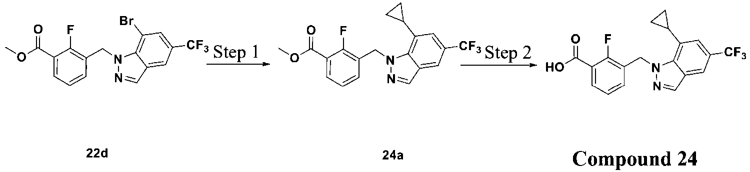

### Step 1:

### Methyl 3-((7-bromo-5-(trifluoromethyl)-1H-indazol-1-yl)methyl)-2-fluorobenzoate (24a)

### Methyl 3-((7-bromo-5-(trifluoromethyl)-1H-indazol-1-yl)methyl)-2-fluorobenzoate

In a 100 mL round-bottomed flask, **22d** (140 mg, 0.33 mmol), cesium carbonate (268.80 mg, 0.83 mmol), cyclopropylboronic acid (55.78 mg, 0.65 mmol), and DPPF palladium dichloride (26.93 mg, 0.03 mmol) were dissolved in 1,4-dioxane (10 mL), and under nitrogen protection, the mixture was heated and reacted under reflux for 8 h. The reaction was complete as monitored by LCMS. 1,4-Dioxane was removed under reduced pressure. The residue was subjected to column chromatography (PE/EA = 20 : 1) to obtain **24a** as a yellow solid (100 mg, yield: 77.51%).

LC-MS m/z (ESI) = 393.65 [M+1].

### Step 2:

### 3-((7-Cyclopropyl-5-(trifluoromethyl)-1H-indazol-1-yl)methyl)-2-fluorobenzoic acid (Compound 24)

### 3-((7-Cyclopropyl-5-(trifluoromethyl)-1H-indazol-1-yl)methyl)-2-fluorobenzoic acid

In a 100 mL round-bottomed flask, **24a** (100 mg, 0.27 mmol) and sodium hydroxide (32.76 mg, 0.82 mmol) were dissolved in ethanol and water (8 : 1 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 24** as a white solid (65 mg, yield: 66.33%).

LC-MS m/z (ESI) = 379.23 [M+1].

¹H NMR (400 MHz, DMSO-*d*₆) δ 8.36 (s, 1H), 8.17 - 8.10 (m, 1H), 7.74 (td, 1H), 7.31 (t 1H), 7.14 (t, 1H), 6.78 (t, 1H), 6.17 (s, 2H), 2.39 - 2.30 (m, 1H), 0.99 - 0.92 (m, 2H), 0.82 - 0.75 (m, 2H).

### Example 25

### 3-((7-Ethynyl-5-(trifluoromethyl)-1H-indazol-1-yl)methyl)-2-fluorobenzoic acid (Compound 25)

### 3-((7-Ethynyl-5-(trifluoromethyl)-1H-indazol-1-yl)methyl)-2-fluorobenzoic acid

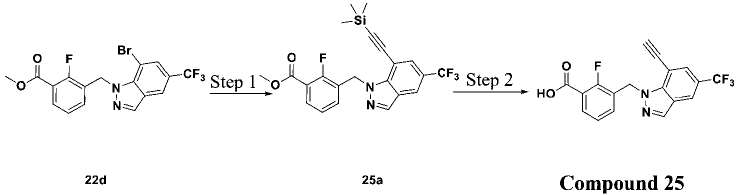

### Step 1:

### Methyl 2-fluoro-3-((5-(trifluoromethyl)-7-((trimethylsilyl)ethynyl)-1H-indazol-1-yl)methyl)benzoate (25a)

### Methyl 2-fluoro-3-((5-(trifluoromethyl)-7-((trimethylsilyl)ethynyl)-1H-indazol-1-yl)methyl)benzoate

In a 100 mL round-bottomed flask, **22d** (120 mg, 0.28 mmol), trimethylsilylacetylene (54.67 mg, 0.56 mmol), cuprous iodide (5.3 mg, 0.03 mmol), triphenylphosphine (3.67 mg, 0.01 mmol), and Pd(dppf)Cl₂ (9.82 mg, 0.01 mmol) were dissolved in triethylamine (10 mL), and under nitrogen protection, the mixture was reacted at room temperature 4 h. The reaction was complete as monitored by LCMS. Triethylamine was removed under reduced pressure. The residue was subjected to column chromatography (PE/EA = 20 : 1) to obtain **25a** as a yellow solid (120 mg, yield: 96.32%).

LC-MS m/z (ESI) = 432.54 [M+1].

### Step 2:

### 3-((7-Ethynyl-5-(trifluoromethyl)-1H-indazol-1-yl)methyl)-2-fluorobenzoic acid (Compound 25)

### 2-Fluoro-3-((7-methyl-5-(trifluoromethyl)-1H-indazol-1-yl)methyl)benzoic acid

In a 100 mL round-bottomed flask, **25a** (120 mg, 0.27 mmol) and sodium hydroxide (37.46 mg, 0.94 mmol) were dissolved in ethanol and water (8 : 1 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 25** as a white solid (60 mg, yield: 49.58%).

LC-MS m/z (ESI) = 436.72 [M+1].

¹H NMR (400 MHz, DMSO-*d*₆) δ 13.28 (s, 1H), 8.44 (s, 1H), 8.38 (s, 1H), 7.86 (s, 1H), 7.78 (td, 1H), 7.19 (t, 1H), 7.08 - 6.97 (m, 1H), 6.16 (s, 2H), 4.81 (s, 1H).

### Example 26

### 3-((5,7-Bis(trifluoromethyl)-1H-indazol-1-yl)methyl)-2-fluorobenzoic acid (Compound 26)

### 3-((5,7-Bis(trifluoromethyl)-1H-indazol-1-yl)methyl)-2-fluorobenzoic acid

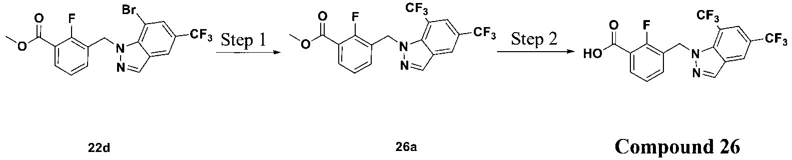

### Step 1:

### Methyl 3-((5,7-bis(trifluoromethyl)-1H-indazol-1-yl)methyl)-2-fluorobenzoate (26a)

### Methyl 3-((5,7-bis(trifluoromethyl)-1H-indazol-1-yl)methyl)-2-fluorobenzoate

### In a 100 mL round-bottomed flask, 22d (100 mg, 0.23 mmol), methyl fluorosulfonyldifluoroacetate (222.61 mg, 1.16 mmol), cuprous iodide (48.03 mg, 0.25 mmol), and hexamethylphosphoric triamide (206.08 mg, 1.16 mmol) were dissolved in DMF (8 mL), and under nitrogen protection, the mixture was reacted at 100°C for 8 h. The reaction was complete as monitored by LCMS. The reaction liquid was extracted with EtOAc and water and dried over anhydrous sodium sulfate. The organic solvent was removed under reduced pressure, and the residue was subjected to column chromatography (PE/EA = 20 : 1) to obtain 26a as a yellow solid (60 mg, yield: 62.11%).

LC-MS m/z (ESI) = 421.54 [M+1].

### Step 2:

### 3-((5,7-Bis(trifluoromethyl)-1H-indazol-1-yl)methyl)-2-fluorobenzoic acid (Compound 26)

### 3-((5,7-Bis(trifluoromethyl)-1H-indazol-1-yl)methyl)-2-fluorobenzoic acid

In a 100 mL round-bottomed flask, **26a** (60 mg, 0.14 mmol) and sodium hydroxide (17.15 mg, 0.43 mmol) were dissolved in ethanol and water (6 : 1 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 26** as a white solid (5 mg, yield: 9%).

LC-MS m/z (ESI) = 408.32 [M+1].

¹H NMR (400 MHz, DMSO-*d*₆) δ 8.76 (s, 1H), 8.64 (s, 1H), 8.11 (s, 1H), 7.86 - 7.72 (m, 1H), 7.18 (t, 1H), 6.92 (t, 1H), 5.86 (s, 2H).

¹⁹F NMR (377 MHz, DMSO-*d*₆) δ -56.17, -59.56, -117.06.

### Example 27

### 3-((7-Bromo-5-(trifluoromethoxy)-1H-indazol-1-yl)methyl)-2-fluorobenzoic acid (Compound 27)

### 3-((7-Bromo-5-(trifluoromethoxy)-1H-indazol-1-yl)methyl)-2-fluorobenzoic acid

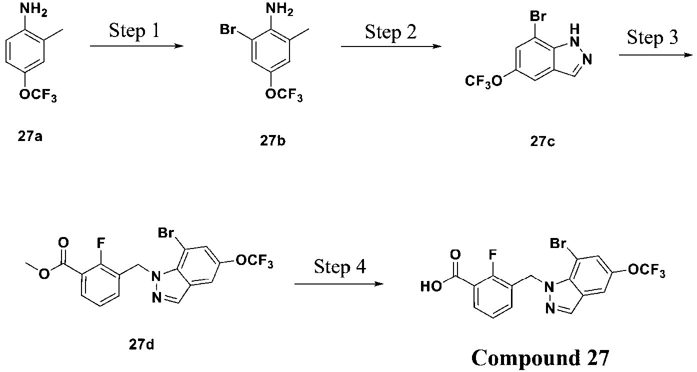

### Step 1:

### 2-Methyl-4-(trifluoromethoxy)aniline (27b)

### 2-Methyl-4-(trifluoromethoxy)aniline

In a 250 mL round-bottomed flask, **27a** (2 g, 10.47 mmol) and N-bromosuccinimide (2.05 g, 11.52 mmol) were dissolved in acetonitrile (80 mL) and reacted at room temperature for 6 h. The reaction was complete as monitored by TLC. Acetonitrile was removed under reduced pressure. The residue was subjected to column chromatography (PE/EA = 50 : 1) to obtain **27b** as a brown liquid (1.2 g, yield: 42.28%).

LC-MS m/z (ESI) = 271.08 [M+1].

### Step 2:

### 7-Bromo-5-(trifluoromethoxy)-1H-indazole (27c)

### 7-Bromo-5-(trifluoromethoxy)-1H-indazole

In a 250 mL round-bottomed flask, **27b** (1.2 g, 4.45 mmol) was dissolved in glacial acetic acid (150 mL) and precooled at 0°C for 5 min, and a saturated aqueous solution of sodium nitrite (490.58 mg, 7.11 mmol) was then added and reacted at room temperature for 6 h. The reaction was complete as monitored by TLC. The reaction liquid was poured into ice water. Next, the pH was adjusted to neutrality with saturated sodium bicarbonate. A solid was precipitated, and after suction filtration, a filter cake was collected to obtain **27c** as a yellow solid (936 mg, yield: 74.88%).

### Step 3:

### Methyl 3-((7-bromo-5-(trifluoromethoxy)-1H-indazol-1-yl)methyl)-2-fluorobenzoate (27d)

### Methyl 3-((7-bromo-5-(trifluoromethoxy)-1H-indazol-1-yl)methyl)-2-fluorobenzoate

In a 100 mL round-bottomed flask, **27c** (400 mg, 0.95 mmol), cesium carbonate (773.83 mg, 2.4 mmol) and methyl 3-(bromomethyl)-2-fluorobenzoate (270.86 mg, 1.05 mmol) were dissolved in acetonitrile (20 mL), and the mixture was heated and reacted under reflux for 3 h. The reaction was complete as monitored by TLC. Acetonitrile was removed under reduced pressure. The residue was subjected to column chromatography (PE/EA = 20 : 1) to **27d** as a yellowish white solid (189 mg, yield: 44.57%).

LC-MS m/z (ESI) = 448.12 [M+1].

### Step 4:

### 3-((7-Chloro-5-(trifluoromethyl)-1H-indazol-1-yl)methyl)-2-fluorobenzoic acid (Compound 27)

### 3-((7-Chloro-5-(trifluoromethyl)-1H-indazol-1-yl)methyl)-2-fluorobenzoic acid

In a 100 mL round-bottomed flask, **27d** (189 mg, 0.43 mmol) and sodium hydroxide (50.72 mg, 1.27 mmol) were dissolved in ethanol and water (8 : 2 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 27** as a white solid (100 mg, yield: 53.76%).

¹H NMR (400 MHz, DMSO-*d*₆) δ 13.22 (s, 1H), 8.38 (s, 1H), 7.98 (dt, 1H), 7.88 - 7.72 (m, 2H), 7.19 (t, 1H), 6.85 (td, 1H), 6.11 (s, 2H).

¹⁹F NMR (377 MHz, DMSO-*d*₆) δ -57.35, -116.25.

### Example 28

### 2-Fluoro-3-((5,6,7-trichloro-1H-indazol-1-yl)methyl)benzoic acid (Compound 28)

### 2-Fluoro-3-((5,6,7-trichloro-1H-indazol-1-yl)methyl)benzoic acid

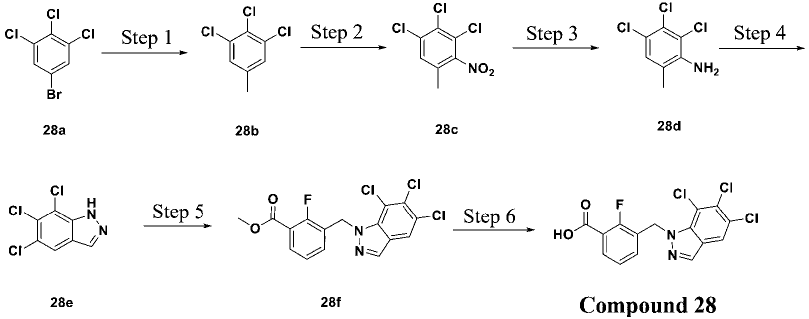

### Step 1:

### 1,2,3-Trichloro-5-toluene (28b)

In a 250 mL round-bottomed flask, **28a** (1 g, 3.84 mmol), cesium carbonate (3.13 g, 9.6 mmol), methylboronic acid (252.92 mg, 4.23 mmol), and DPPF palladium dichloride (313.35 mg, 0.39 mmol) were dissolved in 1,4-dioxane (50 mL), and under nitrogen protection, the mixture was heated and reacted under reflux for 8 h. The reaction was complete as monitored by LCMS. 1,4-Dioxane was removed under reduced pressure. The residue was subjected to column chromatography (PE/EA = 100 : 1) to obtain **28b** as a yellow solid (610 mg, yield: 81.33%).

LC-MS m/z (ESI) = 196.87 [M+1].

### Step 2:

### 1,2,3-Trichloro-5-methyl-4-nitrobenzene (28c)

### 1,2,3-Trichloro-5-methyl-4-nitrobenzene

In a 100 mL round-bottomed flask, **28b** (610 mg, 3.12 mmol) was dissolved in concentrated sulfuric acid (10 mL), and a mixed acid of nitric acid (255.63 mg, 4.06 mmol) and sulfuric acid (1 mL) was then dropwise added at 0°C. After the addition was complete, the mixture was transferred to room temperature and reacted for 1 h. The reaction was complete as monitored by TLC. The reaction liquid was poured into ice water. A solid was precipitated, and after suction filtration, a filter cake was collected to obtain **28c** as a brown solid (730 mg, yield: 94.81%).

### Step 3:

### 2,3,4-Trichloro-6-methylaniline (28d)

### 2,3,4-Trichloro-6-methylaniline

In a 100 mL round-bottomed flask, **28c** (770 mg, 3.2 mmol) was dissolved in ethanol and water (10 : 2 mL), and iron powder (896.51 mg, 16 mmol) and ammonium chloride (85.59 mg, 1.60 mmol) were then added. The mixture was reacted at 80°C for 6 h. The reaction was complete as monitored by TLC. The reaction liquid was subjected to suction filtration, and the organic solvent was removed under reduced pressure. The residue was subjected to column chromatography (PE : EA = 1 : 1) to obtain **28d** as a yellow solid (160 mg, yield: 23.77%).

LC-MS m/z (ESI) = 211.59 [M+1].

### Step 4:

### 5,6,7-Trichloro-1H-indazole (28e)

### 5,6,7-Trichloro-1H-indazole

In a 100 mL round-bottomed flask, **28d** (160 mg, 0.76 mmol) was dissolved in glacial acetic acid (30 mL) and precooled at 0°C for 5 min, and a saturated aqueous solution of sodium nitrite (89.17 mg, 1.29 mmol) was then added and reacted at room temperature for 6 h. The reaction was complete as monitored by TLC. The reaction liquid was poured into ice water. Next, the pH was adjusted to neutrality with saturated sodium bicarbonate. A solid was precipitated, and after suction filtration, a filter cake was collected to obtain **28e** as a yellow solid (127 mg, yield: 76.04%).

LC-MS m/z (ESI) = 222.55 [M+1].

### Step 5:

### Methyl 2-fluoro-3-((5,6,7-trichloro-1H-indazol-1-yl)methyl)benzoate (28f)

### Methyl 2-fluoro-3-((5,6,7-trichloro-1H-indazol-1-yl)methyl)benzoate

In a 100 mL round-bottomed flask, **28e** (127 mg, 0.57 mmol), cesium carbonate (464.29 mg, 1.43 mmol) and methyl 3-(bromomethyl)-2-fluorobenzoate (178.73 mg, 0.69 mmol) were dissolved in acetonitrile (10 mL), and the mixture was heated and reacted under reflux for 3 h. The reaction was complete as monitored by TLC. Acetonitrile was removed under reduced pressure. The residue was subjected to column chromatography (PE/EA = 20 : 1) to **28f** as a yellow solid (90 mg, yield: 40.09%).

LC-MS m/z (ESI) = 388.78 [M+1].

### Step 6:

### 2-Fluoro-3-((5,6,7-trichloro-1H-indazol-1-yl)methyl)benzoic acid (Compound 28)

### 2-Fluoro-3-((5,6,7-trichloro-1H-indazol-1-yl)methyl)benzoic acid

In a 100 mL round-bottomed flask, **28f** (90 mg, 0.23 mmol) and sodium hydroxide (28 mg, 0.69 mmol) were dissolved in ethanol and water (8 : 2 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 28** as a white solid (40 mg, yield: 46.51%).

LC-MS m/z (ESI) = 374.96 [M+1].

¹H NMR (400 MHz, DMSO-*d*₆) δ 8.31 (s, 1H), 8.21 (s, 1H), 7.64 (t, 1H), 7.06 (t, 1H), 6.72 (s, 1H), 6.03 (s, 2H).

### Example 29

### 3-((7-Bromo-5-(difluoromethoxy)-1H-indazol-1-yl)methyl)-2-fluorobenzoic acid (Compound 29)

### 3-((7-Bromo-5-(difluoromethoxy)-1H-indazol-1-yl)methyl)-2-fluorobenzoic acid

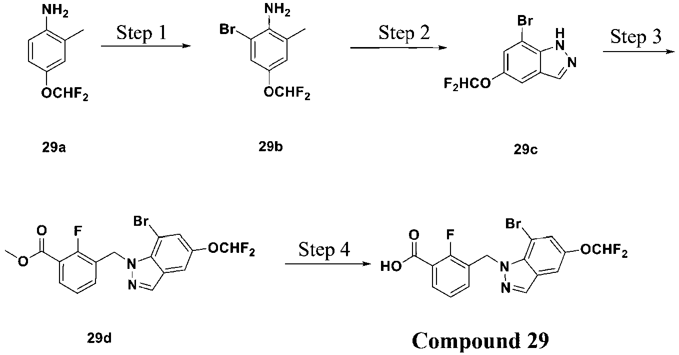

### Step 1:

### 2-Bromo-4-(difluoromethoxy)-6-methylaniline (29b)

### 2-Bromo-4-(difluoromethoxy)-6-methylaniline

In a 250 mL round-bottomed flask, **29a** (500 mg, 2.89 mmol) and N-bromosuccinimide (565.31 mg, 3.17 mmol) were dissolved in acetonitrile (30 mL) and reacted at room temperature for 6 h. The reaction was complete as monitored by TLC. The reaction liquid was filtered. Acetonitrile was removed under reduced pressure. The residue was subjected to column chromatography (PE/EA = 20 : 1) to obtain **29b** as a yellow solid (597 mg, yield: 82.86%).

LC-MS m/z (ESI) = 253.15 [M+1].

### Step 2:

### 7-Bromo-5-(difluoromethoxy)-1H-indazole (29c)

### 7-Bromo-5-(difluoromethoxy)-1H-indazole

In a 250 mL round-bottomed flask, **29b** (597 mg, 2.37 mmol) was dissolved in glacial acetic acid (150 mL) and precooled at 0°C for 5 min, and a saturated aqueous solution of sodium nitrite (261.48 mg, 1.6 mmol) was then added and reacted at room temperature for 6 h. The reaction was complete as monitored by TLC. The reaction liquid was poured into ice water. Next, the pH was adjusted to neutrality with saturated sodium bicarbonate. A solid was precipitated, and after suction filtration, a filter cake was collected to obtain **29c** as a yellow solid (220 mg, yield: 39.91%).

### Step 3:

### Methyl 3-((7-bromo-5-(difluoromethoxy)-1H-indazol-1-yl)methyl)-2-fluorobenzoate (29d)

### Methyl 3-((7-bromo-5-(difluoromethoxy)-1H-indazol-1-yl)methyl)-2-fluorobenzoate

In a 100 mL round-bottomed flask, **29c** (220 mg, 0.84 mmol), cesium carbonate (680 mg, 2.09 mmol) and methyl 3-(bromomethyl)-2-fluorobenzoate (206.63 mg, 0.84 mmol) were dissolved in acetonitrile (20 mL), and the mixture was heated and reacted under reflux for 3 h. The reaction was complete as monitored by TLC. Acetonitrile was removed under reduced pressure. The residue was subjected to column chromatography (PE/EA = 20 : 1) to **29d** as a yellowish white solid (15 mg, yield: 4.21%).

LC-MS m/z (ESI) = 430.23 [M+1].

### Step 4:

### 3-((7-Bromo-5-(trifluoromethyl)-1H-indazol-1-yl)methyl)-2-fluorobenzoic acid (Compound 29)

### 3-((7-Bromo-5-(trifluoromethyl)-1H-indazol-1-yl)methyl)-2-fluorobenzoic acid

In a 100 mL round-bottomed flask, **29d** (15 mg, 0.03 mmol) and sodium hydroxide (4.19 mg, 0.11 mmol) were dissolved in ethanol and water (6 : 1 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 22** as a white solid (10 mg, yield: 79.80%).

LC-MS m/z (ESI) = 415.65 [M+1].

¹H NMR (400 MHz, DMSO-*d*₆) δ 13.30 (s, 1H), 8.32 (s, 1H), 7.77 (t, 1H), 7.71 (s, 1H), 7.60 (s, 1H), 7.17 (t, 1H), 6.79 (t, 1H), 6.08 (s, 2H).

¹⁹F NMR (377 MHz, DMSO-*d*₆) δ -81.97 (d, *J* = 8.2 Hz), -116.24.

### Example 30

### 3-((5,7-Dichloro-1H-indazol-1-yl)methyl)-2-fluorobenzoic acid (Compound 30)

### 3-((5,7-Dichloro-1H-indazol-1-yl)methyl)-2-fluorobenzoic acid

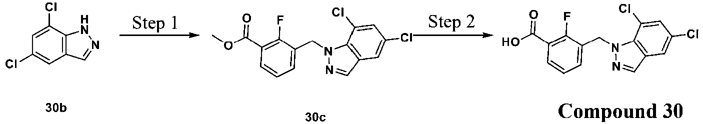

### Step 1:

In a 100 mL round-bottomed flask, methyl 3-(bromomethyl)-2-fluorobenzoate (330 mg, 1.34 mmol) and **30b** (250 mg, 1.34 mmol) were dissolved in acetonitrile (13 mL, 5 vol.), and cesium carbonate (1.09 g, 3.34 mmol) was added. The reaction was carried out at 70°C for 2 h. The reaction was complete as monitored by TLC. The reaction liquid was heated to room temperature, extracted with ethyl acetate (20 mL × 3), dried over anhydrous sodium sulfate, and filtered, and the organic solvent was removed under reduced pressure. The crude product was purified by silica gel column chromatography (PE : EA = 10 : 1) to obtain **30c** as a pale yellow solid (156 mg, yield: 33%).

### Step 2:

In a 100 mL three-necked flask, **30c** (150 mg, 425 µmol) and sodium hydroxide (34 mg, 849 µmol) were dissolved in a mixed solvent of methanol and water (5 : 1, 12 mL). The mixture was reacted at 70°C for 2 h. The reaction was complete as monitored by TLC. The reaction liquid was cooled to room temperature, methanol was removed under reduced pressure, and the pH was adjusted to acidity with 2M HCl. A solid was precipitated, filtered, and then dried to obtain **Compound 30** as a white solid (108 mg, yield: 74.98%).

¹H NMR (400 MHz, DMSO-*d*₆) δ 13.31 (s, 1H), 8.28 (s, 1H), 7.96 (d, 1H), 7.78 (t, 1H), 7.62 (d, 1H), 7.18 (t, 1H), 6.93 (t, 1H), 6.04 (s, 2H).

### Example 31

### 3-((7-Bromo-5-(trifluoromethyl)-1H-indazol-1-yl)methyl)-4-(trifluoromethyl)benzoic acid (Compound 31)

### 3-((7-Bromo-5-(trifluoromethyl)-1H-indazol-1-yl)methyl)-4-(trifluoromethyl)benzoic acid

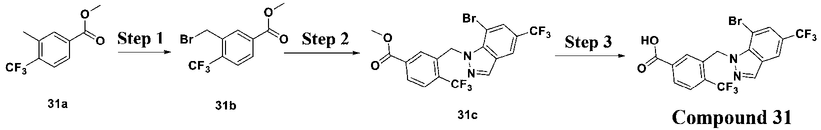

### Step 1:

### Methyl 3-(bromomethyl)-4-(trifluoromethyl)benzoate (31b)

### Methyl 3-(bromomethyl)-4-(trifluoromethyl)benzoate

In a 100 mL round-bottomed flask, **31a** (500 mg, 2.29 mmol), dibenzoyl peroxide (222 mg, 0.917 mmol), and N-bromosuccinimide (611 mg, 3.44 mmol) were dissolved in 1,2-dichloroethane (10 mL). The mixture was reacted at 80°C for 2 h. The reaction was complete as monitored by TLC. The reaction was quenched with an aqueous sodium sulfite solution, the reaction liquid was extracted with ethyl acetate (20 mL × 3), dried over anhydrous sodium sulfate, and filtered, and the organic solvent was removed under reduced pressure. The crude product was subjected to reverse phase purification (70% acetonitrile aqueous solution) to obtain **31b** as a white solid (210 mg, yield: 30.9%).

### Step 2:

### Methyl 3-((7-bromo-5-(trifluoromethyl)-1H-indazol-1-yl)methyl)-4-(trifluoromethyl)benzoate (31c)

### Methyl 3-((7-bromo-5-(trifluoromethyl)-1H-indazol-1-yl)methyl)-4-(trifluoromethyl)benzoate

In a 100 mL three-necked flask, **31b** (210 mg, 0.709 mmol) was dissolved in DMF (10 mL), and 7-bromo-5-(trifluoromethyl)-1H-indazole (170 mg, 0.65 mmol) and cesium carbonate (634 mg, 1.95 mmol) were added under stirring. The mixture was reacted at 80°C for 2 h. The reaction was complete as monitored by TLC. The reaction liquid was extracted with ethyl acetate (10 mL × 3), dried over anhydrous sodium sulfate, and filtered, and the organic solvent was removed under reduced pressure. The residue was purified by column chromatography (PE : EA = 5 : 1) to obtain 31c as a white solid (41 mg, yield: 13.1%).

### Step 3:

### 3-((7-Bromo-5-(trifluoromethyl)-1H-indazol-1-yl)methyl)-4-(trifluoromethyl)benzoic acid (Compound 31)

### 3-((7-Bromo-5-(trifluoromethyl)-1H-indazol-1-yl)methyl)-4-(trifluoromethyl)benzoic acid

In a 100 mL three-necked flask, **31c** (41 mg, 0.085 mmol) and NaOH(10 mg, 0.171 mmol) were dissolved in a mixed solvent of methanol and water (1 : 1, 10 mL). The mixture was reacted at 80°C for 2 h. The reaction was complete as monitored by TLC. The reaction was cooled to room temperature, and the pH was adjusted to acidity with 2 M HCl. The solution was extracted with ethyl acetate (10 mL × 3), dried over anhydrous sodium sulfate, and filtered, and the organic solvent was removed under reduced pressure, The crude product was subjected to reverse phase purification (20% acetonitrile aqueous solution) to obtain **Compound 31** as a white solid (13 mg, yield: 32.5%).

¹H NMR (400 MHz, DMSO-*d*₆) δ 8.55 (s, 1H), 8.43 (s, 1H), 8.04 - 7.95 (m, 2H), 7.90 (d, 1H), 6.89 (s, 1H), 6.26 (s, 2H).

### Examples 32-43

The corresponding compounds of Examples 32-43 are as shown in Table 1, and the corresponding compounds 32-43 are obtained with reference to the synthesis method of **Compound** 9 by using commercially available raw materials or intermediates in the preparation method provided by the present invention as raw materials.

**Table 1**

| Compound | Compound Name | Structure | NMR Date | LC-MS [M+1] |
|---|---|---|---|---|
| 32 | 3-((7-Bromo-5-(trifluoromethyl)-1*H-*indazol-1-yl)methyl)benzoic acid | 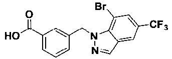 | ¹H NMR (DMSO-*d*₆) δ 13.03 (s, 1H), 8.49 (s, 1H), 8.37 (s, 1H), 7.96 (s, 1H), 7.83 (dt, 1H), 7.64 (s, 1H), 7.45 (t, 1H), 7.33 (dt, 1H), 6.12 (s, 2H). | 401.03 |
| | 3-((7-Bromo-5-(trifluoromethyl)-1*H-*indazol-1-yl)methyl)benzoic acid | | | |
| | | | ¹⁹F NMR (DMSO-*d*₆) δ - 59.45. | |
| 33 | 5-((7-Bromo-5-(trifluoromethyl)-1*H-*indazol-1-yl)methyl)furan-2-carboxylic acid | 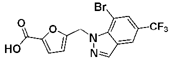 | ¹H NMR (DMSO-*d*₆) δ 8.44 (s, 1H), 8.35 (s, 1H), 7.99 (s, 1H), 7.08 (d, 1H), 6.39 (d, 1H), 6.09 (s, 2H). | 391.58 |
| | | | ¹⁹F NMR (DMSO-*d*₆) δ - 59.47. | |
| | 5-((7-Bromo-5-(trifluoromethyl)-1*H-*indazol-1-yl)methyl)furan-2-carboxylic acid | | | |
| 34 | 4-Bromo-3-((7-bromo-5-(trifluoromethyl)-1*H-*indazol-1-yl)methyl)benzoic acid | 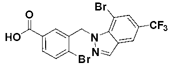 | ¹H NMR (DMSO-*d*₆) δ 13.11 (s, 1H), 8.54 (s, 1H), 8.41 (s, 1H), 7.98 (s, 1H), 7.85 (d, 1H), 7.77 (d, 1H), 6.91 (d, 1H), 6.12 (s, 2H). | 478.65 |
| | 4-Bromo-3-((7-bromo-5-(trifluoromethyl)-1*H-*indazol-1-yl)methyl)benzoic acid | | | |
| | | | ¹⁹F NMR (DMSO-*d*₆) δ - 59.46. | |
| 35 | 3-(1-(7-Bromo-5-(trifluoromethyl)-1*H-*indazol-1-yl)ethyl)-2-fluorobenzoic acid | 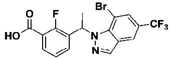 | ¹H NMR (DMSO-*d*₆) δ 13.20 (s, 1H), 8.47 (s, 1H), 8.38 - 8.33 (m, 1H), 7.95 (d, 1H), 7.78 (td, 1H), 7.26-7.14 (m, 3H), 1.95 (d, 3H). | 431.95 |
| | 3-(1-(7-Bromo-5-(trifluoromethyl)-1*H-*indazol-1-yl)ethyl)-2-fluorobenzoic acid | | ¹⁹F NMR (DMSO-*d*₆) δ - 59.49, -116.74. | |
| 36 | 3-((7-Bromo-5-chloro-1*H*-indazol-1-yl)methyl)-2-fluorobenzoic acid | 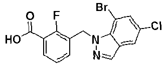 | ¹H NMR (DMSO-*d*₆) δ 13.30 (s, 1H), 8.28 (s, 1H), 8.00 (d, 1H), 7.83 - 7.71 (m, 2H), 7.17 (t, 1H), 6.79 (td, 1H), 6.08 (s, 2H). | 383.09 |
| | 3-((7-Bromo-5-chloro-1*H*-indazol-1-yl)methyl)-2-fluorobenzoic acid | | | |
| | | | ¹⁹F NMR (DMSO-*d*₆) δ - 116.24. | |
| 37 | 3-((6-Bromo-5-chloro-1*H*-indazol-1-yl)methyl)-2-fluorobenzoic acid | 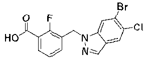 | ¹H NMR (DMSO-*d*₆) δ 8.27 (s, 2H), 8.14 (d, 1H), 7.79 (td, 1H), 7.32 (td, 1H), 7.23 (t, 1H), 5.76 (s, 2H). | 382.69 |
| | 3-((6-Bromo-5-chloro-1*H*-indazol-1-yl)methyl)-2-fluorobenzoic acid | | ¹⁹F NMR (DMSO-*d*₆) δ - 115.35. | |
| 38 | 6-((5,6,7-Trichloro-1*H-*indazol-1-yl)methyl)picolinic acid | | ¹H NMR (Chloroform-*d*) δ 8.03 (s, 2H), 7.76 (s, 2H), 6.96 (s, 1H), 6.08 (s, 2H). | 355.68 |
| | 6-((5,6,7-Trichloro-1*H*-indazol-1-yl)methyl)picolinic acid | 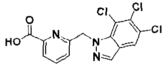 | | |
| 39 | 2-Fluoro-3-(1-(5,6,7-trichloro-1*H*-indazol-1-yl)ethyl)benzoic acid | | ¹H NMR (Chloroform-*d*) δ 8.05 (s, 1H), 7.96 - 7.87 (m, 1H), 7.78 (s, 1H), 7.17 - 7.05 (m, 3H), 2.02 (d, 3H). | 386.77 |
| | 2-Fluoro-3-(1-(5,6,7-trichloro-1*H-*indazol-1-yl)ethyl)benzoic acid | 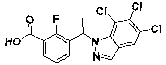 | | |
| 40 | 6-((7-Bromo-5-(trifluoromethyl)-1*H-*indazol-1-yl)methyl)picolinic acid | 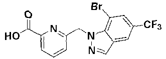 | / | 400.23 |
| | 6-((7-Bromo-5-(trifluoromethyl)-1*H*-indazol-1-yl)methyl)picolinic acid | | | |
| 41 | 5-((7-Bromo-5-(trifluoromethyl)-1*H-*indazol-1-yl)methyl)-2-fluorobenzoic acid | 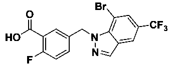 | / | 416.78 |
| | 5-((7-Bromo-5-(trifluoromethyl)-1*H-*indazol-1-yl)methyl)-2-fluorobenzoic acid | | | |
| 42 | 3-[(5,6-Dichloro-1*H-*indazol-1-yl)methyl]-2-fluorobenzoic acid | 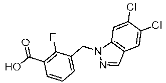 | ¹H NMR (DMSO) δ 8.27 (s, 1H), 8.17 - 8.10 (m, 2H), 7.83 - 7.75 (m, 1H), 7.37 - 7.29 (m, 1H), 7.23 (t, 1H), 5.77 (s, 2H). | / |
| | 3-[(5,6-Dichloro-1*H-*indazol-1-yl)methyl]-2-fluorobenzoic acid | | | |
| 43 | 3-((6,7-Dichloro-1H-indazol-1-yl)methyl)-2-fluorobenzoic acid | 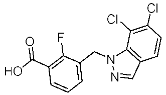 | ¹H NMR (DMSO) δ 8.33 (s, 1H), 7.85 (d, 1H), 7.77 (t, 1H), 7.41 (d, 1H), 7.18 (t, 1H), 6.90 (t, 1H), 6.07 (s, 2H). | / |
| | 3-((6,7-Dichloro-1H-indazol-1-yl)methyl)-2-fluorobenzoic acid | | | |

### Example 45

### 3-((5,7-Dichloro-6-(1H-pyrazol-1-yl)-1H-pyrazol-1-yl)methyl)-2-fluorobenzoic acid (Compound 45)

### 3-((5,7-Dichloro-6-(1H-pyrazol-1-yl)-1H-indazol-1-yl)methyl)-2-fluorobenzoic acid

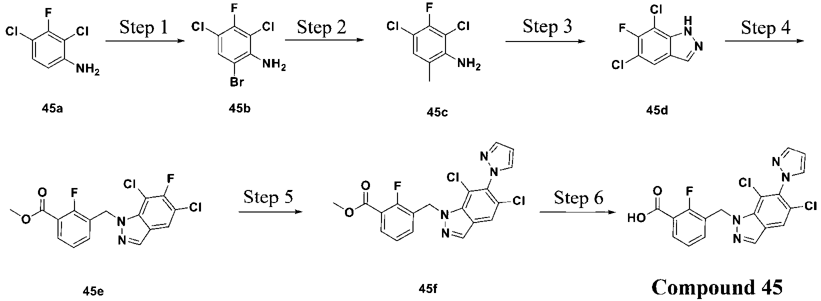

### Step 1:

### 6-Bromo-2,4-dichloro-3-fluoroaniline (45b)

### 6-Bromo-2,4-dichloro-3-fluoroaniline

In a 250 mL round-bottomed flask, **45a** (5 g, 27.78 mmol) and N-bromosuccinimide (5.44 g, 30.56 mmol) were dissolved in acetonitrile (50 mL) and reacted at room temperature for 6 h. The reaction was complete as monitored by TLC. The reaction liquid was filtered. Acetonitrile was removed under reduced pressure. The residue was subjected to column chromatography (PE : EA = 20 : 1) to obtain **45b** as a brown liquid (6.75 g, yield: 93.97%).

LC-MS m/z (ESI) = 260.1 [M+1].

### Step 2:

### 2,4-Dichloro-3-fluoro-6-methylaniline (45c)

### 2,4-Dichloro-3-fluoro-6-methylaniline

In a 250 mL round-bottomed flask, **45b** (6.76 g, 26.11 mmol), cesium carbonate (21.27 g, 65.28 mmol), methylboronic acid (2.03 g, 59.86 mmol), and DPPF palladium dichloride (1.91 g, 2.61 mmol) were dissolved in 1,4-dioxane (100 mL), and under nitrogen protection, the mixture was heated and reacted under reflux for 8 h. The reaction was complete as monitored by LCMS. 1,4-Dioxane was removed under reduced pressure. The residue was subjected to column chromatography (PE : EA = 20 : 1) to obtain **45c** as a brown liquid (4.19 g, yield: 82.71%).

LC-MS m/z (ESI) = 195.22 [M+1].

### Step 3:

### 5,7-Dichloro-6-fluoro-1H-indazole (45d)

### 5,7-Dichloro-6-fluoro-1H-indazole

In a 250 mL round-bottomed flask, **45c** (4.19 g, 21.59 mmol) was dissolved in glacial acetic acid (150 mL) and precooled at 0°C for 5 min, and a saturated aqueous solution of sodium nitrite (2.24 g, 32.39 mmol) was then added and reacted at room temperature for 6 h. The reaction was complete as monitored by TLC. The reaction liquid was poured into ice water. Next, the pH was adjusted to neutrality with saturated sodium bicarbonate. A solid was precipitated, and after suction filtration, a filter cake was collected to obtain **45d** as a yellow solid (1.6 g, yield: 36.53%).

LC-MS m/z (ESI) = 204.55 [M+1].

### Step 4:

### Methyl 3-((5,7-dichloro-6-fluoro-1H-indazol-1-yl)methyl)-2-fluorobenzoate (45e)

### Methyl 3-((5,7-dichloro-6-fluoro-1H-indazol-1-yl)methyl)-2-fluorobenzoate

In a 100 mL round-bottomed flask, **45d** (500 mg, 2.46 mmol), cesium carbonate (2 g, 6.15 mmol) and methyl 3-(bromomethyl)-2-fluorobenzoate (608.53 mg, 2.46 mmol) were dissolved in acetonitrile (15 mL), and the mixture was heated and reacted under reflux for 3 h. The reaction was complete as monitored by TLC. Acetonitrile was removed under reduced pressure. The residue was subjected to column chromatography (PE/EA = 20 : 1) to **45e** as a yellow solid (260 mg, yield: 28.48%).

LC-MS m/z (ESI) = 372.33 [M+1].

### Step 5:

### Methyl 3-((5,7-dichloro-6-(1H-pyrazol-1-yl)-1H-pyrazol-1-yl)methyl)-2-fluorobenzoate (45f)

### Methyl 3-((5,7-dichloro-6-(1H-pyrazol-1-yl)-1H-indazol-1-yl)methyl)-2-fluorobenzoate

In a 100 mL round-bottomed flask, **45e** (100 mg, 0.27 mmol), cesium carbonate (219.93 mg, 0.68 mmol), and pyrazole (27.51 mg, 0.41 mmol) were dissolved in acetonitrile (10 mL), and the mixture was heated and reacted under reflux for 3 h. The reaction was complete as monitored by TLC. Acetonitrile was removed under reduced pressure. The residue was subjected to column chromatography (PE/EA = 10 : 1) to **45f** as a yellowish white solid (70 mg, yield: 62.5%).

LC-MS m/z (ESI) = 417.33 [M+1].

### Step 6:

### 3-((5,7-Dichloro-6-(1H-pyrazol-1-yl)-1H-pyrazol-1-yl)methyl)-2-fluorobenzoic acid (Compound 45)

### 3-((5,7-Dichloro-6-(1H-pyrazol-1-yl)-1H-indazol-1-yl)methyl)-2-fluorobenzoic acid

In a 50 mL round-bottomed flask, **45d** (70 mg, 0.17 mmol) and sodium hydroxide (16.83 mg, 0.42 mmol) were dissolved in ethanol and water (8:2 mL), and the mixture was heated and reacted under reflux for 3 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 45** as a white solid (35 mg, yield: 51.47%).

### LC-MS m/z (ESI) = 413.96 [M+1].

¹H NMR (400 MHz, DMSO-*d*₆) δ 8.31 (s, 1H), 8.21 (s, 1H), 7.64 (t, 1H), 7.06 (t, 1H), 6.72 (s, 1H), 6.03 (s, 2H).

### Example 46

### 3-((5-Chloro-4-methyl-1H-indazol-1-yl)methyl)-2-fluorobenzoic acid (Compound 46)

### 3-((5-Chloro-4-methyl-1H-indazol-1-yl)methyl)-2-fluorobenzoic acid

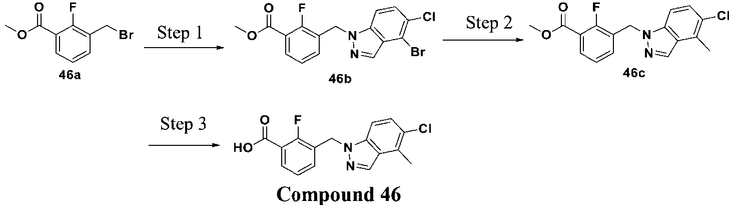

### Step 1:

### Methyl 3-((4-bromo-5-chloro-1H-indazol-1-yl)methyl)-2-fluorobenzoate (46b)

### Methyl 3-((4-bromo-5-chloro-1H-indazol-1-yl)methyl)-2-fluorobenzoate

In a 50 mL single-necked flask, **46a** (1.173 g, 4.75 mmol) and 4-bromo-5-chloro-1H-indazole (1.0 g, 4.3 mmol) were dissolved in 5 mL of acetonitrile, and cesium carbonate (3.5 g, 10.75 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **46b** as a white solid (300 mg, yield: 18%).

LCMS m/z (ESI) = 396.90[M+1].

¹H NMR (400 MHz, DMSO-*d*₆) δ 8.11 (d, 1H), 7.89 (d, 1H), 7.85 - 7.78 (m, 1H), 7.62 (d, 1H), 7.42 (d, 1H), 7.27 (t, 1H), 5.80 (s, 2H), 3.83 (s, 3H).

### Step 2:

### Methyl 3-((5-chloro-4-methyl-1H-indazol-1-yl)methyl)-2-fluorobenzoate (46c)

### Methyl 3-((5-chloro-4-methyl-1H-indazol-1-yl)methyl)-2-fluorobenzoate

In a 50 mL three-necked flask, **46b** (100 mg, 0.25 mmol), methylboronic acid (30 mg, 0.5 mmol), Pd (dppf)Cl₂ (18.3 mg, 0.025 mmol), and anhydrous cesium carbonate (203.6 mg, 0.625 mmol) were dissolved in 1,4-dioxane (3 mL). Under inert gas protection, the mixture was reacted at 105°C for 16 h. The reaction was complete as monitored by TLC. The solid was removed by filtration through diatomite. The resulting material was washed with ethyl acetate, and the organic solvent was removed under reduced pressure. The crude product was purified by normal phase chromatography (PE : EA = 8 : 1) to obtain **46c** as a white solid (70 mg, yield: 84%).

LCMS m/z (ESI) = 333.10 [M+1].

### Step 3:

### 3-((5-Chloro-4-methyl-1H-indazol-1-yl)methyl)-2-fluorobenzoic acid (Compound 46)

### 3-((5-Chloro-4-methyl-1H-indazol-1-yl)methyl)-2-fluorobenzoic acid

In a 50 mL single-necked flask, **46c** (70 mg, 0.21 mmol) was dissolved in a mixed solvent of methanol (2 mL) and water (0.5 mL), and sodium hydroxide (54 mg, 1.35 mmol) was added. The mixture was reacted at 60°C for 3 h. The reaction was complete as monitored by LCMS. The solvent was removed under reduced pressure, and the resulting material was diluted with water. The pH was adjusted to 3 with 1M dilute hydrochloric acid, and the solution was extracted with ethyl acetate. The organic phases were combined, dried with anhydrous Na₂SO₄, filtered, and concentrated. The crude product was purified by reversed-phase preparative thin-layer chromatography (20% acetonitrile aqueous solution) to obtain **Compound 46** as a white solid (46.7 mg, yield: 70%).

¹H NMR (400 MHz, DMSO-*d*₆) δ 13.31 (s, 1H), 8.25 (d, 1H), 7.77 (td, 1H), 7.62 (d, 1H), 7.41 (d, 1H), 7.33 - 7.24 (m, 1H), 7.21 (t, 1H), 5.74 (s, 2H), 2.56 (s, 3H).

### Example 47

### 3-((4-Chloro-5-cyano-1H-indazol-1-yl)methyl)-2-fluorobenzoic acid (Compound 47)

### 3-((4-Chloro-5-cyano-1H-indazol-1-yl)methyl)-2-fluorobenzoic acid

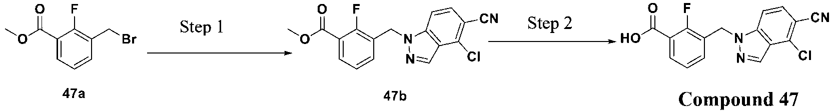

### Step 1:

### Methyl 3-((4-chloro-5-cyano-1H-indazol-1-yl)methyl)-2-fluorobenzoate (47b)

### Methyl 3-((4-chloro-5-cyano-1H-indazol-1-yl)methyl)-2-fluorobenzoate

In a 50 mL single-necked flask, **47a** (125 mg, 0.5 mmol) and 4-chloro-5-cyano-1H-indazole (177.59 mg, 0.5 mmol) were dissolved in 5 mL of acetonitrile, and cesium carbonate (407.2 g, 1.25 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 1 : 0-10 : 1) to obtain **47b** as a white solid (41 mg, yield: 24%).

LCMS m/z (ESI) = 344.0 [M+1].

¹H NMR (400 MHz, DMSO-*d*₆) δ 8.44 (d, 1H), 8.02 (dd, 1H), 7.89 (d, 1H), 7.86 - 7.80 (m, 1H), 7.54 - 7.46 (m, 1H), 7.29 (t, 1H), 5.86 (d, 2H), 3.83 (s, 3H).

### Step 2:

### 3-((4-Chloro-5-cyano-1H-indazol-1-yl)methyl)-2-fluorobenzoic acid (Compound 47)

### 3-((4-Chloro-5-cyano-1H-indazol-1-yl)methyl)-2-fluorobenzoic acid

In a 50 mL single-necked flask, **47b** (35 mg, 0.1 mmol) was dissolved in a mixed solvent of methanol (3 mL) and water (0.5 mL), and sodium hydroxide (20 mg, 0.5 mmol) was added. The mixture was reacted at 50°C for 3 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 47** as a white solid (7 mg, yield: 21%).

¹H NMR (400 MHz, DMSO-*d*₆) δ 13.35 (s, 1H), 8.45 (s, 1H), 8.02 (d, 1H), 7.90 (d, 1H), 7.80 (t, 1H), 7.44 (t, 1H), 7.25 (t, 1H), 5.85 (s, 2H).

### Example 48

### 3-((5-Chloro-4-fluoro-1H-indazol-1-yl)methyl)-2-fluorobenzoic acid (Compound 48)

### 3-((5-Chloro-4-fluoro-1H-indazol-1-yl)methyl)-2-fluorobenzoic acid

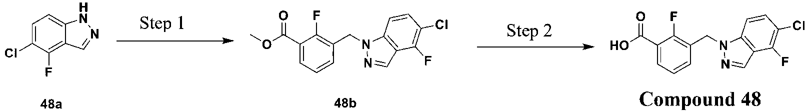

### Step 1:

### Methyl 3-((5-chloro-4-fluoro-1H-indazol-1-yl)methyl)-2-fluorobenzoate (48b)

### Methyl 3-((5-chloro-4-fluoro-1H-indazol-1-yl)methyl)-2-fluorobenzoate

In a 50 mL single-necked flask, **48a** (62 mg, 0.36 mmol) and methyl 3-(bromomethyl)-2-fluorobenzoate (90 mg, 0.36 mmol) were dissolved in 2 mL of acetonitrile, and cesium carbonate (297 mg, 0.91 mmol) was added. The mixture was reacted at 70°C for 2 h. The reaction was complete as monitored by TLC. The reaction liquid was filtered, and the organic solvent was removed under reduced pressure. The crude product was purified by silica gel column chromatography (PE:DCM = 10 : 1-1 : 2) to obtain **48b** as a yellow oil (72 mg, yield: 59.0%).

LCMS m/z (ESI) = 337.1 [M+1].

### Step 2:

### 3-((5-Chloro-4-fluoro-1H-indazol-1-yl)methyl)-2-fluorobenzoic acid (Compound 48)

### 3-((5-Chloro-4-fluoro-1H-indazol-1-yl)methyl)-2-fluorobenzoic acid

In a 50 mL single-necked flask, **48b** (72 mg, 0.21 mmol) was dissolved in a mixed solvent of ethanol (4 mL) and water (1 mL), and sodium hydroxide (34 mg, 0.86 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 48** as a white solid (38.2 mg, yield 55.1%).

¹H NMR (400 MHz, DMSO-*d*₆) δ 13.30 (s, 1H), 8.28 (s, 1H), 7.81 - 7.74 (m, 1H), 7.67 (d, J = 8.9 Hz, 1H), 7.54 (dd, J = 8.9, 6.9 Hz, 1H), 7.35 (t, J = 7.1 Hz, 1H), 7.21 (t, J = 7.7 Hz, 1H), 5.77 (s, 2H).

¹⁹F NMR (377 MHz, DMSO-*d*₆) δ -115.37, -120.86.

### Example 49

### 3-((5-Cyano-4-methyl-1H-indazol-1-yl)methyl)-2-fluorobenzoic acid (Compound 49)

### 3-((5-Cyano-4-methyl-1H-indazol-1-yl)methyl)-2-fluorobenzoic acid

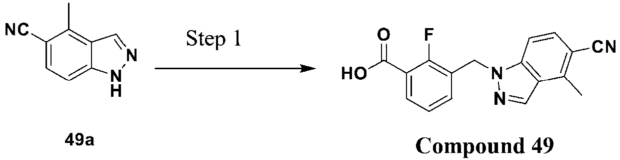

### Step 1:

### Methyl 2-fluoro-3-((5-morpholino-1H-indol-1-yl)methyl)benzoate (Compound 49) 2-Fluoro-3-((5-morpholino-1H-indol-1-yl)methyl)benzoate

In a 50 mL single-necked flask, **49a** (76 mg, 0.49 mmol) and methyl 3-(bromomethyl)-2-fluorobenzoate (120 mg, 0.49 mmol) were dissolved in 2 mL of acetonitrile, and cesium carbonate (396 mg, 1.21 mmol) was added. The mixture was reacted at 70°C for 2 h. The reaction was complete as monitored by TLC. The reaction liquid was filtered, and the organic solvent was removed under reduced pressure. The crude product was purified by medium pressure preparative reversed-phase chromatography (ACN/H₂O (TFA)) to obtain **Compound 49** as a white solid (44.36 mg, yield: 29.5%).

LCMS m/z (ESI) = 310.2 [M+1].

¹H NMR (400 MHz, DMSO-*d*₆) δ 8.90 (s, 1H), 7.82 (t, J = 7.2 Hz, 1H), 7.55 (dd, J = 8.4, 5.7 Hz, 2H), 7.38 (d, J = 8.9 Hz, 1H), 7.27 (t, J = 7.7 Hz, 1H), 5.77 (s, 2H), 2.69 (s, 3H).

¹⁹F NMR (377 MHz, DMSO-*d*₆) δ -115.79.

### Example 50

### 3-((5-Chloro-4-cyclopropyl-1H-indazol-1-yl)methyl)-2-fluorobenzoic acid (Compound 50)

### 3-((5-Chloro-4-cyclopropyl-1H-indazol-1-yl)methyl)-2-fluorobenzoic acid

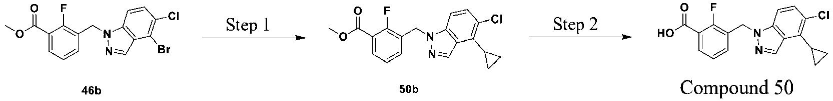

### Step 1:

### Methyl 3-((5-chloro-4-cyclopropyl-1H-indazol-1-yl)methyl)-2-fluorobenzoate (50b)

### Methyl 3-((5-chloro-4-cyclopropyl-1H-indazol-1-yl)methyl)-2-fluorobenzoate

In a 50 mL single-necked flask, **46b** (150 mg, 0.38 mmol) and cyclopropylboronic acid (65 mg, 0.76 mmol) were dissolved in 4 mL of 1,4-dioxane and 1 mL of water, and 1,1-bis(diphenylphosphine)palladium(II) dichloride (28 mg, 0.04 mmol) and cesium carbonate (309 mg, 0.95 mmol) were added. After nitrogen displacement, the mixture was reacted at 100°C for 8 h. The reaction was complete as monitored by TLC. The reaction liquid was filtered, and the organic solvent was removed under reduced pressure. The crude product was purified by silica gel column chromatography (PE : EA = 15 : 1-5 : 1) to obtain **50b** as a yellow oil (96 mg, yield: 70.8%).

LCMS m/z (ESI) = 359.2 [M+1].

### Step 2:

### 3-((5-Chloro-4-cyclopropyl-1H-indazol-1-yl)methyl)-2-fluorobenzoic acid (Compound 50)

### 3-((5-Chloro-4-cyclopropyl-1H-indazol-1-yl)methyl)-2-fluorobenzoic acid

In a 50 mL single-necked flask, **50b** (96 mg, 0.27 mmol) was dissolved in a mixed solvent of ethanol (4 mL) and water (1 mL), and sodium hydroxide (43 mg, 1.07 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 50** as a white solid (34.48 mg, yield 37.5%).

LCMS m/z (ESI) = 345.2[M+1].

¹H NMR (400 MHz, DMSO-*d*₆) δ 8.14 (d, J = 0.9 Hz, 1H), 7.74 (td, J = 7.4, 1.9 Hz, 1H), 7.61 (d, J = 8.9 Hz, 1H), 7.40 (d, J = 8.9 Hz, 1H), 7.27 (td, J = 7.2, 6.8, 1.9 Hz, 1H), 7.19 (t, J = 7.6 Hz, 1H), 5.71 (s, 2H), 2.25 (tt, J = 8.6, 5.4 Hz, 1H), 1.16 - 1.09 (m, 2H), 1.03 - 0.96 (m, 2H).

¹⁹F NMR (377 MHz, DMSO-*d*₆) δ -115.84.

### Example 51

### 3-((5,7-Dichloro-6-fluoro-1H-indazol-1-yl)methyl)-2-fluorobenzoic acid (Compound 51)

### 3-((5,7-Dichloro-6-fluoro-1H-indazol-1-yl)methyl)-2-fluorobenzoic acid

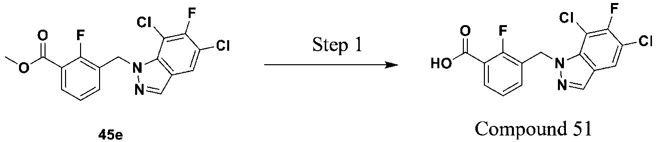

### Step 1:

### 3-((5,7-Dichloro-6-fluoro-1H-indazol-1-yl)methyl)-2-fluorobenzoic acid (Compound 51)

### 3-((5,7-Dichloro-6-fluoro-1H-indazol-1-yl)methyl)-2-fluorobenzoic acid

In a 50 mL single-necked flask, **45e** (60 mg, 0.16 mmol) was dissolved in a mixed solvent of ethanol (4 mL) and water (1 mL), and sodium hydroxide (26 mg, 0.65 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 51** as a white solid (25.74 mg, yield 44.6%).

LCMS m/z (ESI) = 357.2[M+1].

¹H NMR (400 MHz, DMSO-*d*₆) δ 13.34 (s, 1H), 8.28 (s, 1H), 8.13 (d, J = 6.8 Hz, 1H), 7.78 (td, J = 7.5, 1.8 Hz, 1H), 7.19 (t, J = 7.7 Hz, 1H), 6.99 (td, J = 7.3, 1.8 Hz, 1H), 6.01 (s, 2H).

¹⁹F NMR (377 MHz, DMSO-*d*₆) δ -116.39, -118.21.

### Example 52

### 3-((5,7-Dichloro-1H-indol-1-yl)methyl)-2-fluorobenzamide (Compound 52)

### 3-((5,7-Dichloro-1H-indol-1-yl)methyl)-2-fluorobenzamide

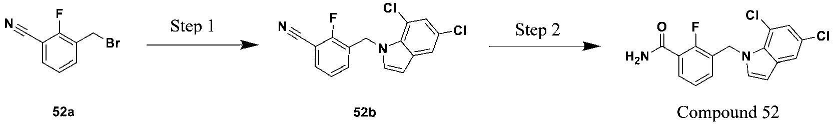

### Step 1:

### 3-((5,7-Dichloro-1H-indol-1-yl)methyl)-2-fluorobenzonitrile (52b)

### 3-((5,7-Dichloro-1H-indol-1-yl)methyl)-2-fluorobenzonitrile

In a 150 mL single-necked flask, **52a** (1.5 g, 7.04 mmol) and 5,7-dichloro-1H-indole (1303 mg, 7.04 mmol) were dissolved in 35 mL of acetonitrile, and cesium carbonate (5737 mg, 7.14 mmol) was added. The reaction was carried out at 70°C for 4 h. The reaction was complete as monitored by TLC. The reaction liquid was filtered, and the organic solvent was removed under reduced pressure. The crude product was purified by silica gel column chromatography (PE : EA = 30 : 1-20 : 1) to obtain **52b** as a white solid (684 mg, yield: 30.5%).

LCMS m/z (ESI) = 319.1 [M+1].

### Step 2:

### 3-((5,7-Dichloro-1H-indol-1-yl)methyl)-2-fluorobenzamide (Compound 52)

### 3-((5,7-Dichloro-1 H-indol-1-yl)methyl)-2-fluorobenzamide

In a 50 mL single-necked flask, **52b** (100 mg, 0.31 mmol) was dissolved in 2 mL of dimethyl sulfoxide, cesium carbonate (404 mg, 1.24 mmol) was added, and the mixture was cooled to 0°C. At this temperature, 1 mL of 30% hydrogen peroxide was dropwise added slowly, and the mixture was reacted at room temperature for 3 h. The reaction was complete as monitored by LCMS. The reaction was quenched with water, and the product was extracted with EA. The organic phase was dried over sodium sulfate, and the organic solvent was removed under reduced pressure. The crude product was purified by silica gel column chromatography (PE : EA = 5 : 1-2 : 1) and lyophilized to obtain **Compound 52** as a white solid (58.43 mg, yield: 54.8%).

LCMS m/z (ESI) = 337.1 [M+1].

¹H NMR (400 MHz, DMSO-*d*₆) δ 7.80 (s, 1H), 7.71 - 7.62 (m, 3H), 7.52 (td, 1H), 7.21 (d, 1H), 7.10 (t, J = 7.7 Hz, 1H), 6.65 (d, J = 3.2 Hz, 1H), 6.44 (td, J = 7.4, 1.7 Hz, 1H), 5.84 (s, 2H).

### Example 53

### 2-((5,7-Dichloro-1H-indol-1-yl)methyl)oxazole-4-carboxylic acid (Compound 53)

### 2-((5,7-Dichloro-1H-indol-1-yl)methyl)oxazole-4-carboxylic acid

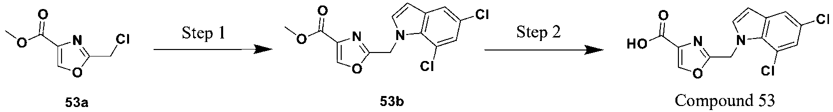

### Step 1:

### Methyl 2-((5,7-dichloro-1H-indol-1-yl)methyl)oxazole-4-carboxylate (53b)

### Methyl 2-((5,7-dichloro-1H-indol-1-yl)methyl)oxazole-4-carboxylate

In a 50 mL single-necked flask, **53a** (80 mg, 0.46 mmol) and 5,7-dichloro-1H-indole (93 mg, 0.50 mmol) were dissolved in 3 mL of acetonitrile, and cesium carbonate (371 mg, 1.14 mmol) was added. The reaction was carried out at 70°C for 2 h. The reaction was complete as monitored by TLC. The reaction liquid was filtered, and the organic solvent was removed under reduced pressure. The crude product was purified by silica gel column chromatography (PE : EA = 5 : 1-2 : 1) to obtain **53b** as a white solid (133 mg, yield: 90%).

LCMS m/z (ESI) = 325.1 [M+1].

### Step 2:

### 2-((5,7-Dichloro-1H-indol-1-yl)methyl)oxazole-4-carboxylic acid (Compound 53)

### 2-((5,7-Dichloro-1H-indol-1-yl)methyl)oxazole-4-carboxylic acid

In a 50 mL single-necked flask, **53b** (133 mg, 0.41 mmol) was dissolved in a mixed solvent of methanol (4 mL) and water (1 mL), and sodium hydroxide (25 mg, 0.625 mmol) was added. The mixture was reacted at 40°C for 2 h. The reaction was complete as monitored by LCMS. The reaction liquid was diluted with water, and the methanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 53** as a white solid (100.93 mg, yield: 79.3%).

LCMS m/z (ESI) = 311.0 [M+1].

¹H NMR (400 MHz, DMSO-*d*₆) δ 8.71 (s, 1H), 7.66 (d, 1H), 7.62 (d, 1H), 7.23 (d, 1H), 6.61 (d, 1H), 5.94 (s, 2H).

### Example 54

### 2-((7-Methyl-5-(trifluoromethyl)-1H-indol-1-yl)methyl)oxazole-4-carboxylic acid (Compound 54)

### 2-((7-Methyl-5-(trifluoromethyl)-1H-indol-1-yl)methyl)oxazole-4-carboxylic acid

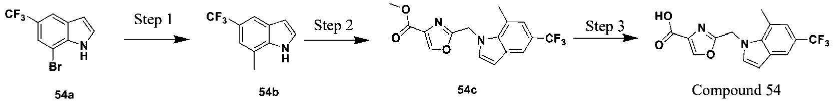

### Step 1:

### 7-Methyl-5-(trifluoromethyl)-1H-indole (54b)

### 7-Methyl-5-(trifluoromethyl)-1H-indole

In a 250 mL single-necked flask, **54a** (3 g, 11.4 mmol) and methylboronic acid (1.023 g, 17.05 mmol) were dissolved in 50 mL of 1,4-dioxane and 10 mL of water, and 1,1-bis(diphenylphosphine)palladium(II) dichloride (833 mg, 1.14 mmol) and cesium carbonate (9.256 g, 28.41 mmol) were added. After nitrogen displacement, the mixture was reacted at 100°C for 8 h. The reaction was complete as monitored by TLC. The reaction liquid was filtered, and the organic solvent was removed under reduced pressure. The crude product was purified by silica gel column chromatography (PE : EA = 20 : 0-5 : 1) to obtain **54b** as a white solid (2.22 g, yield: 98%).

LCMS m/z (ESI) = 200.1 [M+1].

### Step 2:

### Methyl-2-((7-methyl-5-(trifluoromethyl)-1H-indol-1-yl)methyl)oxazole-4-carboxylate (54c)

### Methyl-2-((7-methyl-5-(trifluoromethyl)-1H-indol-1-yl)methyl)oxazole-4-carboxylate

In a 50 mL single-necked flask, **54b** (91 mg, 0.46 mmol) and methyl 2-(chloromethyl)oxazole-4-carboxylate (80 mg, 0.46 mmol) were dissolved in 2 mL of acetonitrile, and cesium carbonate (371 mg, 1.14 mmol) was added. The reaction was carried out at 70°C for 1 h. The reaction was complete as monitored by TLC. The reaction liquid was filtered, and the organic solvent was removed under reduced pressure. The crude product was purified by silica gel column chromatography (PE : EA = 10 : 1-4 : 1) to obtain **54c** as a white solid (97 mg, yield: 62.8%).

LCMS m/z (ESI) = 339.1 [M+1].

### Step 3:

### 2-((7-Methyl-5-(trifluoromethyl)-1H-indol-1-yl)methyl)oxazole-4-carboxylic acid (Compound 54)

### 2-((7-Methyl-5-(trifluoromethyl)-1H-indol-1-yl)methyl)oxazole-4-carboxylic acid

In a 50 mL single-necked flask, **54c** (97 mg, 0.26 mmol) was dissolved in a mixed solvent of methanol (4 mL) and water (1 mL), and sodium hydroxide (15 mg, 0.38 mmol) was added. The mixture was reacted at 50°C for 2 h. The reaction was complete as monitored by LCMS. The reaction liquid was diluted with water, and the methanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 54** as a white solid (42 mg, yield: 45.2%).

LCMS m/z (ESI) = 325.1 [M+1].

¹H NMR (400 MHz, DMSO-*d*₆) δ 8.72 (s, 1H), 7.85 - 7.79 (m, 1H), 7.59 (d, 1H), 7.18 - 7.10 (m,1H), 6.67 (d, 1H), 5.89 (s, 2H), 2.66 (s, 3H).

### Example 55

### 3-(3-((5,7-Dichloro-1H-indol-1-yl)methyl)-2-fluorophenyl)-1,2,4-oxadiazol-5(4H)-one (Compound 55)

### 3-(3-((5,7-Dichloro-1H-indol-1-yl)methyl)-2-fluorophenyl)-1,2,4-oxadiazol-5(4H)-one

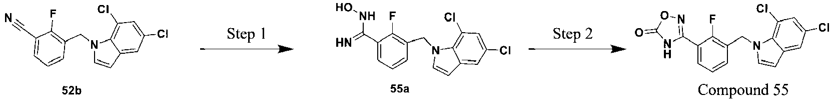

### Step 1:

### 3-((5,7-Dichloro-1H-indol-1-yl)methyl)-2-fluoro-N-hydroxybenzimidamide (55a)

### 3-((5,7-Dichloro-1H-indol-1-yl)methyl)-2-fluoro-N-hydroxybenzimidamide

In a 50 mL single-necked flask, **52b** (150 mg, 0.45 mmol) was dissolved in 3 mL of dimethyl sulfoxide, and hydroxylamine hydrochloride (38 mg, 0.57 mmol) and sodium carbonate (100 mg, 0.94 mmol) were added. The mixture was reacted at 80°C for 4 h. The reaction was complete as monitored by TLC. The reaction was quenched with water. A solid was precipitated, and the precipitated solid was filtered, washed with water, and dried under reduced pressure to obtain the desired **55a** as a white solid (148 mg, yield: 89.3%).

LCMS m/z (ESI) = 352.1 [M+1].

### Step 2:

### 3-(3-((5,7-Dichloro-1H-indol-1-yl)methyl)-2-fluorophenyl)-1,2,4-oxadiazol-5(4H)-one (Compound 55)

### 3-(3-((5,7-Dichloro-1H-indol-1-yl)methyl)-2-fluorophenyl)-1,2,4-oxadiazol-5(4H)-one

In a 50 mL single-necked flask, **55a** (148 mg, 0.42 mmol) was dissolved in 3 mL of dichloromethane, and N,N'-carbonyldiimidazole (75 mg, 0.46 mmol) and 1,8-diazabicyclo[5,4,0]undec-7-ene (128 mg, 0.84 mmol) were added. The mixture was reacted at room temperature overnight. The reaction was complete as monitored by LCMS. and the solvent was removed under reduced pressure. The crude product was purified by medium pressure preparative reversed-phase chromatography (ACN/H₂O) and lyophilized to obtain **Compound 55** as a white solid (2 mg, yield: 1.3%).

LCMS m/z (ESI) = 378.1 [M+1].

¹H NMR (400 MHz, DMSO-*d*₆) δ 7.90 (s, 1H), 7.71 - 7.53 (m, 3H), 7.48 (td, 1H), 7.02 (d, 1H), 6.82 (d, 1H), 6.44 (td, 1H), 5.60 (s, 2H).

### Example 56

### 5,7-Dichloro-1-(2-fluoro-3-(2H-tetrazol-5-yl)benzyl)-1H-indole (Compound 56)

### 5,7-Dichloro-1-(2-fluoro-3-(2H-tetrazol-5-yl)benzyl)-1H-indole

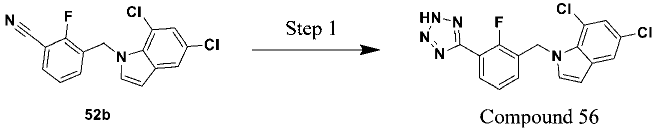

### Step 1:

In a 50 mL single-necked flask, **52b** (100 mg, 0.31 mmol) was dissolved in 3 mL of toluene, and trimethylsilyl azide (73 mg, 0.63 mmol) and dibutyltin oxide (22 mg, 0.06 mmol) were added. The mixture was reacted at 100°C for 8 h. The reaction was complete as monitored by LCMS. and the solvent was removed under reduced pressure. The crude product was purified by silica gel column chromatography (DCM : MeOH = 30 : 1-15 : 1) to obtain **Compound 56** as a white solid (18.8 mg, yield: 16.6%).

LCMS m/z (ESI) = 378.1 [M+1].

¹H NMR (400 MHz, DMSO-*d*₆) δ 8.64 (d, 1H), 8.58 (d, 1H), 8.55 - 8.48 (m, 1H), 7.98(t, 1H), 7.91 - 7.83 (m, 1H), 7.62 (td, 1H), 7.33 (t, 1H), 5.59 (s, 2H).

### Example 57

### 5-((5,7-Dichloro-1H-indol-1-yl)methyl)oxazole-2-carboxylic acid (Compound 57)

### 5-((5,7-Dichloro-1H-indol-1-yl)methyl)oxazole-2-carboxylic acid

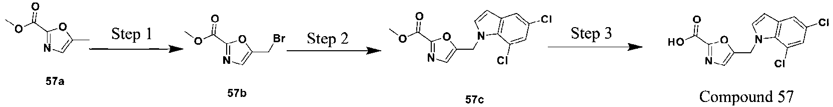

### Step 1:

### Methyl 5-(bromomethyl)oxazole-2-carboxylate (57b)

### Methyl 5-(bromomethyl)oxazole-2-carboxylate

In a 50 mL single-necked flask, **57a** (250 mg, 1.77 mmol) was dissolved in 9 mL of dichloroethane, and N-bromosuccinimide (315 mg, 1.77 mmol) and azodiisobutyronitrile (29 mg, 0.18 mmol) were added. The mixture was reacted at 80°C for 4 h. The reaction was complete as monitored by LCMS. and the solvent was removed under reduced pressure. The crude product was purified by silica gel column chromatography (EA : PE = 10 : 1-2 : 1) to obtain **57b** as a white solid (360 mg, yield: 92.7%).

LCMS m/z (ESI) = 219.9 [M+1].

### Step 2:

### Methyl 5-((5,7-dichloro-1H-indol-1-yl)methyl)oxazole-2-carboxylate (57c)

### Methyl 5-((5,7-dichloro-1H-indol-1-yl)methyl)oxazole-2-carboxylate

In a 50 mL single-necked flask, **57b** (360 mg, 1.64 mmol) and 5,7-dichloro-1H-indole (306 mg, 7.04 mmol) were dissolved in 4 mL of acetonitrile, and cesium carbonate (1.34 g, 4.11 mmol) was added. The reaction was carried out at 70°C for 2 h. The reaction was complete as monitored by TLC. The reaction liquid was filtered, and the organic solvent was removed under reduced pressure. The crude product was purified by silica gel column chromatography (PE : EA = 10 : 1-1 : 1) to obtain **57c** as a white solid (140 mg, yield: 26.3%).

LCMS m/z (ESI) 325.1 [M+1].

### Step 3:

### 5-((5,7-Dichloro-1H-indol-1-yl)methyl)oxazole-2-carboxylic acid (Compound 57)

### 5-((5,7-Dichloro-1H-indol-1-yl)methyl)oxazole-2-carboxylic acid

In a 50 mL single-necked flask, **57c** (140 mg, 0.43 mmol) was dissolved in a mixed solvent of ethanol (4 mL) and water (1 mL), and sodium hydroxide (26 mg, 0.64 mmol) was added. The reaction was carried out at 70°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 57** as a white solid (31.58 mg, yield 23.6 %).

LCMS m/z (ESI) = 311.0 [M+1].

¹H NMR (400 MHz, DMSO-*d*₆) δ 8.29 (s, 1H), 7.62 (dd, 2H), 7.24 (d, 1H), 7.08 (s,1H), 6.58 (d, 1H), 5.83 (d, 2H).

### Example 58

### 2-Fluoro-3-((7-(methoxymethyl)-5-(trifluoromethyl)-1H-indol-1-yl)methyl)benzoic acid (Compound 58)

### 2-Fluoro-3-((7-(methoxymethyl)-5-(trifluoromethyl)-1H-indol-1-yl)methyl)benzoic acid

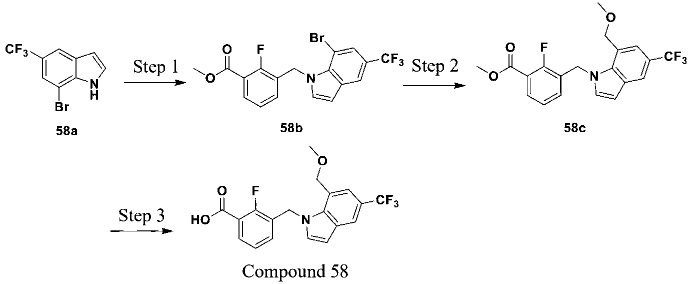

### Step 1:

### Methyl 3-((7-bromo-5-(trifluoromethyl)-1H-indol-1-yl)methyl)-2-fluorobenzoate (58b)

### Methyl 3-((7-bromo-5-(trifluoromethyl)-1H-indol-1-yl)methyl)-2-fluorobenzoate

In a 100 mL single-necked flask, **58a** (1 g, 4.05 mmol) and methyl 3-(bromomethyl)-2-fluorobenzoate (1.07 g, 4.05 mmol) were dissolved in 20 mL of acetonitrile, and cesium carbonate (3.3 g, 10.12 mmol) was added. The mixture was reacted at 70°C for 3 h. The reaction was complete as monitored by TLC. The reaction liquid was filtered, and the organic solvent was removed under reduced pressure. The crude product was purified by silica gel column chromatography (PE : EA = 20 : 1-10 : 1) to obtain **58b** as a light yellow solid (1.08 g, yield: 62.2%).

LCMS m/z (ESI) = 430.1 [M+1].

### Step 2:

### Methyl-2-fluoro-3-((7-(methoxymethyl)-5-(trifluoromethyl)-1H-indol-1-yl)methyl)benzoate (58c)

### Methyl-2-fluoro-3-((7-(methoxymethyl)-5-(trifluoromethyl)-1H-indol-1-yl)methyl)benzoate

In a 50 mL single-necked flask, **58b** (160 mg, 0.37 mmol) and potassium methoxymethyltrifluoroborate (170 mg, 1.12 mmol) were dissolved in 4 mL of 1,4-dioxane and 1 mL of water, and 1,1-bis(diphenylphosphine)palladium(II) dichloride (27 mg, 0.04 mmol) and cesium carbonate (365 mg, 1.12 mmol) were added. After nitrogen displacement, the mixture was reacted at 110°C for 8 h. The reaction was complete as monitored by TLC. The reaction liquid was filtered, and the organic solvent was removed under reduced pressure. The crude product was purified by silica gel column chromatography (PE : EA = 10 : 0-1 : 1) to obtain **58c** as a yellow oil (60 mg, yield: 40.7%).

LCMS m/z (ESI) = 396.2 [M+1].

### Step 3:

### 2-Fluoro-3-((7-(methoxymethyl)-5-(trifluoromethyl)-1H-indol-1-yl)methyl)benzoic acid (Compound 58)

### 2-Fluoro-3-((7-(methoxymethyl)-5-(trifluoromethyl)-1H-indol-1-yl)methyl)benzoic acid

In a 50 mL single-necked flask, **58c** (60 mg, 0.15 mmol) was dissolved in a mixed solvent of ethanol (4 mL) and water (1 mL), and sodium hydroxide (24 mg, 0.60 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 58** as a white solid (5 mg, yield 8.6%).

LCMS m/z (ESI) = 382.2[M+1].

¹H NMR (400 MHz, DMSO-*d*₆) δ 8.04 (d, 1H), 7.78 - 7.69 (m, 1H), 7.62 (d, 1H), 7.39 (d, 1H), 7.11 (t, 1H), 6.81 (d, 1H), 6.37 (t, 1H), 5.78 (s, 2H), 4.47 (s, 2H), 3.21 (s, 3H).

¹⁹F NMR (377 MHz, DMSO-*d*₆) δ -58.74, -117.01.

### Example 59

### 3-((5-Ethoxy-1H-indol-1-yl)methyl)-2-fluorobenzoic acid (Compound 59)

### 3-((5-Ethoxy-1H-indol-1-yl)methyl)-2-fluorobenzoic acid

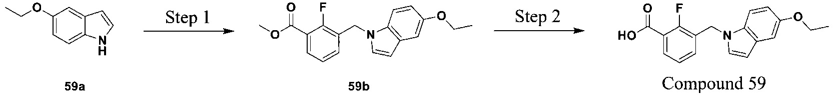

### Step 1:

### Methyl 3-((5-ethoxy-1H-indol-1-yl)methyl)-2-fluorobenzoate (59b)

### Methyl 3-((4-chloro-5-methoxy-1H-indol-1-yl)methyl)-2-fluorobenzoate

In a 50 mL single-necked flask, **59a** (65 mg, 0.4 mmol) and methyl 3-(bromomethyl)-2-fluorobenzoate (100 mg, 0.4 mmol) were dissolved in 2 mL of acetonitrile, and cesium carbonate (330 mg, 1.01 mmol) was added. The mixture was reacted at 70°C for 2 h. The reaction was complete as monitored by TLC. The reaction liquid was filtered, and the organic solvent was removed under reduced pressure. The crude product was purified by silica gel column chromatography (PE : EA = 20 : 1-10 : 1) to obtain **59b** as a white oil (105 mg, yield: 79.3%).

LCMS m/z (ESI) =328.2 [M+1].

### Step 2:

### 3-((5-Ethoxy-1H-indol-1-yl)methyl)-2-fluorobenzoic acid (Compound 59)

### 3-((5-Ethoxy-1H-indol-1-yl)methyl)-2-fluorobenzoic acid

In a 50 mL single-necked flask, **59b** (105 mg, 0.32 mmol) was dissolved in a mixed solvent of ethanol (4 mL) and water (1 mL), and sodium hydroxide (51 mg, 1.28 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 59** as a white solid (38.37 mg, yield 38.2%).

LCMS m/z (ESI) = 314.2[M+1].

¹H NMR (400 MHz, DMSO-*d*₆) δ 13.30 (s, 1H), 7.74 (td, 1H), 7.40 (d, 1H), 7.34 (d, 1H), 7.22 - 7.10 (m, 2H), 7.05 (d, 1H), 6.75 (dd, 1H), 6.39 (dd, 1H), 5.46 (s, 2H), 3.99 (q, J = 6.9 Hz, 2H), 1.32 (t, 3H).

¹⁹F NMR (377 MHz, DMSO-*d*₆) δ -115.84.

### Example 60

### 3-((4-Chloro-5-methoxy-1H-indol-1-yl)methyl)-2-fluorobenzoic acid (Compound 60)

### 3-((4-Chloro-5-methoxy-1H-indol-1-yl)methyl)-2-fluorobenzoic acid

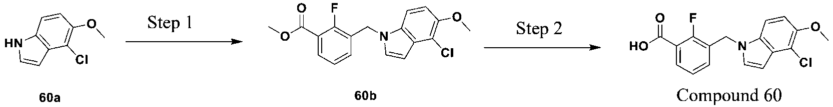

### Step 1:

### Methyl 3-((4-chloro-5-methoxy-1H-indol-1-yl)methyl)-2-fluorobenzoate (60b)

### Methyl 3-((4-chloro-5-methoxy-1H-indol-1-yl)methyl)-2-fluorobenzoate

In a 50 mL single-necked flask, 4-chloro-5-methoxy-1H-indole **60a** (74 mg, 0.4 mmol) and methyl 3-(bromomethyl)-2-fluorobenzoate (100 mg, 0.4 mmol) were dissolved in 2 mL of acetonitrile, and cesium carbonate (330 mg, 1.01 mmol) was added. The reaction was carried out at 70°C for 2 h. The reaction was complete as monitored by TLC. The reaction liquid was filtered, and the organic solvent was removed under reduced pressure. The crude product was purified by silica gel column chromatography (PE : EA = 20 : 1-10 : 1) to obtain **60b** as a white oil (120 mg, yield: 85.4%).

LCMS m/z (ESI) = 348.1 [M+1].

### Step 2:

### 3-((4-Chloro-5-methoxy-1H-indol-1-yl)methyl)-2-fluorobenzoic acid (Compound 60)

### 3-((4-Chloro-5-methoxy-1H-indol-1-yl)methyl)-2-fluorobenzoic acid

In a 50 mL single-necked flask, **60b** (120 mg, 0.35 mmol) was dissolved in a mixed solvent of ethanol (4 mL) and water (1 mL), and sodium hydroxide (55 mg, 1.38 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 60** as a white solid (37.65 mg, yield 32.7%).

LCMS m/z (ESI) = 334.1 [M+1].

¹H NMR (400 MHz, DMSO-*d*₆) δ 13.33 (s, 1H), 7.76 (dq, 1H), 7.55 (d, J = 3.1 Hz, 1H), 7.43 (d, 1H), 7.22 - 7.16 (m, 2H), 7.04 (d, 1H), 6.45 (dd, 1H), 5.52 (s, 2H), 3.83 (s, 3H).

¹⁹F NMR (377 MHz, DMSO-*d*₆) δ -115.55.

### Example 61

### 2-Fluoro-3-((5-isopropoxy-1H-indol-1-yl)methyl)benzoic acid (Compound 61)

### 2-Fluoro-3-((5-isopropoxy-1H-indol-1-yl)methyl)benzoic acid

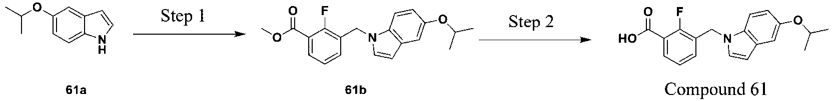

### Step 1:

### Methyl 2-fluoro-3-((5-isopropoxy-1H-indol-1-yl)methyl)benzoate (61b)

### Methyl 2-fluoro-3-((5-isopropoxy-1H-indol-1-yl)methyl)benzoate

In a 50 mL single-necked flask, 5-isopropoxy-1H-indole **61a** (71 mg, 0.4 mmol) and methyl 3-(bromomethyl)-2-fluorobenzoate (100 mg, 0.4 mmol) were dissolved in 2 mL of acetonitrile, and cesium carbonate (330 mg, 1.01 mmol) was added. The reaction was carried out at 70°C for 2 h. The reaction was complete as monitored by TLC. The reaction liquid was filtered, and the organic solvent was removed under reduced pressure. The crude product was purified by silica gel column chromatography (PE : EA = 20 : 1-10 : 1) to obtain **61b** as a yellow oil (129 mg, yield: 93.5%).

LCMS m/z (ESI) = 342.2 [M+1].

### Step 2:

### 2-Fluoro-3-((5-isopropoxy-1H-indol-1-yl)methyl)benzoic acid (Compound 61)

### 2-Fluoro-3-((5-isopropoxy-1H-indol-1-yl)methyl)benzoic acid

In a 50 mL single-necked flask, **61b** (129 mg, 0.38 mmol) was dissolved in a mixed solvent of ethanol (4 mL) and water (1 mL), and sodium hydroxide (61 mg, 1.51 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 61** as a white solid (40.32 mg, yield 32.6%).

LCMS m/z (ESI) = 328.2[M+1].

¹H NMR (400 MHz, DMSO-*d*₆) δ 13.31 (s, 1H), 7.80 - 7.70 (m, 1H), 7.40 (d, 1H), 7.33 (d, 1H), 7.22 - 7.14 (m, 2H), 7.06 (d, 1H), 6.73 (dd, 1H), 6.38 (dd, 1H), 5.46 (s, 2H), 4.50 (p, 1H), 1.24 (d, 6H).

¹⁹F NMR (377 MHz, DMSO-*d*₆) δ -115.74.

### Example 62

### 3-((4-Chloro-5-methyl-1H-indol-1-yl)methyl)-2-fluorobenzoic acid (Compound 62)

### 3-((4-Chloro-5-methyl-1H-indol-1-yl)methyl)-2-fluorobenzoic acid

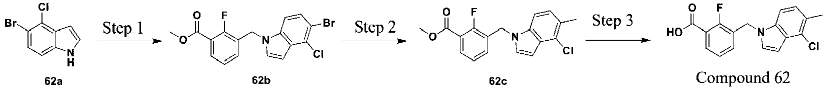

### Step 1:

### Methyl 3-((5-bromo-4-chloro-1H-indol-1-yl)methyl)-2-fluorobenzoate (62b)

### Methyl 3-((5-bromo-4-chloro-1H-indol-1-yl)methyl)-2-fluorobenzoate

In a 50 mL single-necked flask, 5-bromo-4-chloro-1H-indole **62a** (588 mg, 2.55 mmol) and methyl 3-(bromomethyl)-2-fluorobenzoate (700 mg, 2.55 mmol) were dissolved in 12 mL of acetonitrile, and cesium carbonate (2.078 g, 6.38 mmol) was added. The reaction was carried out at 70°C for 2 h. The reaction was complete as monitored by TLC. The reaction liquid was filtered, and the organic solvent was removed under reduced pressure. The crude product was purified by silica gel column chromatography (PE : EA = 20 : 1-10 : 1) to obtain **62b** as a yellow oil (851 mg, yield: 84.5%).

LCMS m/z (ESI) = 395.9 [M+1].

### Step 2:

### Methyl 3-((4-chloro-5-methyl-1H-indol-1-yl)methyl)-2-fluorobenzoate (62c)

### Methyl 3-((4-chloro-5-methyl-1H-indol-1-yl)methyl)-2-fluorobenzoate

In a 50 mL single-necked flask, **62b** (160 mg, 0.38 mmol) and methylboronic acid (46 mg, 0.76 mmol) were dissolved in 4 mL of 1,4-dioxane and 1 mL of water, and 1,1-bis(diphenylphosphine)palladium(II) dichloride (28 mg, 0.04 mmol) and cesium carbonate (309 mg, 0.95 mmol) were added. After nitrogen displacement, the mixture was reacted at 110°C for 8 h. The reaction was complete as monitored by TLC. The reaction liquid was filtered, and the organic solvent was removed under reduced pressure. The crude product was purified by silica gel column chromatography (PE : EA = 20 : 1-10 : 1) to obtain **62c** as a yellow oil (119 mg, yield: 95.2%).

LCMS m/z (ESI) = 332.2 [M+1].

### Step 3:

### 2-Fluoro-3-((5-isopropoxy-1H-indol-1-yl)methyl)benzoic acid (Compound 62)

### 2-Fluoro-3-((5-isopropoxy-1H-indol-1-yl)methyl)benzoic acid

In a 50 mL single-necked flask, **62c** (119 mg, 0.36 mmol) was dissolved in a mixed solvent of ethanol (4 mL) and water (1 mL), and sodium hydroxide (58 mg, 1.44 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 62** as a white solid (49.85 mg, yield 43.9%).

LCMS m/z (ESI) = 318.1 [M+1].

¹H NMR (400 MHz, DMSO-*d*₆) δ 7.73 (td, 1H), 7.50 (d, 1H), 7.35 (d, 1H), 7.20 - 7.10 (m, 2H), 7.05 (d, 1H), 6.46 (d, 1H), 5.50 (s, 2H), 2.36 (s, 3H).

¹⁹F NMR (377 MHz, DMSO-*d*₆) δ -115.54.

### Example 63

### 2-Fluoro-3-((5-methoxy-4-methyl-1H-indol-1-yl)methyl)benzoic acid (Compound 63)

### 2-Fluoro-3-((5-methoxy-4-methyl-1H-indol-1-yl)methyl)benzoic acid

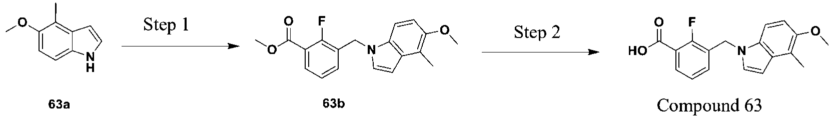

### Step 1:

### 2-Fluoro-3-((5-methoxy-4-methyl-1H-indol-1-yl)methyl)benzoate (63b)

### 2-Fluoro-3-((5-methoxy-4-methyl-1H-indol-1-yl)methyl)benzoate

In a 50 mL single-necked flask, **63a** (59 mg, 0.36 mmol) and methyl 3-(bromomethyl)-2-fluorobenzoate (90 mg, 0.36 mmol) were dissolved in 2 mL of acetonitrile, and cesium carbonate (297 mg, 0.91 mmol) was added. The mixture was reacted at 70°C for 2 h. The reaction was complete as monitored by TLC. The reaction liquid was filtered, and the organic solvent was removed under reduced pressure. The crude product was purified by silica gel column chromatography (PE : DCM = 10 : 1-2 : 1) to obtain **63b** as a yellow oil (34 mg, yield: 28.3%).

LCMS m/z (ESI) =328.2 [M+1].

### Step 2:

### 2-Fluoro-3-((5-methoxy-4-methyl-1H-indol-1-yl)methyl)benzoic acid (Compound 63)

### 2-Fluoro-3-((5-methoxy-4-methyl-1H-indol-1-yl)methyl)benzoic acid

In a 50 mL single-necked flask, **63b** (34 mg, 0.10 mmol) was dissolved in a mixed solvent of ethanol (4 mL) and water (1 mL), and sodium hydroxide (17 mg, 0.42 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 63** as a white solid (10.47 mg, yield 32.2%).

LCMS m/z (ESI) = 314.2[M+1].

¹H NMR (400 MHz, DMSO-*d*₆) δ 7.74 (td, 1H), 7.41 (d, 1H), 7.25 - 7.09 (m, 3H), 6.87 (d, 1H), 6.45 (dd, 1H), 5.46 (s, 2H), 3.75 (s, 3H), 2.30 (s, 3H).

¹⁹F NMR (377 MHz, DMSO-*d*₆) δ -115.74.

### Example 64

### 2-Fluoro-3-((5-morpholino-1H-indol-1-yl)methyl)benzoic acid (Compound 64)

### 2-Fluoro-3-((5-morpholino-1H-indol-1-yl)methyl)benzoic acid

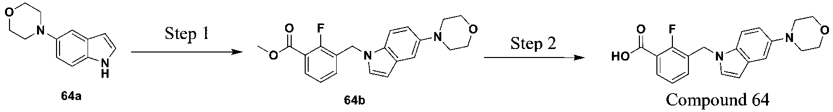

### Step 1:

### Methyl 2-fluoro-3-((5-morpholino-1H-indol-1-yl)methyl)benzoate (64b)

### 2-Fluoro-3-((5-morpholino-1H-indol-1-yl)methyl)benzoate

In a 50 mL single-necked flask, **64a** (74 mg, 0.36 mmol) and methyl 3-(bromomethyl)-2-fluorobenzoate (90 mg, 0.36 mmol) were dissolved in 2 mL of acetonitrile, and cesium carbonate (297 mg, 0.91 mmol) was added. The mixture was reacted at 70°C for 2 h. The reaction was complete as monitored by TLC. The reaction liquid was filtered, and the organic solvent was removed under reduced pressure. The crude product was purified by silica gel column chromatography (PE : DCM = 10 : 1-1 : 2) to obtain **64b** as a yellow oil (80 mg, yield: 59.7%).

LCMS m/z (ESI) = 369.2 [M+1].

### Step 2:

### 2-Fluoro-3-((5-morpholino-1H-indol-1-yl)methyl)benzoic acid (Compound 64)

### 2-Fluoro-3-((5-morpholino-1H-indol-1-yl)methyl)benzoic acid

In a 50 mL single-necked flask, **64b** (80 mg, 0.22 mmol) was dissolved in a mixed solvent of ethanol (4 mL) and water (1 mL), and sodium hydroxide (35 mg, 0.87 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 64** as a white solid (42.6 mg, yield 55.3%).

LCMS m/z (ESI) = 355.2[M+1].

¹H NMR (400 MHz, DMSO-*d*₆) δ 13.22 (s, 1H), 7.74 (td, 1H), 7.39 - 7.31 (m, 2H), 7.21 - 7.11 (m, 2H), 7.03 (d, 1H), 6.90 (dd, 1H), 6.38 (d, 1H), 5.46 (s, 2H), 3.81 - 3.67 (m, 4H), 3.05 - 2.95 (m, 4H).

¹⁹F NMR (377 MHz, DMSO-*d*₆) δ -115.74.

### Example 65

### 3-((7-Cyano-5-(trifluoromethyl)-1H-indol-1-yl)methyl)-2-fluorobenzoic acid (Compound 65)

### 3-((7-Cyano-5-(trifluoromethyl)-1H-indol-1-yl)methyl)-2-fluorobenzoic acid

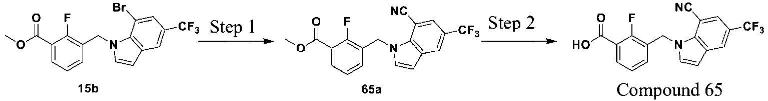

### Step 1:

### Methyl 3-((7-cyano-5-(trifluoromethyl)-1H-indol-1-yl)methyl)-2-fluorobenzoate (65a)

### Methyl 3-((7-cyano-5-(trifluoromethyl)-1H-indol-1-yl)methyl)-2-fluorobenzoate

In a 50 mL single-necked flask, **15b** (160 mg, 0.37 mmol) and zinc cyanide (87 mg, 0.75 mmol) were dissolved in 4 mL N,N-dimethylformamide, and tetrakis(triphenylphosphine)palladium (43 mg, 0.04 mmol) was added. After nitrogen displacement, the mixture was reacted at 100°C for 8 h. The reaction was complete as monitored by LCMS. The reaction liquid was filtered, and the organic solvent was removed under reduced pressure. The crude product was purified by medium pressure preparative reversed-phase chromatography (ACN/H₂O (TFA)) to obtain **65a** as a white solid (4 mg, yield: 2.9%).

LCMS m/z (ESI) = 377.2 [M+1].

### Step 2:

### 3-((7-Cyano-5-(trifluoromethyl)-1H-indol-1-yl)methyl)-2-fluorobenzoic acid (Compound 65)

### 3-((7-Cyano-5-(trifluoromethyl)-1H-indol-1-yl)methyl)-2-fluorobenzoic acid

In a 50 mL single-necked flask, **65a** (4 mg, 0.01 mmol) was dissolved in a mixed solvent of ethanol (2 mL) and water (0.5 mL), and sodium hydroxide (35 mg, 0.87 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 65** as a white solid (0.66 mg, yield: 55.3%).

LCMS m/z (ESI) = 363.2 [M+1].

### Example 66

### 3-((5-Chloro-4-fluoro-1H-indol-1-yl)methyl)-2-fluorobenzoic acid (Compound 66)

### 3-((5-Chloro-4-fluoro-1H-indol-1-yl)methyl)-2-fluorobenzoic acid

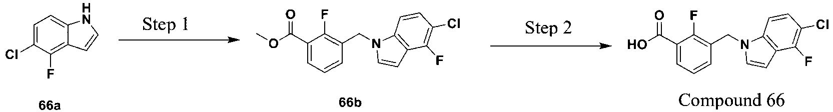

### Step 1:

### Methyl 3-((5-chloro-4-fluoro-1H-indol-1-yl)methyl)-2-fluorobenzoate (66b)

### Methyl 3-((5-chloro-4-fluoro-1H-indol-1-yl)methyl)-2-fluorobenzoate

In a 50 mL single-necked flask, 5-chloro-4-fluoro-1H-indole **66a** (62 mg, 0.36 mmol) and methyl 3-(bromomethyl)-2-fluorobenzoate (90 mg, 0.36 mmol) were dissolved in 3 mL of acetonitrile, and cesium carbonate (297 mg, 0.91 mmol) was added. The reaction was carried out at 70°C for 2 h. The reaction was complete as monitored by TLC. The reaction liquid was filtered, and the organic solvent was removed under reduced pressure. The crude product was purified by silica gel column chromatography (PE : DCM = 10 : 1-2 : 1) to obtain **66b** as a white oil (110 mg, yield: 90.2%).

LCMS m/z (ESI) 336.1 [M+1].

### Step 2:

### 3-((5-Chloro-4-fluoro-1H-indol-1-yl)methyl)-2-fluorobenzoic acid (Compound 66)

### 3-((5-Chloro-4-fluoro-1H-indol-1-yl)methyl)-2-fluorobenzoic acid

In a 50 mL single-necked flask, **66b** (110 mg, 0.33 mmol) was dissolved in a mixed solvent of ethanol (4 mL) and water (1 mL), and sodium hydroxide (53 mg, 1.31 mmol) was added. The mixture was reacted at 50°C for 2 h. The reaction was complete as monitored by LCMS. The reaction liquid was diluted with water, and the ethanol was removed by rotary evaporation. The pH was adjusted to 3 with 1M dilute hydrochloric acid, and the product was extracted with EA. Most of the solvent was removed under reduced pressure, and the resulting material was diluted with water and lyophilized to obtain **Compound 66** as a white solid (45.76 mg, yield: 43.6%).

LCMS m/z (ESI) = 322.1 [M+1].

¹H NMR (400 MHz, DMSO-*d*₆) δ 13.32 (s, 1H), 7.77 (td, 1H), 7.59 (d, 1H), 7.40 (d, J = 8.8 Hz, 1H), 7.27 - 7.16 (m, 3H), 6.62 (d, 1H), 5.57 (s, 2H).

¹⁹F NMR (377 MHz, DMSO-*d*₆) δ -115.37, -125.12.

### Example 67

### 4-((5,7-Dichloro-1H-indol-1-yl)methyl)-3-fluorothiophene-2-carboxylic acid (Compound 67)

### 4-((5,7-Dichloro-1H-indol-1-yl)methyl)-3-fluorothiophene-2-carboxylic acid

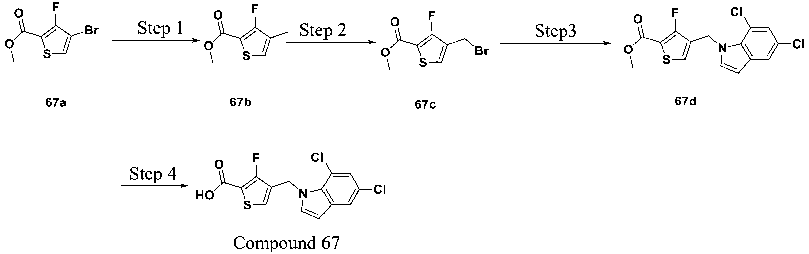

### Step 1:

### Methyl 3-fluoro-4-methylthiophene-2-carboxylate (67b)

### Methyl 3-fluoro-4-methylthiophene-2-carboxylate

In a 100 mL single-necked flask, **67a** (550 mg, 2.3 mmol) and methylboronic acid (206.58 mg, 3.45 mmol) were dissolved in 15 mL of 1,4-dioxane and 0.5 mL of water, and 1,1-bis(diphenylphosphine)palladium(II) dichloride (168.13 mg, 0.23 mmol) and cesium carbonate (1873.47 mg, 5.75 mmol) were added. After nitrogen displacement, the mixture was reacted at 100°C for 8 h. The reaction was complete as monitored by TLC. The reaction liquid was filtered, and the organic solvent was removed under reduced pressure. The crude product was purified by silica gel column chromatography (PE : EA = 10 : 1) to obtain **67b** as a white solid (140 mg, yield: 34.98%).

LCMS m/z (ESI) = 175.09 [M+1].

### Step 2:

### Methyl 4-(bromomethyl)-3-fluorothiophene-2-carboxylate (67c)

### Methyl 4-(bromomethyl)-3-fluorothiophene-2-carboxylate

In a 50 mL three-necked flask, **67b** (180 mg, 1.03 mmol) was dissolved in 1,2-dichloroethane (10 mL), and N-bromosuccinimide (202.53 mg, 1.14 mmol), and dibenzoyl peroxide (24.95 mg, 0.1 mmol) were added. The mixture was reacted at 80°C for 4 h. The reaction was complete as monitored by TLC. The organic solvent was removed under reduced pressure from the reaction liquid. The crude product was purified by silica gel column chromatography (PE : EA = 10 : 1) to obtain **67c** as a pink liquid (142 mg, yield: 55.47%).

LCMS m/z (ESI) = 252.97[M+1].

### Step 3:

### Methyl 4-((5,7-dichloro-1H-indol-1-yl)methyl)-3-fluorothiophene-2-carboxylate (67d)

### Methyl 4-((5,7-dichloro-1H-indol-1-yl)methyl)-3-fluorothiophene-2-carboxylate

In a 50 mL single-necked flask, **67c** (70 mg, 0.28 mmol) and 5,7-dichloroindole (51.46 mg, 0.28 mmol) were dissolved in 5 mL of acetonitrile, and cesium carbonate (228.08 mg, 0.7 mmol) was added. The mixture was reacted at 60°C for 5 h. The reaction was complete as monitored by TLC. The reaction liquid was filtered, and the organic solvent was removed under reduced pressure. The crude product was purified by silica gel column chromatography (PE : EA = 5 : 1) to obtain **67d** as a yellow solid (24 mg, yield: 23.91%).

LCMS m/z (ESI) = 357.99[M+1].

### Step 4:

### 4-((5,7-Dichloro-1H-indol-1-yl)methyl)-3-fluorothiophene-2-carboxylic acid (Compound 67)

### 4-((5,7-Dichloro-1H-indol-1-yl)methyl)-3-fluorothiophene-2-carboxylic acid

In a 50 mL three-necked flask, **67d** (24 mg, 0.07 mmol) was dissolved in a mixed solvent of ethanol (3 mL) and water (1 mL), and sodium hydroxide (6.69 mg, 0.17 mmol) was added. The reaction was carried out at 70°C for 1 h. The reaction was complete as monitored by LCMS. The reaction liquid was concentrated under reduced pressure, and the pH was adjusted to acidity with 6N dilute hydrochloric acid. A solid was precipitated, and after suction filtration, a filter cake was collected to obtain **Compound 67** as a white solid (15 mg, yield: 62.29%).

LC-MS m/z (ESI) = 343.97 [M+1].

¹H NMR (400 MHz, DMSO-*d*₆) δ 13.47 (s, 1H), 7.66 (d, 1H), 7.61 (d, 1H), 7.23 (d, 1H), 7.18 (d, 1H), 6.61 (d, 1H), 5.66 (s, 2H).

¹⁹F NMR (377 MHz, DMSO-*d*₆) δ -58.24.

### Example 68

### 2-Fluoro-3-((7-methyl-5-(trifluoromethyl)-1H-indol-1-yl)methyl)benzamide (Compound 68)

### 2-Fluoro-3-((7-methyl-5-(trifluoromethyl)- 1H-indol-1-yl)methyl)benzamide

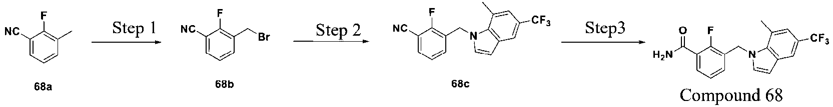

### Step 1:

### 3-(Bromomethyl)-2-fluorobenzonitrile (68b)

### 3-(Bromomethyl)-2-fluorobenzonitrile

In a 100 mL three-necked flask, **68a** (1 g, 7.4 mmol) was dissolved in 1,2-dichloroethane (30 mL), and N-bromosuccinimide (1.3 g, 7.4 mmol) and dibenzoyl peroxide (121 mg, 0.74 mmol) were added. The mixture was reacted at 80°C for 2 h. The reaction was complete as monitored by TLC. The organic solvent was removed under reduced pressure from the reaction liquid. The crude product was purified by silica gel column chromatography (PE : EA = 10 : 1) to obtain **68b** as a yellow oil (1.4 g, yield: 90%).

LCMS m/z (ESI) = 214.1 [M+1].

### Step 2:

### 2-Fluoro-3-((7-methyl-5-(trifluoromethyl)-1H-indol-1-yl)methyl)benzonitrile (68c)

### 2-Fluoro-3-((7-methyl-5-(trifluoromethyl)-1H-indol-1-yl)methyl)benzonitrile

In a 100 mL single-necked flask, **68b** (1.07 g, 5 mmol) and 7-methyl-5-(trifluoromethyl)-1H-indole (995 mg, 5 mmol) were dissolved in 2 mL of acetonitrile, and cesium carbonate (4.06 g, 12.5 mmol) was added. The reaction was carried out at 70°C for 1 h. The reaction was complete as monitored by TLC. The reaction liquid was filtered, the organic solvent was removed under reduced pressure. The crude product was purified by silica gel column chromatography (PE : EA = 10 : 1-4 : 1) to obtain **68c** as a white solid (998 mg, yield: 60.1%).

LCMS m/z (ESI) = 333.1 [M+1].

### Step 3:

### 2-Fluoro-3-((7-methyl-5-(trifluoromethyl)-1H-indol-1-yl)methyl)benzamide (Compound 68)

### 2-Fluoro-3-((7-methyl-5-(trifluoromethyl)-1H-indol-1-yl)methyl)benzamide

In a 50 mL single-necked flask, **68c** (100 mg, 0.3 mmol) was dissolved in DMSO (2 mL), and potassium carbonate (166 mg, 1.2 mmol) was added. After cooling to 0°C, 1 mL of 30% hydrogen peroxide was dropwise added, and the mixture was reacted at room temperature for 3 h. The reaction was complete as monitored by TLC. The reaction liquid was filtered, diluted with water, and extracted three times with ethyl acetate (20 mL). The organic phases were combined, most of the organic phase was removed under reduced pressure, and **Compound 68** as a white solid (50 mg, yield: 47.6%) was naturally precipitated.

LCMS m/z (ESI) = 351.1 [M+1].

¹H NMR (400 MHz, DMSO-*d*₆) δ 7.88 - 7.80 (m, 2H), 7.68 (s, 1H), 7.58 (d, *J* = 3.2 Hz, 1H), 7.52 (td, *J* = 7.4*,* 1.7 Hz, 1H), 7.17 - 7.13 (m, 1H), 7.10 (t, *J =* 7.7 Hz, 1H), 6.72 (d, *J =* 3.2 Hz, 1H), 6.34 (td, *J =* 7.4, 1.7 Hz, 1H), 5.78 (s, 2H), 2.52 (s, 3H).

### Example 69

### 3-(2-Fluoro-3-((7-methyl-5-(trifluoromethyl)-1H-indol-1-yl)methyl)phenyl)-1,2,4-oxadiazol-5(4H)-one (Compound 69)

### 3-(2-Fluoro-3-((7-methyl-5-(trifluoromethyl)-1H-indol-1-yl)methyl)phenyl)-1,2,4-oxadiazol-5(4H)-one

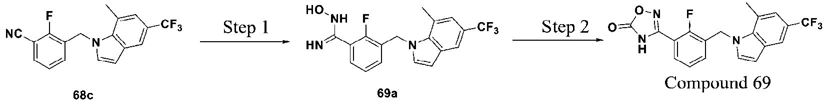

### Step 1:

### 2-Fluoro-N-hydroxy-3-((7-methyl-5-(trifluoromethyl)-1H-indol-1-yl)methyl)benzimidamide (69a)

### 2-Fluoro-N-hydroxy-3-((7-methyl-5-(trifluoromethyl)-1H-indol-1-yl)methyl)benzimidamide

In a 50 mL single-necked flask, **68c** (100 mg, 0.301 mmol) was dissolved in 3 mL of dimethyl sulfoxide, and hydroxylamine hydrochloride (25.1 mg, 0.361 mmol) and sodium carbonate (63.8 mg, 0.602 mmol) were added. The mixture was reacted at 80°C for 3 h. The reaction was complete as monitored by TLC. The reaction was quenched with water. A solid was precipitated, and the precipitated solid was filtered, washed with water, and dried under reduced pressure to obtain the desired **69a** as a white solid (58 mg, yield: 52.8%).

LCMS m/z (ESI) = 366.2[M+1].

### Step 2:

### 3-(2-Fluoro-3-((7-methyl-5-(trifluoromethyl)-1H-indol-1-yl)methyl)phenyl)-1,2,4-oxadiazol-5(4H)-one (Compound 69)

### 3-(2-Fluoro-3-((7-methyl-5-(trifluoromethyl)-1H-indol-1-yl)methyl)phenyl)-1,2,4-oxadiazol-5(4H)-one

In a 50 mL single-necked flask, **69a** (90 mg, 0.246 mmol) was dissolved in 3 mL of dichloromethane, and N,N'-carbonyldiimidazole (44 mg, 0.271 mmol) and 1,8-diazabicyclo[5,4,0]undec-7-ene (75 mg, 0.492 mmol) were added. The mixture was reacted at room temperature overnight. The reaction was complete as monitored by LCMS. and the solvent was removed under reduced pressure. The crude product was purified by medium pressure preparative reversed-phase chromatography (ACN/H₂O) and lyophilized to obtain **Compound 69** as a white solid (39 mg, yield: 40.6%).

LCMS m/z (ESI) = 392.1 [M+1].

¹H NMR (400 MHz, DMSO-*d*₆) δ 7.85 (d, 1H), 7.69 (td, 1H), 7.60 (d, 1H), 7.26 (t, 1H), 7.15 (d,1H), 6.74 (d, 1H), 6.51 (td, 1H), 2.51 (s, 3H).

### Example 70

### 3-(5,7-Dichloro-1H-indole-3-carbonyl)-2-fluorobenzoic acid (Compound 70)

### 3-(5,7-Dichloro-1H-indole-3-carbonyl)-2-fluorobenzoic acid

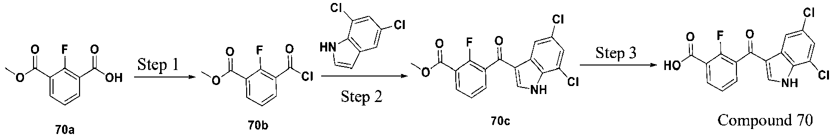

### Step 1:

### Methyl 3-(chlorocarbonyl)-2-fluorobenzoate (70b)

### Methyl 3-(chlorocarbonyl)-2-fluorobenzoate

In a 50 mL single-necked flask, at 0°C, thionyl chloride (5 mL) was slowly dropwise added to 2-fluoro-3-(methoxycarbonyl)benzoic acid **70a** (260 mg, 1.3 mmol), and after the addition was complete, the reaction system was transferred to 40°C and reacted for 3 h. After the reaction was complete as monitored, the reaction liquid was concentrated under reduced pressure and subjected to rotary evaporation to obtain the crude target product **70b** as a yellow solid (261 mg, yield: 94%).

LCMS m/z (ESI) = 213.0 [M+1].

### Step 2:

### Methyl 3-(5,7-dichloro-1H-indole-3-carbonyl)-2-fluorobenzoate (70c)

### Methyl 3-(5,7-dichloro-1H-indole-3-carbonyl)-2-fluorobenzoate

In a 50 mL single-necked flask, aluminum trichloride (243 mg, 1.825 mmol) was dissolved in dry DCE (5 mL), and **70b** (317 mg, 1.46 mmol) was slowly added to the above mixed solvent. 5,7-Dichloroindole (135.8 mg, 0.73 mmol) was then slowly added to the reaction system, and after the addition was complete, the mixture was reacted at 80°C for 3 h under inert gas protection. The reaction was complete as monitored by TLC. The reaction liquid was cooled to 0°C. The pH was adjusted to 4-5 with 1M dilute hydrochloric acid. The liquid was extracted with DCM. The organic phases were combined, dried over anhydrous Na₂SO₄, and filtered, and the solvent was removed under reduced pressure. The crude product was purified by preparative thin-layer chromatography (PE : EA = 5 : 1) to obtain **70c** an orange-red solid (366 mg, yield: 68%).

LCMS m/z (ESI) = 366.0[M+1].

### Step 3:

### 3-(5,7-Dichloro-1H-indole-3-carbonyl)-2-fluorobenzoic acid (Compound 70)

### 3-(5,7-Dichloro-1H-indole-3-carbonyl)-2-fluorobenzoic acid

In a 50 mL single-necked flask, **70c** (116 mg, 0.32 mmol) was dissolved in a mixed solvent of methanol (2 mL) and water (0.5 mL), and sodium hydroxide (64 mg, 1.6 mmol) was added. The mixture was reacted at 60°C for 3 h. The reaction was complete as monitored by LCMS. The solvent was removed under reduced pressure. The resulting material was diluted with water. The pH was adjusted to 3 with 1M dilute hydrochloric acid, and the liquid was extracted with ethyl acetate. The organic phases were combined, dried over anhydrous Na₂SO₄, filtered, and concentrated. The crude product was purified by reversed-phase preparative thin-layer chromatography (20% acetonitrile aqueous solution) to obtain **Compound 70** as a white solid (30 mg, yield: 27%).

LCMS m/z (ESI) = 352.0 [M+1].

¹H NMR (400 MHz, DMSO-*d*₆) δ 8.16 (d, 1H), 7.86 (td, 1.9 Hz, 1H), 7.82 (s, 1H), 7.57 - 7.50 (m, 2H), 7.28 (t, 1H).

### Example 71

### 5-((5,7-Dichloro-1H-indol-1-yl)methyl)-4-fluorothiophene-3-carboxylic acid (Compound 71)

### 5-((5,7-Dichloro-1H-indol-1-yl)methyl)-4-fluorothiophene-3-carboxylic acid

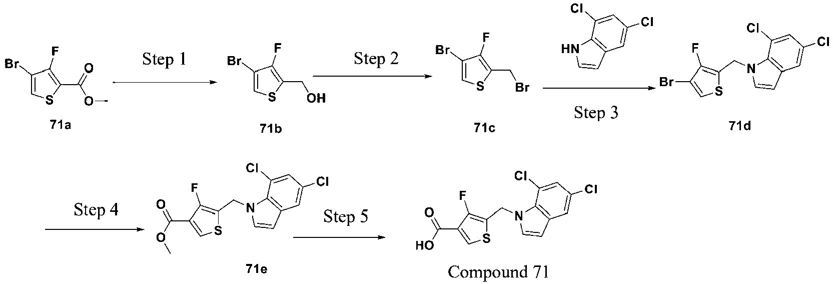

### Step 1:

### (4-Bromo-3-fluorothiophen-2-yl)methanol (71b)

### (4-Bromo-3-fluorothiophen-2-yl)methanol

In a 50 mL three-necked flask, **71a** (250 mg, 1.05 mmol) was dissolved in 3 mL of DCM, and the solution was cooled to 0°C. Under inert gas protection, DIBAl-H (1.5 M, 2.1 mL, 3.15 mmol) was slowly dropwise added to the reaction system. After the addition was complete, the mixture was naturally heated to room temperature and reacted for 3 h. The reaction was complete as monitored by TLC. The reaction was quenched with sodium sulfate decahydrate, and after stirring for 30 min, the reaction liquid was directly filtered to obtain a crude product, which was purified by column chromatography (PE : EA = 5 : 1) to obtain **71b** as a light yellow oily liquid (200 mg, yield: 91%).

¹H NMR (400 MHz, Chloroform-*d*) δ 7.16 (d, 1H), 4.78 (dd, 2H).

### Step 2:

### 4-Bromo-2-(bromomethyl)-3-fluorothiophene (71c)

### 4-Bromo-2-(bromomethyl)-3-fluorothiophene

In a 50 mL single-necked flask, **71b** (105 mg, 0.5 mmol) and triphenylphosphine (157.4 mg, 0.6 mmol) were dissolved in 5 mL of DCM. Under inert gas protection at 0°C, CBr₄ (215.6 mg, 0.65 mmol) was added, and after the addition was complete, the mixture was naturally heated to room temperature and reacted for 2 h. The reaction was complete as monitored by TLC. The organic solvent was removed by concentration under reduced pressure to obtain the crude product 71c as a light yellow solid (137 mg), which was directly used in the subsequent reaction.

### Step 3:

### 1-((4-Bromo-3-fluorothiophen-2-yl)methyl)-5,7-dichloro-1H-indole (71d)

### 1-((4-Bromo-3-fluorothiophen-2-yl)methyl)-5,7-dichloro-1H-indole

In a 50 mL single-necked flask, **71c** (137 mg, 0.5 mmol), 5,7-dichloroindole (93 mg, 0.5 mmol), and cesium carbonate (407 mg, 1.25 mmol) were dissolved in acetonitrile (3 mL). The reaction was carried out at 70°C for 3 h. The reaction was complete as monitored by TLC. Excess cesium carbonate was removed by filtration through diatomite, and the organic phase was concentrated under reduced pressure to obtain a crude product. The crude product was purified by preparative thin-layer chromatography (PE : EA = 10 : 1) to obtain **71d** as a white solid (120 mg, yield: 63%).

### Step 4:

### Methyl 5-((5,7-dichloro-1H-indol-1-yl)methyl)-4-fluorothiophene-3-carboxylate (71e)

### Methyl 5-((5,7-dichloro-1H-indol-1-yl)methyl)-4-fluorothiophene-3-carboxylate

In a 50 mL single-necked flask, **71d** (120 mg, 0.32 mmol), anhydrous MeOH (153 mg, 4.8 mmol), Na₂CO₃ (106 mg, 0.64 mmol), Pd (OAc)₂ (7 mg, 0.03 mmol), and XantPhos (29 mg, 0.05 mmol) were dissolved in toluene (3 mL). The mixture was heated to 80°C under CO aeration and reacted for 16 h. The reaction was complete as monitored by TLC. Na₂CO₃ and Pd(OAc)₂ were removed by filtration through diatomite, followed by washing with EA. The organic solvent was removed by concentration under reduced pressure to obtain the crude product. The crude product was purified by a chromatographic column (PE : EA = 10 : 1) to obtain **71e** as a white solid (44 mg, yield: 39%).

### Step 5:

### 5-((5,7-Dichloro-1H-indol-1-yl)methyl)-4-fluorothiophene-3-carboxylic acid (Compound 71)

### 5-((5,7-Dichloro-1H-indol-1-yl)methyl)-4-fluorothiophene-3-carboxylic acid

In a 50 mL single-necked flask, **71e** (44 mg, 0.13 mmol) was dissolved in a mixed solvent of methanol (2 mL) and water (0.5 mL), and sodium hydroxide (26 mg, 0.26 mmol) was added. The mixture was reacted at 60°C for 3 h. The reaction was complete as monitored by LCMS. and the solvent was removed under reduced pressure. The resulting material was diluted with water. The pH was adjusted to 3 with 1M dilute hydrochloric acid, and the liquid was extracted with ethyl acetate. The organic phases were combined, dried over anhydrous Na₂SO₄, filtered, and concentrated. The crude product was purified by reversed-phase preparative thin-layer chromatography (20% acetonitrile aqueous solution) to obtain **Compound 71** as a white solid (21 mg, yield: 48%).

¹H NMR (400 MHz, DMSO-*d*₆) δ 13.06 (s, 1H), 8.11 (d, 1H), 7.64 (dd, 2H), 7.27 (d, 1H), 6.61 (d, 1H), 5.87 (s, 2H).

### Examples 72-75

The corresponding compounds of Examples 72-76 are as shown in Table 2, and the corresponding compounds 72-75 are obtained with reference to the synthesis method of **Compound** 9 by using commercially available raw materials or intermediates in the preparation method provided by the present invention as raw materials.

**Table 2**

| Compound | Compound Name | Structure | NMR Date | LC-MS [M+1] |
|---|---|---|---|---|
| 72 | 3-((7-Ethyl-5-(trifluoromethyl)-1H-indol-1-yl)methyl)-2-fluorobenzoic acid | 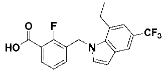 | 1H NMR (400 MHz, DMSO-d6) δ 13.38 (s, 1H), 8.09 (d, 1H), 7.86 (d, 1H), 7.59 (d, 2H), 7.17 (d, 1H), 6.85 (d, 1H), 6.75 (d, 1H), 5.97 (s, 2H), 5.75 (s, 2H), 1.15 (t, 3H). | 366.2 |
| | 3-((7-Ethyl-5-(trifluoromethyl)-1H-indol-1-yl)methyl)-2-fluorobenzoic acid | | | |
| 73 | 3-((7-(2-Cyanoethyl)-5-(trifluoromethyl)-1H-indol-1-yl)methyl)-2-fluorobenzoic acid | 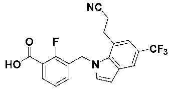 | 1H NMR (400 MHz, DMSO-d6) δ 13.36 (s, 1H), 7.95 (t, 1H), 7.78 (td, 1H), 7.61 (d, 1H), 7.30 (d, 1H), 7.15 (t, 1H), 6.78 (d, 1H), 6.49 (t, 1H), 5.76 (s, 2H), 3.13 (t, 2H), 2.84 (t, 2H). | 391.2 |
| | 3-((7-(2-Cyanoethyl)-5-(trifluoromethyl)-1H-indol-1-yl)methyl)-2-fluorobenzoic acid | | | |
| | | | 19F NMR (377 MHz, DMSO-d6) δ -58.80 (d, J = 18.5 Hz), - 116.12. | |
| 74 | 3-((4-Chloro-5-cyano-1H-indol-1-yl)methyl)-2-fluorobenzoic acid | 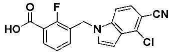 | 1H NMR (400 MHz, DMSO-d6) δ 13.37 (s, 1H), 7.81 - 7.68 (m, 3H), 7.59 (d, 1H), 7.31 - 7.24 (m, 1H), 7.20 (t, 1H), 6.71 (d, 1H), 5.62 (s, 2H). | 329.1 |
| | 3-((4-Chloro-5-cyano-1H-indol-1-yl)methyl)-2-fluorobenzoic acid | | | |
| | | | 19F NMR (377 MHz, DMSO-d6) δ -115.16. | |
| 75 | 3-((4-Chloro-5-cyclopropyl-1H-indol-1-yl)methyl)-2-fluorobenzoic acid | 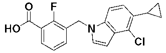 | 1H NMR (400 MHz, DMSO-d6) δ 7.57 (t, 1H), 7.50 (d, 1H), 7.35 (d, 1H), 6.97 (d, 2H), 6.76 (d, 1H), 6.46 (d, 1H), 5.43 (s, 2H), 2.19 (td, 1H), 1.00 - 0.90 (m, 2H), 0.69 - 0.58 (m, 2H). | 344.1 |
| | 3-((4-Chloro-5-cyclopropyl-1H-indol-1-yl)methyl)-2-fluorobenzoic acid | | | |
| | | | 19F NMR (377 MHz, DMSO-d6) δ -118.80. | |

### Example 76

### 2-((5,7-Dichloro-1H-indol-1-yl)methyl)thiazole-4-carboxylic acid (Compound 76)

### 2-((5,7-Dichloro-1H-indol-1-yl)methyl)thiazole-4-carboxylic acid

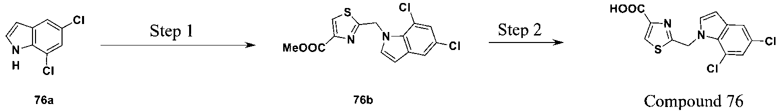

### Step 1:

### Methyl 2-((5,7-dichloro-1H-indol-1-yl)methyl)thiazole-4-carboxylate (76b)

### Methyl 2-((5,7-dichloro-1H-indol-1-yl)methyl)thiazole-4-carboxylate

In a 50 mL single-necked flask, **76a** (56 mg, 0.30 mmol) and methyl 2-chloromethylthiazole-4-carboxylate (57 mg, 0.20 mmol) were dissolved in 5 mL of acetonitrile, and cesium carbonate (132 mg, 0.40 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **76b** as a white solid (63 mg, yield: 62%).

LCMS m/z (ESI) = 341.0 [M+1].

### Step 2:

### 2-((5,7-Dichloro-1H-indol-1-yl)methyl)thiazole-4-carboxylic acid (Compound 76)

### 2-((5,7-Dichloro-1H-indol-1-yl)methyl)thiazole-4-carboxylic acid

In a 50 mL single-necked flask, **76b** (63 mg, 0.19 mmol) was dissolved in a mixed solvent of methanol (1 mL) and water (1 mL), and sodium hydroxide (22 mg, 0.57 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 76** as a white solid (51 mg, yield: 82%).

¹H NMR (400 MHz, DMSO-*d*₆) δ 8.32 (s, 1H), 7.71 (dd, 2.6 Hz, 2H), 7.27 (d, 1H), 6.67 (d, 1H), 6.08 (s, 2H).

### Example 77

### 3-((4,6-Dichloro-2-methyl-1H-indol-1-yl)methyl)-2-fluorobenzoic acid (Compound 77)

### 3-((4,6-Dichloro-2-methyl-1H-indol-1-yl)methyl)-2-fluorobenzoic acid

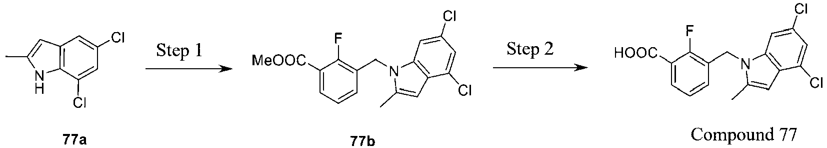

### Step 1:

### Methyl 3-((4,6-dichloro-2-methyl-1H-indol-1-yl)methyl)-2-fluorobenzoate (77b)

### Methyl 3-((4,6-dichloro-2-methyl-1H-indol-1-yl)methyl)-2-fluorobenzoate

In a 50 mL single-necked flask, **77a** (80 mg, 0.40 mmol) and methyl 3-(bromomethyl)-2-fluorobenzoate (98 mg, 0.40 mmol) were dissolved in 5 mL of acetonitrile, and cesium carbonate (326 mg, 1.00 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **77b** as a white solid (30 mg, yield: 20%).

LCMS m/z (ESI) = 366.0 [M+1].

### Step 2:

### 3-((4,6-Dichloro-2-methyl-1H-indol-1-yl)methyl)-2-fluorobenzoic acid (Compound 77)

### 3-((4,6-Dichloro-2-methyl-1H-indol-1-yl)methyl)-2-fluorobenzoic acid

In a 50 mL single-necked flask, **77b** (30 mg, 0.09 mmol) was dissolved in a mixed solvent of methanol (1 mL) and water (1 mL), and sodium hydroxide (10 mg, 0.27 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 77** as a white solid (26 mg, yield: 81%).

¹H NMR (400 MHz, DMSO-*d*₆) δ 7.75 (td, 1H), 7.64 (t, 1H), 7.20 - 7.14 (m, 2H), 6.73 - 6.66 (m, 1H), 6.42 (t, 1H), 2.34 (d, 3H).

¹⁹F NMR (376 MHz, DMSO-*d*₆) δ -115.24.

### Example 78

### 3-((4-Chloro-2,3-dimethyl-1H-indol-1-yl)methyl)-2-fluorobenzoic acid (Compound 78)

### 3-((4-Chloro-2,3-dimethyl-1H-indol-1-yl)methyl)-2-fluorobenzoic acid

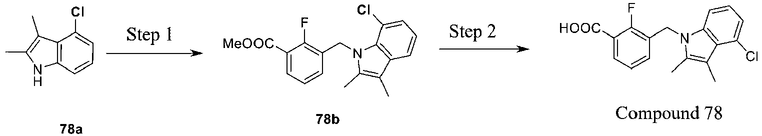

### Step 1:

### Methyl 3-((4-chloro-2,3-dimethyl-1H-indol-1-yl)methyl)-2-fluorobenzoate (78b)

### Methyl 3-((7-chloro-2,3-dimethyl-1H-indol-1-yl)methyl)-2-fluorobenzoate

In a 50 mL single-necked flask, **78a** (72 mg, 0.40 mmol) and methyl 3-(bromomethyl)-2-fluorobenzoate (98 mg, 0.240 mmol) were dissolved in 5 mL of acetonitrile, and cesium carbonate (326 mg, 1.00 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **78b** as a white solid (67 mg, yield: 49%).

LCMS m/z (ESI) = 346.0 [M+1].

### Step 2:

### 3-((4-Chloro-2,3-dimethyl-1H-indol-1-yl)methyl)-2-fluorobenzoic acid (Compound 78)

### 3-((4-Chloro-2,3-dimethyl-1H-indol-1-yl)methyl)-2-fluorobenzoic acid

In a 50 mL single-necked flask, **78b** (55 mg, 0.16 mmol) was dissolved in a mixed solvent of methanol (1 mL) and water (1 mL), and sodium hydroxide (19 mg, 0.48 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 78** as a white solid (46 mg, yield: 87%).

¹H NMR (400 MHz, DMSO-*d*₆) δ 7.72 (td, 1H), 7.49 (d, 1H), 7.39 (d, 1H), 7.13 (t, 1H), 7.03 (dd, 1H), 6.64 (td, 1H), 5.49 (s, 2H), 2.26 (s, 3H), 2.20 (s, 3H).

¹⁹F NMR (376 MHz, DMSO-*d*₆) δ -115.67.

### Example 79

### 3-((4,6-Dichloro-1H-indol-1-yl)methyl)-2-fluorobenzoic acid (Compound 79)

### 3-((4,6-Dichloro-1H-indol-1-yl)methyl)-2-fluorobenzoic acid

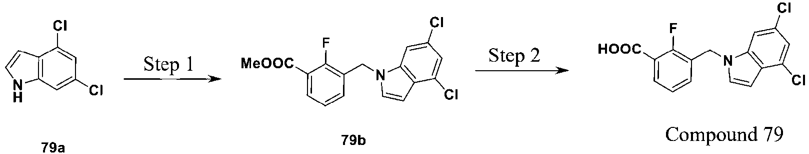

### Step 1:

### Methyl 3-((4,6-dichloro-1H-indol-1-yl)methyl)-2-fluorobenzoate (79b)

### Methyl 3-((4,6-dichloro-1H-indol-1-yl)methyl)-2-fluorobenzoate

In a 50 mL single-necked flask, **79a** (74 mg, 0.40 mmol) and methyl 3-(bromomethyl)-2-fluorobenzoate (98 mg, 0.40 mmol) were dissolved in 5 mL of acetonitrile, and cesium carbonate (326 mg, 1.00 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **79b** as a white solid (133 mg, yield: 92%).

LCMS m/z (ESI) = 352.0 [M+1].

### Step 2:

### 3-((4,6-Dichloro-1H-indol-1-yl)methyl)-2-fluorobenzoic acid (Compound 79)

### 3-((4,6-Dichloro-1H-indol-1-yl)methyl)-2-fluorobenzoic acid

In a 50 mL single-necked flask, **79b** (117 mg, 0.33 mmol) was dissolved in a mixed solvent of methanol (1 mL) and water (1 mL), and sodium hydroxide (40 mg, 1.00 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 79** as a white solid (89 mg, yield: 80%).

¹H NMR (400 MHz, DMSO-*d*₆) δ 7.78 (dq, 1H), 7.74 - 7.71 (m, 1H), 7.61 (d, 1H), 7.30 - 7.18 (m, 3H), 6.58 (d, 1H), 5.58 (s, 2H).

¹⁹F NMR (376 MHz, DMSO-*d*₆) δ -115.16.

### Example 80

### 3-((4,7-Dichloro-1H-indol-1-yl)methyl)-2-fluorobenzoic acid (Compound 80)

### 3-((4,7-Dichloro-1H-indol-1-yl)methyl)-2-fluorobenzoic acid

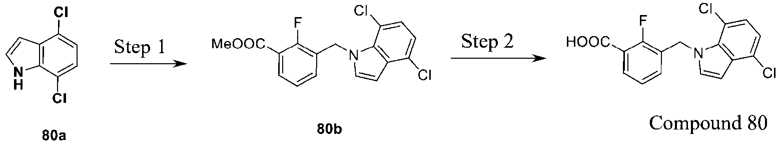

### Step 1:

### Methyl 3-((4,7-dichloro-1H-indol-1-yl)methyl)-2-fluorobenzoate (80b)

### Methyl 3-((4,7-dichloro-1H-indol-1-yl)methyl)-2-fluorobenzoate

In a 50 mL single-necked flask, **80a** (74 mg, 0.40 mmol) and methyl 3-(bromomethyl)-2-fluorobenzoate (98 mg, 0.40 mmol) were dissolved in 5 mL of acetonitrile, and cesium carbonate (326 mg, 1.00 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **80b** as a white solid (110 mg, yield: 86%).

LCMS m/z (ESI) = 352.0 [M+1].

### Step 2:

### 3-((4,7-Dichloro-1H-indol-1-yl)methyl)-2-fluorobenzoic acid (Compound 80)

### 3-((4,7-Dichloro-1H-indol-1-yl)methyl)-2-fluorobenzoic acid

In a 50 mL single-necked flask, **80b** (110 mg, 0.31 mmol) was dissolved in a mixed solvent of methanol (1 mL) and water (1 mL), and sodium hydroxide (38 mg, 0.93 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 80** as a white solid (96 mg, yield: 91%).

¹H NMR (400 MHz, DMSO-*d*₆) δ 7.77 (td, 1H), 7.71 (d, 1H), 7.17 (s, 3H), 6.72 (d, 1H), 6.61 (td, 1H), 5.92 (s, 2H).

¹⁹F NMR (376 MHz, DMSO-*d*₆) δ -116.62.

### Example 81

### 3-((4,5-Dichloro-1H-indol-1-yl)methyl)-2-fluorobenzoic acid (Compound 81)

### 3-((4,5-Dichloro-1H-indol-1-yl)methyl)-2-fluorobenzoic acid

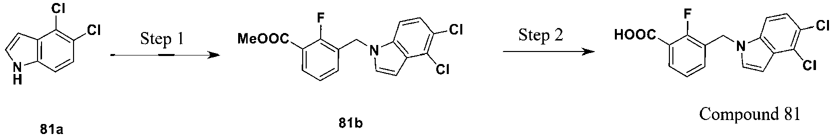

### Step 1:

### Methyl 3-((4,5-dichloro-1H-indol-1-yl)methyl)-2-fluorobenzoate (81b)

### Methyl 3-((4,5-dichloro-1H-indol-1-yl)methyl)-2-fluorobenzoate

In a 50 mL single-necked flask, **81a** (74 mg, 0.40 mmol) and methyl 3-(bromomethyl)-2-fluorobenzoate (98 mg, 0.40 mmol) were dissolved in 5 mL of acetonitrile, and cesium carbonate (326 mg, 1.00 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **81b** as a white solid (72 mg, yield: 51%).

LCMS m/z (ESI) = 352.0 [M+1].

### Step 2:

### 3-((4,5-Dichloro-1H-indol-1-yl)methyl)-2-fluorobenzoic acid (Compound 81)

### 3-((4,5-Dichloro-1H-indol-1-yl)methyl)-2-fluorobenzoic acid

In a 50 mL single-necked flask, **81b** (80 mg, 0.23 mmol) was dissolved in a mixed solvent of methanol (1 mL) and water (1 mL), and sodium hydroxide (27 mg, 0.68 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 81** as a white solid (72 mg, yield: 93%).

¹H NMR (400 MHz, DMSO-*d*₆) δ 7.83 - 7.72 (m, 1H), 7.66 (d, 1H), 7.56 (d,1H), 7.32 (d, 1H), 7.27 - 7.16 (m, 2H), 6.59 (d, 1H), 5.59 (s, 2H).

¹⁹F NMR (376 MHz, DMSO-*d*₆) δ -115.54.

### Example 82

### 2-Chloro-3-((5,7-dichloro-1H-indol-1-yl)methyl)benzoic acid (Compound 82)

### 2-Chloro-3-((5,7-dichloro-1H-indol-1-yl)methyl)benzoic acid

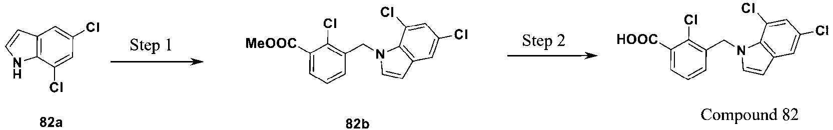

### Step 1:

### Methyl 2-chloro-3-((5,7-dichloro-1H-indol-1-yl)methyl)benzoate (82b)

### Methyl 2-chloro-3-((5,7-dichloro-1 H-indol-1-yl)methyl)benzoate

In a 50 mL single-necked flask, **82a** (56 mg, 0.30 mmol) and methyl 3-(bromomethyl)-2-chlorobenzoate (79 mg, 0.30 mmol) were dissolved in 5 mL of acetonitrile, and cesium carbonate (244 mg, 0.75 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **82b** as a white solid (105 mg, yield: 90%).

LCMS m/z (ESI) = 357.0 [M+1].

### Step 2:

### 2-Chloro-3-((5,7-dichloro-1H-indol-1-yl)methyl)benzoic acid (Compound 82)

### 2-Chloro-3-((5,7-dichloro-1H-indol-1-yl)methyl)benzoic acid

In a 50 mL single-necked flask, **82b** (105 mg, 0.3 mmol) was dissolved in a mixed solvent of methanol (1 mL) and water (1 mL), and sodium hydroxide (36 mg, 0.90 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 82** as a white solid (27 mg, yield: 27%).

¹H NMR (400 MHz, DMSO-*d*₆) δ 7.71 (d, 1H), 7.66 (d, 1H), 7.60 (dd, 1H), 7.25 (t, 1H), 7.21 (d, 1H), 6.69 (d, 1H), 6.17 (dd, 1H), 5.86 (s, 2H).

### Example 83

### 2-Chloro-3-((7-chloro-1H-indol-1-yl)methyl)benzoic acid (Compound 83)

### 2-Chloro-3-((7-chloro-1H-indol-1-yl)methyl)benzoic acid

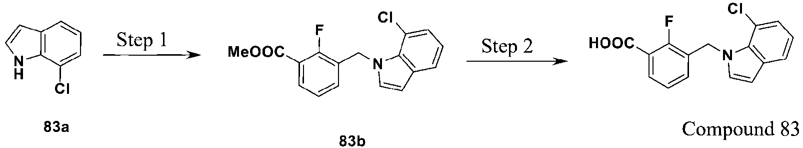

### Step 1:

### Methyl 2-chloro-3-((7-chloro-1H-indol-1-yl)methyl)benzoate (83b)

### Methyl 2-chloro-3-((7-chloro-1H-indol-1-yl)methyl)benzoate

In a 50 mL single-necked flask, **83a** (100 mg, 0.66 mmol) and methyl 3-(bromomethyl)-2-fluorobenzoate (162 mg, 0.66 mmol) were dissolved in 5 mL of acetonitrile, and cesium carbonate (537 mg, 1.65 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **83b** as a white solid (145 mg, yield: 66%).

LCMS m/z (ESI) = 334.0 [M+1].

### Step 2:

### 2-Chloro-3-((7-chloro-1H-indol-1-yl)methyl)benzoic acid (Compound 83)

### 2-Chloro-3-((7-chloro-1H-indol-1-yl)methyl)benzoic acid

In a 50 mL single-necked flask, **83b** (145 mg, 0.46 mmol) was dissolved in a mixed solvent of methanol (1 mL) and water (1 mL), and sodium hydroxide (55 mg, 1.38 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 83** as a white solid (121 mg, yield: 86%).

¹H NMR (400 MHz, DMSO-*d*₆) δ 7.74 (td, 1H), 7.64 - 7.49 (m, 2H), 7.11 (ddd, 2H), 7.02 (t, 1H), 6.65 (d, 1H), 6.55 (td, 1H), 5.87 (s, 2H).

¹⁹F NMR (376 MHz, DMSO-*d*₆) δ -116.64.

### Example 84

### 3-((7-Chloro-5-fluoro-1H-indol-1-yl)methyl)-2-fluorobenzoic acid (Compound 84)

### 3-((7-Chloro-5-fluoro-1H-indol-1-yl)methyl)-2-fluorobenzoic acid

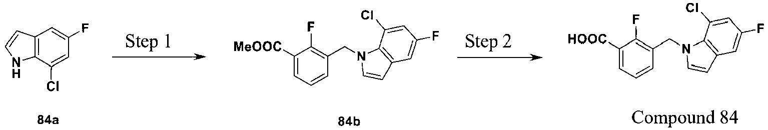

### Step 1:

### Methyl 3-((7-chloro-5-fluoro-1H-indol-1-yl)methyl)-2-fluorobenzoate (84b)

### Methyl 3-((7-chloro-5-fluoro-1H-indol-1-yl)methyl)-2-fluorobenzoate

In a 50 mL single-necked flask, **84a** (100 mg, 0.59 mmol) and methyl 3-(bromomethyl)-2-fluorobenzoate (145 mg, 0.59 mmol) were dissolved in 5 mL of acetonitrile, and cesium carbonate (480 mg, 1.48 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **84b** as a white solid (145 mg, yield: 74%).

LCMS m/z (ESI) = 322.0 [M+1].

### Step 2:

### 3-((7-Chloro-5-fluoro-1H-indol-1-yl)methyl)-2-fluorobenzoic acid (Compound 84)

### 3-((7-Chloro-5-fluoro-1H-indol-1-yl)methyl)-2-fluorobenzoic acid

In a 50 mL single-necked flask, **84b** (145 mg, 0.44 mmol) was dissolved in a mixed solvent of methanol (1 mL) and water (1 mL), and sodium hydroxide (52 mg, 1.30 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 84** as a white solid (135 mg, yield: 95%).

¹H NMR (400 MHz, DMSO-*d*₆) δ 7.74 (td, 1H), 7.63 (d, 1H), 7.42 (dd, 1H), 7.25 - 7.02 (m, 2H), 6.65 (d, 1H), 6.61 - 6.48 (m, 1H), 5.84 (s, 2H).

¹⁹F NMR (376 MHz, DMSO-*d*₆) δ -116.68, -123.13.

### Example 85

### 2-Fluoro-3-((5-fluoro-7-methyl-1H-indol-1-yl)methyl)benzoic acid (Compound 85)

### 2-Fluoro-3-((5-fluoro-7-methyl-1H-indol-1-yl)methyl)benzoic acid

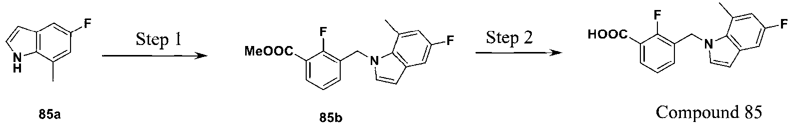

### Step 1:

### Methyl 2-fluoro-3-((5-fluoro-7-methyl-1H-indol-1-yl)methyl)benzoate (85b)

### Methyl 2-fluoro-3-((5-fluoro-7-methyl-1H-indol-1-yl)methyl)benzoate

In a 50 mL single-necked flask, **85a** (90 mg, 0.60 mmol) and methyl 3-(bromomethyl)-2-fluorobenzoate (148 mg, 0.60 mmol) were dissolved in 5 mL of acetonitrile, and cesium carbonate (489 mg, 1.50 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **85b** as a white solid (85 mg, yield: 45%).

LCMS m/z (ESI) = 316.0 [M+1].

### Step 2:

### 2-Fluoro-3-((5-fluoro-7-methyl-1H-indol-1-yl)methyl)benzoic acid (Compound 85)

### 2-Fluoro-3-((5-fluoro-7-methyl-1H-indol-1-yl)methyl)benzoic acid

In a 50 mL single-necked flask, **85b** (85 mg, 0.27 mmol) was dissolved in a mixed solvent of methanol (1 mL) and water (1 mL), and sodium hydroxide (32 mg, 0.81 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 85** as a white solid (61 mg, yield: 75%).

¹H NMR (400 MHz, DMSO-*d*₆) δ 7.75 (td, 1H), 7.47 (d, 1H), 7.19 (dd, 1H), 7.12 (t, 1H), 6.71 (dd, 1H), 6.52 (d, 1H), 6.42 (td, 1H), 5.72 (s, 2H), 2.40 (s, 3H).

¹⁹F NMR (376 MHz, DMSO-*d*₆) δ -116.58, -125.27.

### Example 86

### 3-((7-Bromo-5-(trifluoromethoxy)-1H-indol-1-yl)methyl)-2-fluorobenzoic acid (Compound 86)

### 3-((7-Bromo-5-(trifluoromethoxy)-1H-indol-1-yl)methyl)-2-fluorobenzoic acid

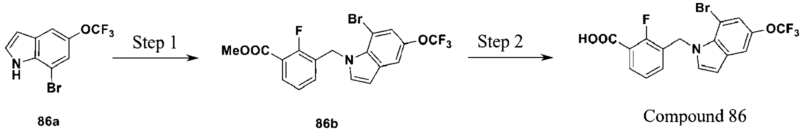

### Step 1:

### Methyl 3-((7-bromo-5-(trifluoromethoxy)-1H-indol-1-yl)methyl)-2-fluorobenzoate (86b)

### Methyl 3-((7-bromo-5-(trifluoromethoxy)-1H-indol-1-yl)methyl)-2-fluorobenzoate

In a 50 mL single-necked flask, **86a** (154 mg, 0.55 mmol) and methyl 3-(bromomethyl)-2-fluorobenzoate (136 mg, 0.55 mmol) were dissolved in 5 mL of acetonitrile, and cesium carbonate (448 mg, 1.38 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **86b** as a white solid (175 mg, yield: 71%).

LCMS m/z (ESI) = 446.0 [M+1].

### Step 2:

### 3-((7-Bromo-5-(trifluoromethoxy)-1H-indol-1-yl)methyl)-2-fluorobenzoic acid (Compound 86)

### 3-((7-Bromo-5-(trifluoromethoxy)-1H-indol-1-yl)methyl)-2-fluorobenzoic acid

In a 50 mL single-necked flask, **86b** (65 mg, 0.15 mmol) was dissolved in a mixed solvent of methanol (1 mL) and water (1 mL), and sodium hydroxide (18 mg, 0.45 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 86** as a white solid (60 mg, yield: 92%).

¹H NMR (400 MHz, DMSO-*d*₆) δ 7.74 (td, 1H), 7.69 (d, 2H), 7.35 (d, 1H), 7.13 (t, 1H), 6.75 (d, 1H), 6.48 (td, 1H), 5.92 (s, 2H).

¹⁹F NMR (376 MHz, DMSO-*d*₆) δ -57.17, -116.55.

### Example 87

### 2-Fluoro-3-((7-methyl-5-(trifluoromethoxy)-1H-indol-1-yl)methyl)benzoic acid (Compound 87)

### 2-Fluoro-3-((7-methyl-5-(trifluoromethoxy)-1H-indol-1-yl)methyl)benzoic acid

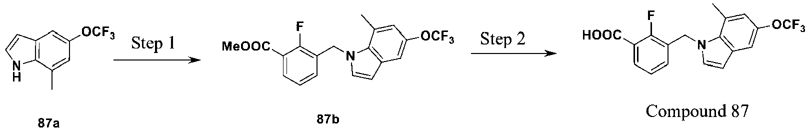

### Step 1:

### Methyl 2-fluoro-3-((7-methyl-5-(trifluoromethoxy)-1H-indol-1-yl)methyl)benzoate (87b)

### Methyl 2-fluoro-3-((7-methyl-5-(trifluoromethoxy)-1H-indol-1-yl)methyl)benzoate

In a 50 mL single-necked flask, **87a** (80 mg, 0.37 mmol) and methyl 3-(bromomethyl)-2-fluorobenzoate (81 mg, 0.37 mmol) were dissolved in 5 mL of acetonitrile, and cesium carbonate (304 mg, 0.93 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **87b** as a white solid (85 mg, yield: 53%).

LCMS m/z (ESI) = 432.0 [M+1].

### Step 2:

### 2-Fluoro-3-((7-methyl-5-(trifluoromethoxy)-1H-indol-1-yl)methyl)benzoic acid (Compound 87)

### 2-Fluoro-3-((7-methyl-5-(trifluoromethoxy)-1H-indol-1-yl)methyl)benzoic acid

In a 50 mL single-necked flask, **87b** (85 mg, 0.27 mmol) was dissolved in a mixed solvent of methanol (1 mL) and water (1 mL), and sodium hydroxide (32 mg, 0.81 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 87** as a white solid (61 mg, yield: 75%).

¹H NMR (400 MHz, DMSO-*d*₆) δ 7.75 (td, 1H), 7.53 (d, 1H), 7.42 (d, 1H), 7.13 (t, 1H), 6.84 (d, 1H), 6.61 (d, 1H), 6.55 - 6.41 (m, 1H), 5.76 (s, 2H), 2.45 (s, 3H).

¹⁹F NMR (376 MHz, DMSO-*d*₆) δ -56.82, -116.69.

### Example 88

### 3-((5-Chloro-3-cyano-1H-indol-1-yl)methyl)-2-fluorobenzoic acid (Compound 88)

### 3-((5-Chloro-3-cyano-1H-indol-1-yl)methyl)-2-fluorobenzoic acid

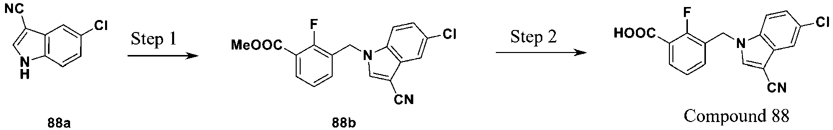

### Step 1:

### Methyl 3-((5-chloro-3-cyano-1H-indol-1-yl)methyl)-2-fluorobenzoate (88b)

### Methyl 3-((5-chloro-3-cyano-1H-indol-1-yl)methyl)-2-fluorobenzoate

In a 50 mL single-necked flask, **88a** (53 mg, 0.30 mmol) and methyl 3-(bromomethyl)-2-fluorobenzoate (74 mg, 0.30 mmol) were dissolved in 5 mL of acetonitrile, and cesium carbonate (244 mg, 0.75 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **88b** as a white solid (89 mg, yield: 86%).

LCMS m/z (ESI) = 343.0 [M+1].

### Step 2:

### 3-((5-Chloro-3-cyano-1H-indol-1-yl)methyl)-2-fluorobenzoic acid (Compound 88)

### 3-((5-Chloro-3-cyano-1H-indol-1-yl)methyl)-2-fluorobenzoic acid

In a 50 mL single-necked flask, **88b** (89 mg, 0.26 mmol) was dissolved in a mixed solvent of methanol (1 mL) and water (1 mL), and sodium hydroxide (31 mg, 0.78 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 88** as a white solid (71 mg, yield: 84%).

¹H NMR (400 MHz, DMSO-*d*₆) δ 8.47 (s, 1H), 7.70 (dd, 2H), 7.65 (td, 1H), 7.36 (dd, 1H), 7.22 (td, 1H), 7.11 (t, 1H), 5.59 (s, 2H).

¹⁹F NMR (376 MHz, DMSO-*d*₆) δ -117.86.

### Example 89

### 3-((5,7-Dichloro-3-methyl-1H-indol-1-yl)methyl)-2-fluorobenzoic acid (Compound 89)

### 3-((5,7-Dichloro-3-methyl-1H-indol-1-yl)methyl)-2-fluorobenzoic acid

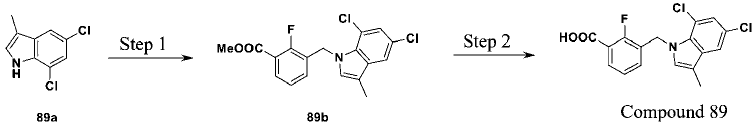

### Step 1:

### Methyl 3-((5,7-dichloro-3-methyl-1H-indol-1-yl)methyl)-2-fluorobenzoate (89)

### Methyl 3-((5,7-dichloro-3-methyl-1H-indol-1-yl)methyl)-2-fluorobenzoate

In a 50 mL single-necked flask, **89a** (50 mg, 0.25 mmol) and methyl 3-(bromomethyl)-2-fluorobenzoate (62 mg, 0.25 mmol) were dissolved in 5 mL of acetonitrile, and cesium carbonate (204 mg, 0.63 mmol) was added. The mixture was reacted at 60°C for 5 h. The reaction was complete as monitored by TLC. The reaction liquid was filtered, the organic solvent was removed under reduced pressure. The crude product was purified by silica gel column chromatography (PE : EA = 1 : 0-10 : 1) to obtain **89b** as a white solid (50 mg, yield: 55%).

LCMS m/z (ESI) = 366.0 [M+1].

### Step 2:

### 3-((5,7-Dichloro-3-methyl-1H-indol-1-yl)methyl)-2-fluorobenzoic acid (Compound 89)

### 3-((5,7-Dichloro-3-methyl-1H-indol-1-yl)methyl)-2-fluorobenzoic acid

In a 50 mL single-necked flask, **89b** (50 mg, 0.14 mmol) was dissolved in a mixed solvent of methanol (1 mL) and water (1 mL), and sodium hydroxide (16 mg, 0.41 mmol) was added. The mixture was reacted at 80°C for 1 h. The reaction was complete as monitored by TLC. The pH was adjusted to 3 with 1M dilute hydrochloric acid, and the solvent was removed under reduced pressure. The crude product was purified by medium pressure preparative reversed-phase chromatography (ACN/H₂O (TFA)) and lyophilized to obtain **Compound 89** as a white solid (40 mg, yield: 82%).

¹H NMR (400 MHz, DMSO-*d*₆) δ 13.35 (s, 1H), 7.75 (td, 1H), 7.61 (d, 1H), 7.39 (d, 1H), 7.20 (d, 1H), 7.15 (t, 1H), 6.61 (td, 1H), 5.79 (s, 2H), 2.26 (d,3H).

¹⁹F NMR (376 MHz, DMSO-*d*₆) δ -116.63.

### Biological test examples

### 1. Detection of MRGPRX4 activity

HEK-293 cells stably expressing MRGPRX4 were cultured in a DMEM medium containing 10% of FBS and 1% of a double antibody under the culture conditions of 37°C and 5% CO₂. Cells in the exponential growth phase were collected and inoculated into a 384-well plate at an inoculation density of 2 × 10⁴ cells/well, with 10 µL per well. 2 µL/well of a gradient-diluted compound was added and co-incubated with the cells for 45 min. After the incubation was complete, 2 µL/well of deoxycholic acid (MCE, Cat: #HY-N0593) was added to continue the incubation for 45 min. After the incubation was complete, IP-one Gq kit (PE, Cat: #62IPAPEB) was used to detect the content of IP1 according to the instructions. HTRF values were substituted into GraphPad 8.0, and IC₅₀ was calculated by fitting. The results are as shown in Table 3.

**Table 3 Test results of compounds of the present invention on MRGPRX4 activity**

| Compound | MRGPRX4 (IC₅₀) | Compound | MRGPRX4 (IC₅₀) |
|---|---|---|---|
| 5 | A | 48 | B |
| 6 | A | 51 | A |
| 15 | A | 62 | A |
| 16 | A | 66 | A |
| 22 | B | 74 | A |
| 27 | A | 79 | A |
| 28 | A | 80 | C |
| 30 | A | 81 | A |
| 36 | A | 82 | B |
| 39 | A | 87 | A |
| 41 | C | / | / |

| | | | |
|---|---|---|---|
| A: IC₅₀ ≤ 200 nM, B: 200 < IC₅₀ ≤ 500 nM, C: 500 < IC₅₀ ≤ 1 µM, D: IC₅₀ > 1 µM. Conclusions: The above compounds have good antagonistic effect on MRGPRX4. | | | |

### 2. Study of pharmacokinetics in mice in vivo

An appropriate amount of the test substance was weighed, and the test substance was prepared into a 2 mg/mL transparent clear solution using 5% DMSO + 5% HS-15 (Solutol HS-15) + 90% D5W (5% glucose) for intravenous administration. The test substance was prepared into a 5 mg/mL uniform suspension using 5% DMSO + 5% HS-15 + 90% PBS for intragastric administration.

18 healthy male C57BL/6 mice (GemPharmatech Co., Ltd., SPF grade) were taken. The intravenous administration group and the intragastric administration group respectively had 9 animals. At each time point, blood samples were taken from 3 animals via an orbital microsampling method, with a sampling volume of approximately 0.04 mL per animal per time point.

For intravenous administration of the test substance (10 mg/kg), blood samples were taken before administration and at 5 min, 15 min, 0.5 h, 1 h, 2 h, 4 h, 8 h, and 24 h after administration, and for intragastric administration of the test substance (50 mg/kg), blood samples were taken before administration and at 15 min, 0.5 h, 1 h, 2 h, 4 h, 8 h, and 24 h after administration. After anticoagulation with EDTA-K2, the blood samples were centrifuged at 6000 g at 4°C for 5 min to separate plasma, and all plasma samples were stored at -70°C for testing. The drug concentration of the test substance in plasma was determined by LC-MS/MS method (with tolbutamide as an internal standard), and the main pharmacokinetic parameters were calculated by Winnolin 8.3 non-compartment model. As shown in Table 4, the compounds of the present invention have better pharmacokinetic characteristics than the reference compound.

**Table 4 Pharmacokinetic parameters in mice**

| Example | Route of administration | Dosage (mg/kg) | Cₘₐₓ (µg/mL) | AUC (µg·h/mL) | t_{1/2} (h) |
|---|---|---|---|---|---|
| 5 | po | 50 | 121 | 725 | 3.49 |
| 16 | po | 50 | 93 | 612 | 3.39 |
| 28 | po | 50 | 132 | 1585 | 9.81 |

### 3. Study of pharmacokinetics in rats in vivo

After being fasted overnight (free access to water), healthy adult SD rats weighing 180-220 g were divided into a tail vein administration group and an intragastric administration group. In the tail vein administration group, 0.1 mL of blood samples were taken from the orbital venous plexus before administration and at 5 min, 15 min, 0.5 h, 1 h, 2 h, 4 h, 8 h, 12 h, and 24 h after administration, respectively, and the blood samples were centrifuged at 4°C for 5 min to separate plasma, which was stored at -20°C for testing. In the intragastric administration group, 0.1 mL of blood samples were collected from the orbital venous plexus before administration and at 5 min, 15 min, 0.5 h, 1 h, 2 h, 4 h, 8 h, 12 h, and 24 h after administration, and the treatment method was the same as in the intravenous injection administration group. The concentration of the original drug in the plasma was determined by LC-MS/MS method.

The results showed that the tested compounds had a good exposure level in rats.

### 4. Construction of humanized MRGPRX4 mice

Using CRISPR/Cas9 technology, ROSA26 CAG-loxp-STOP-loxP-hMRGPRX4 CDS-P2A-EGFP-WPRE-PA gene knocking into mice and Mrgpra3 iCre-P2A-tdtomato WPRE-PA gene knocking into mice were carried out, respectively, and the two transgenic mouse strains were mated to obtain transgenic mice that specifically expressed hMRGPRX4, which provided an animal model for studying the function of hMRGPRX4 and screening anti-pruritic drugs against the target hMRGPRX4.

The results showed that the test compounds can significantly inhibit the scratching times of the mice.

The specification of the present invention describes specific embodiments in detail, and those skilled in the art should realize that the above embodiments are exemplary and should not be understood as limiting the present invention. For those skilled in the art, without departing from the principle of the present invention, several improvements and modifications are made to the present invention, and the technical solutions obtained by these improvements and modifications also fall within the scope of protection of the claims of the present invention.

## Claims

1. A compound represented by formula (I), or a stereoisomer, deuterated compound, pharmaceutically acceptable salt, solvate, prodrug, metabolite, or co-crystal thereof: 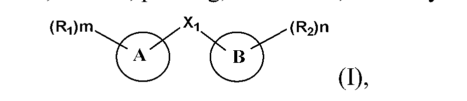
**characterized in that** ring A is selected from 6- to 10-membered aryl or 5- to 10-membered heteroaryl, wherein the 5- to 10-membered heteroaryl contains 1 to 3 heteroatoms selected from N, O, or S;
ring B is selected from 6- to 12-membered carbocyclyl or 6- to 12-membered heterocyclyl, wherein the 6- to 12-membered heterocyclyl contains 1 to 3 heteroatoms selected from N, O, or S;
X₁ is selected from C₁₋₆ alkylene, C₁₋₆ alkenylene, C₁₋₆ alkynylene, -C(O)-, -NR₃-, -O-, -S-, or -C(O)NR₃-, wherein the C₁₋₆ alkylene is optionally further substituted with a C₁₋₆ alkyl substituent;
R₁, R₂, and R₃ are each independently selected from H, cyano, halogen, hydroxyl, carboxyl, C₁₋₆ alkyl, C₂₋₆ alkenyl, C₂₋₆ alkynyl, C₁₋₆ haloalkyl, -O-C₁₋₆ alkyl, -O-C₁₋₆ haloalkyl, -C(O)NH₂, 3- to 6-membered cycloalkyl, 3- to 6-membered heterocycloalkyl, 5- to 6-membered heterocyclyl, or -C₁₋₆ alkylene-O-C₁₋₆ alkyl, wherein the C₁₋₆ alkyl substituent is optionally further substituted with a -O-C₁₋₆ alkyl, or cyano substituent; and
m and n are each independently selected from 0, 1, 2, 3, or 4.

2. The compound, or the stereoisomer, deuterated compound, pharmaceutically acceptable salt, solvate, prodrug, metabolite, or co-crystal thereof according to claim 1, wherein the compound is selected from compounds represented by general formula (II): 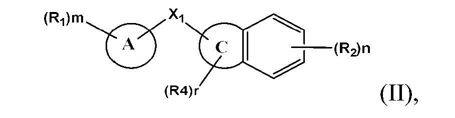 ,
**characterized in that** ring A is selected from 6- to 8-membered aryl or 5- to 6-membered heteroaryl, wherein the 5- to 6-membered heteroaryl contains 1 to 3 heteroatoms selected from N, O, or S;
ring C is selected from 5- to 6-membered heterocyclyl, wherein the 5- to 6-membered heterocyclyl contains 1 to 3 heteroatoms selected from N, O, or S;
X₁ is selected from C₁₋₆ alkylene or -C(O)-, wherein the C₁₋₆ alkylene is optionally further substituted with a C₁₋₆ alkyl substituent;
each R₁ is independently selected from halogen, cyano, carboxyl, -C(O)NH₂, C₁₋₆ haloalkyl, or 5-membered heterocyclyl, wherein the 5-membered heterocyclyl contains 3 to 4 heteroatoms selected from N or O, and the 5-membered heterocyclyl is optionally further substituted with a =O substituent;
each R₂ is independently selected from halogen, cyano, C₁₋₆ alkyl, C₂₋₆ alkynyl, C₁₋₆ haloalkyl, -O-C₁₋₆ haloalkyl, -C₁₋₆ alkylene-O-C₁₋₆ alkyl, 3- to 6-membered cycloalkyl, 5-membered heteroaryl, or 6-membered heterocycloalkyl, wherein the C₁₋₆ alkyl is optionally further substituted with a C₁₋₆ alkoxy, cyano, or halogen substituent;
each R₄ is independently selected from C₁₋₆ alkyl or cyano;
m is selected from 1 or 2;
r is selected from 0, 1, or 2; and
n is selected from 0, 1, 2, 3, or 4.

3. The compound, or the stereoisomer, deuterated compound, pharmaceutically acceptable salt, solvate, prodrug, metabolite, or co-crystal thereof according to claim 2, **characterized in that**
ring A is selected from 6-membered aryl or 5- to 6-membered heteroaryl, wherein the 5- to 6-membered heteroaryl contains 1 to 3 heteroatoms selected from N, O, or S;
ring C is selected from 5- to 6-membered azaheterocyclyl;
X₁ is selected from C₁₋₄ alkylene or -C(O)-, wherein the C₁₋₄ alkylene is optionally further substituted with a C₁₋₄ alkyl substituent;
each R₁ is independently selected from halogen, cyano, carboxyl, -C(O)NH₂, C₁₋₄ haloalkyl, or 5-membered heterocyclyl, wherein the 5-membered heterocyclyl contains 3 to 4 heteroatoms selected from N or O, and the 5-membered heterocyclyl is optionally further substituted with a =O substituent;
each R₂ is independently selected from halogen, cyano, C₁₋₄ alkyl, C₂₋₄ alkynyl, C₁₋₄ haloalkyl, -O-C₁₋₄ haloalkyl, -C₁₋₄ alkylene-O-C₁₋₄ alkyl, 3- to 6-membered cycloalkyl, 5-membered heteroaryl, or 6-membered heterocycloalkyl, wherein the C₁₋₄ alkyl is optionally further substituted with a C₁₋₄ alkoxy, cyano, or halogen substituent; and
each R₄ is independently selected from C₁₋₄ alkyl or cyano.

4. The compound, or the stereoisomer, deuterated compound, pharmaceutically acceptable salt, solvate, prodrug, metabolite, or co-crystal thereof according to claim 3, **characterized in that**
ring C is selected from 5-membered azaheterocyclyl.

5. The compound, or the stereoisomer, deuterated compound, pharmaceutically acceptable salt, solvate, prodrug, metabolite, or co-crystal thereof according to claim 4, **characterized in that**
ring A is selected from 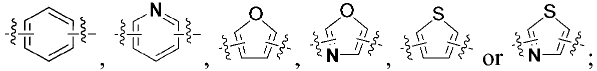 a condensed ring system formed from ring C and a benzene ring is selected from 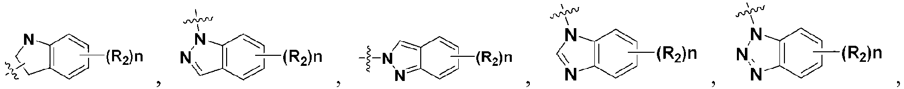 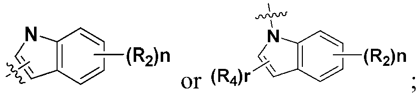
each R₂ is independently selected from halogen, cyano, C₁₋₄ alkyl, C₂₋₄ alkynyl, C₁₋₄ haloalkyl, -O-C₁₋₄ haloalkyl, 3- to 6-membered cycloalkyl, 5-membered heteroaryl, or 6-membered heterocycloalkyl, wherein the C₁₋₄ alkyl is optionally further substituted with a C₁₋₄ alkoxy, cyano or halogen substituent.

6. The compound, or the stereoisomer, deuterated compound, pharmaceutically acceptable salt, solvate, prodrug, metabolite, or co-crystal thereof according to claim 5, **characterized in that** the compound is selected from the following structures: 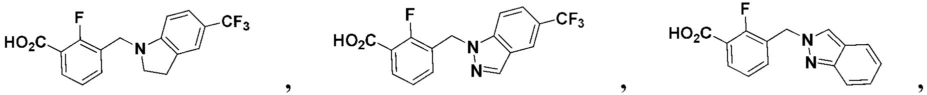 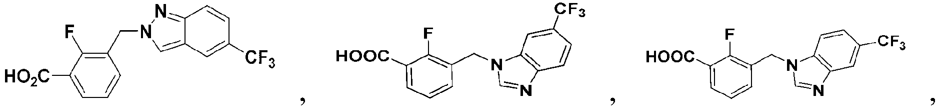 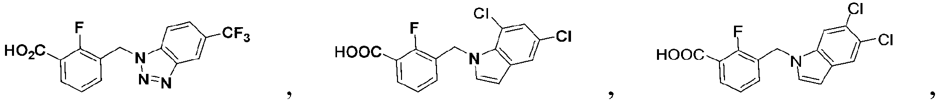 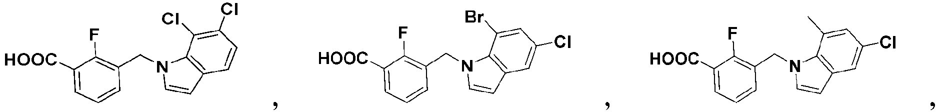 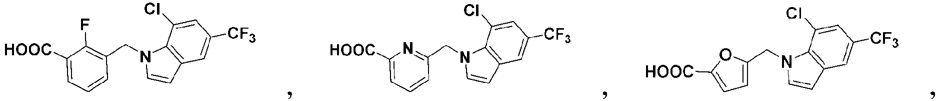 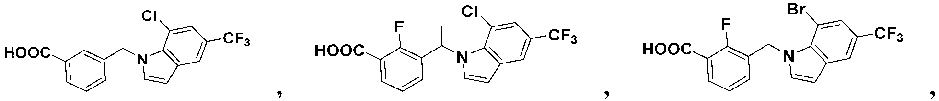 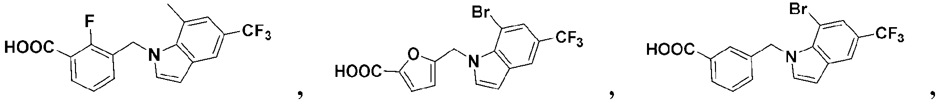 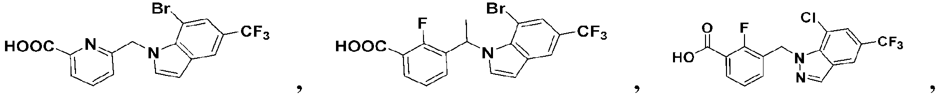 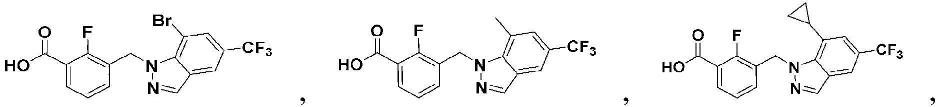 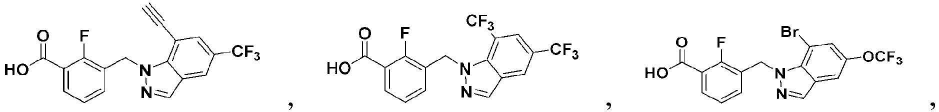 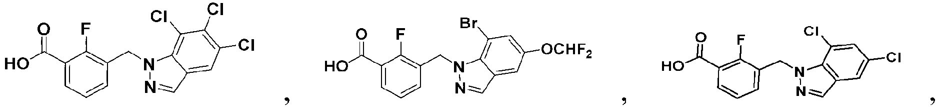 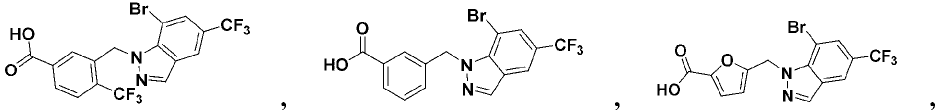 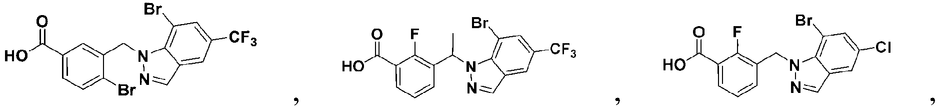 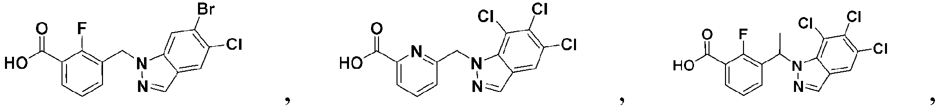 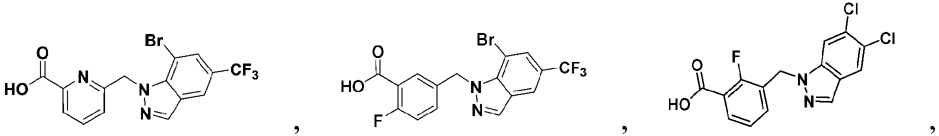 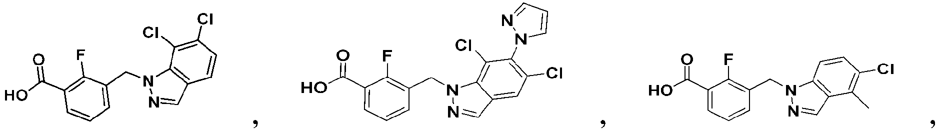 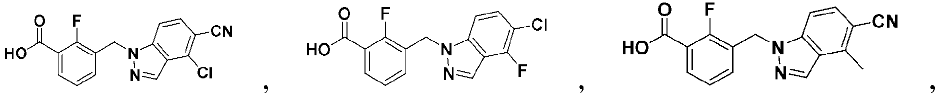 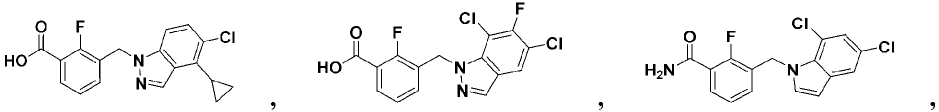 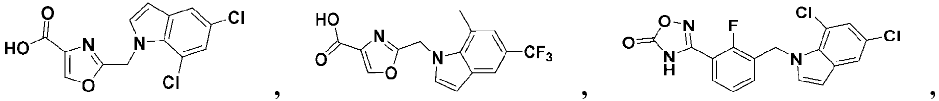 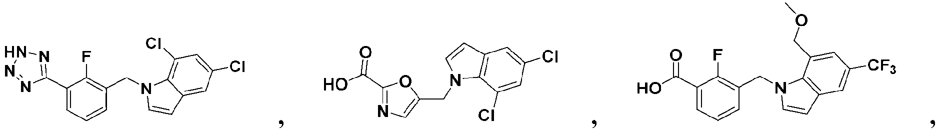 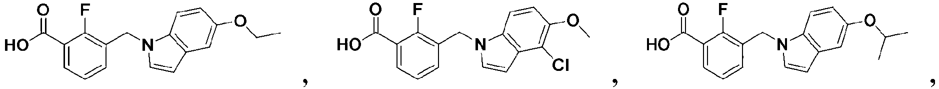 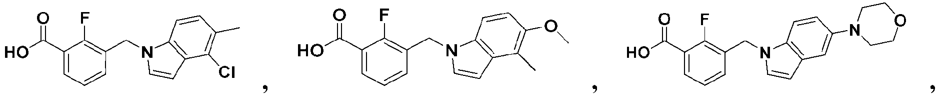 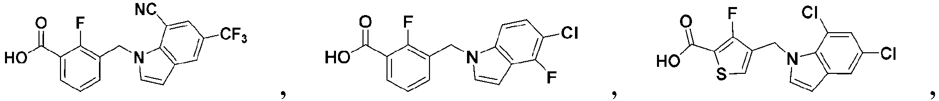 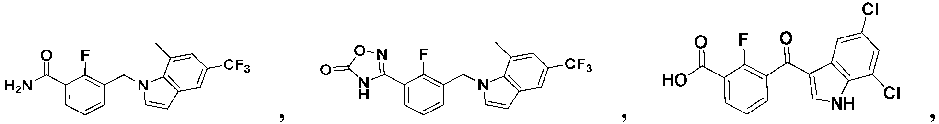 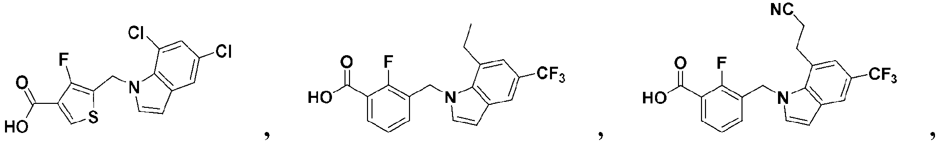 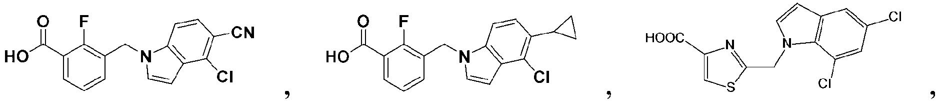 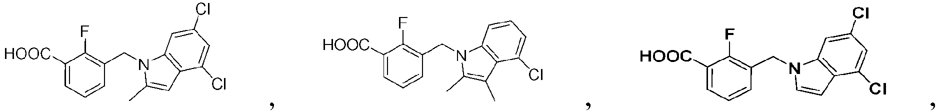 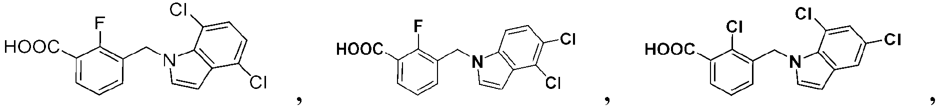 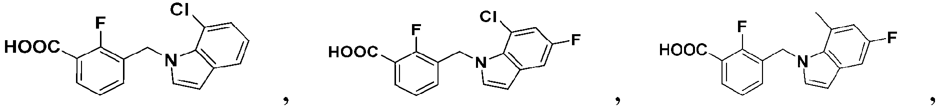 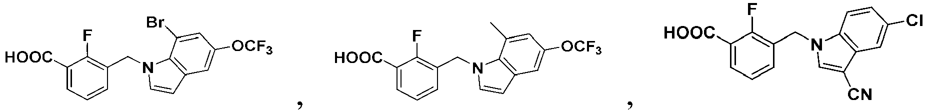 or

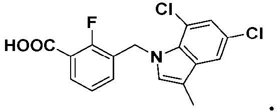

7. A pharmaceutical composition, **characterized by** comprising:
the compound, or the stereoisomer, deuterated compound, pharmaceutically acceptable salt, solvate, prodrug, metabolite, or co-crystal thereof according to any one of claims 1-6; and
one or more pharmaceutically acceptable carriers and/or excipients.

8. Use of the pharmaceutical composition according to claim 7 or the compound, or the stereoisomer, deuterated compound, pharmaceutically acceptable salt, solvate, prodrug, metabolite, or co-crystal thereof according to any one of claims 1-6 in the preparation of a medicament for treating an MRGPRX4-dependent condition.

9. The use according to claim 8, **characterized in that** the MRGPRX4-dependent condition is a pruritus-related condition, a pain-related condition, or an autoimmune condition.
